(19) Europäisches Patentamt
European Patent Office
Office européen des brevets

(11) EP 1 994 020 B1

(12) # EUROPÄISCHE PATENTSCHRIFT

(45) Veröffentlichungstag und Bekanntmachung des Hinweises auf die Patenterteilung:
**22.06.2011 Patentblatt 2011/25**

(21) Anmeldenummer: **07711438.7**

(22) Anmeldetag: **02.02.2007**

(51) Int Cl.:
***C07D 401/04*** (2006.01) ***C07D 498/04*** (2006.01)
***C07D 471/10*** (2006.01) ***C07D 498/10*** (2006.01)
***C07D 401/14*** (2006.01) ***A61K 31/4709*** (2006.01)
***A61P 31/22*** (2006.01)

(86) Internationale Anmeldenummer:
**PCT/EP2007/000923**

(87) Internationale Veröffentlichungsnummer:
**WO 2007/090579 (16.08.2007 Gazette 2007/33)**

(54) **Substituierte Chinolone III**

Substituted quinolones III

Quinolones substituées III

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **09.02.2006 DE 102006005861**

(43) Veröffentlichungstag der Anmeldung:
**26.11.2008 Patentblatt 2008/48**

(73) Patentinhaber: **AiCuris GmbH & Co. KG**
**42117 Wuppertal (DE)**

(72) Erfinder:
- **FUERSTNER, Chantal**
**45478 Muelheim an der Ruhr (DE)**
- **THEDE, Kai**
**10405 Berlin (DE)**
- **ZIMMERMANN, Holger**
**42111 Wuppertal (DE)**
- **BRUECKNER, David**
**45128 Essen (DE)**
- **HENNINGER, Kerstin**
**42289 Wuppertal (DE)**
- **LANG, Dieter**
**42553 Velbert (DE)**
- **SCHOHE-LOOP, Rudolf**
**42327 Wuppertal (DE)**

(74) Vertreter: **Scheuermann, Erik et al**
**Witte, Weller & Partner**
**Phoenixbau**
**Königstrasse 5**
**70173 Stuttgart (DE)**

(56) Entgegenhaltungen:
**WO-A-00/40561 WO-A-02/085886**

EP 1 994 020 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung betrifft substituierte Chinolone und Verfahren zu ihrer Herstellung sowie ihre Verwendung zur Herstellung von Arzneimitteln zur Behandlung und/oder Prophylaxe von Krankheiten, insbesondere zur Verwendung als antivirale Mittel, insbesondere gegen Cytomegaloviren.

WO 00/040561 und US 4,959,363 beschreiben Chinolone mit Wirkung gegen Viren der Herpes-Familie. EP-A 612731 beschreibt Chinolone als antivirale Mittel, besonders gegen HIV. WO 02/009758, WO 02/085886 und WO 03/050107 beanspruchen Chinolone als Breitspektrum Antibiotika. In WO 97/004775 und WO 97/004779 werden Chinolone als Inhibitoren von PDE4 und TNF$\alpha$ unter anderem für die Behandlung von inflammatorischen Erkrankungen, HIV und HCMV beschrieben. EP-A 276700 beschreibt 8-Cyano-Chinolone als Antibiotika. WO 02/026713 beschreibt Chinolone als antiparasitäre Verbindungen.

Auf dem Markt sind strukturell andersartige, antiviral wirkende Mittel vorhanden, deren Anwendungsbreite aufgrund eines ausgeprägten Nebenwirkungsprofils und möglicher Resistenzentwicklung stark eingeschränkt ist. Neue Mittel für eine bessere und wirksamere Therapie sind daher wünschenswert.

Eine Aufgabe der vorliegenden Erfindung ist es daher, neue Verbindungen mit gleicher oder verbesserter antiviraler Wirkung zur Behandlung von viralen Infektionskrankheiten bei Menschen und Tieren zur Verfügung zu stellen.

Überraschenderweise wurde gefunden, dass die in der vorliegenden Erfindung beschriebenen substituierten Chinolone antiviral wirksam sind.

Gegenstand der Erfindung sind Verbindungen der Formel

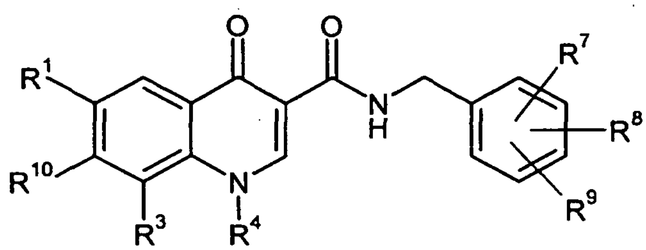 (I),

in welcher

$R^1$ für Wasserstoff, Fluor, Chlor oder Trifluormethyl steht,

$R^3$ für Halogen, Hydroxy, $C_1$-$C_4$-Alkoxy, Cyano, Trifluormethyl, Monofluor- methoxy, Difluormethoxy, Trifluormethoxy oder Ethinyl steht,

$R^4$ für $C_1$-$C_6$-Alkyl oder $C_3$-$C_8$-Cycloalkyl steht, wobei Alkyl substituiert sein kann mit 1 bis 3 Substituenten, wobei die Substi- tuenten unabhängig voneinander ausgewählt werden aus der Gruppe beste- hend aus Halogen, Hydroxy, Amino, Cyano, Trifluormethyl, Hydroxycarbo- nyl, Aminocarbonyl, $C_1$-$C_6$-Alkoxy, $C_1$-$C_6$-Alkylamino, $C_1$-$C_6$-Alkylcarbonyl und $C_1$-$C_6$-Alkoxycarbonyl,
und
wobei Cycloalkyl substituiert sein kann mit 1 bis 3 Substituenten, wobei die Substituenten unabhängig voneinander ausgewählt werden aus der Gruppe bestehend aus Halogen, Hydroxy, Amino, Cyano, Trifluormethyl, Hydroxycarbonyl, Aminocarbonyl, $C_1$-$C_6$-Alkyl, $C_1$-$C_6$-Alkoxy, $C_1$-$C_6$-Alkylamino, $C_1$-$C_6$- Alkylcarbonyl und $C_1$-$C_6$-Alkoxycarbonyl,

oder

$R^3$ und $R^4$ bilden gemeinsam mit den Atomen an die sie gebunden sind einen Ring durch eine Gruppe der Formel

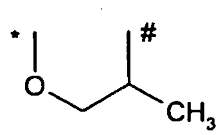

wobei

* die Anknüpfstelle an das Kohlenstoffatom ist,
und
\# die Anknüpfstelle an das Stickstoffatom ist,

$R^7$ und $R^8$ unabhängig voneinander für Halogen, Hydroxy, Cyano, Trifluor- methyl, Monofluormethoxy, Difluormethoxy, Trifluormethoxy, $C_1$-$C_3$-Alkyl oder $C_1$-$C_3$-Alkoxy stehen,
und

$R^9$ für Wasserstoff, Halogen, Hydroxy, Cyano, Trifluormethyl, Monofluormeth- oxy, Difluormethoxy, Trifluormethoxy, $C_1$-$C_3$-Alkyl oder $C_1$-$C_3$-Alkoxy steht,
oder

$R^8$ für Trifluormethoxy steht,
und

$R^7$ und $R^9$ für Wasserstoff stehen,

$R^{10}$ für eine Gruppe der Formel

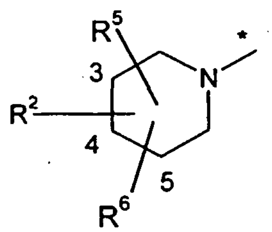
,
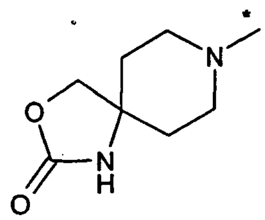
oder
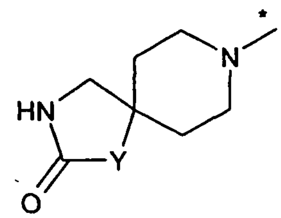

steht,
wobei
* die Anknüpfstelle an das Kohlenstoffatom ist,
$R^2$ an die Position 3 oder 4 gebunden ist und für Hydroxy, Hydro- xycarbonyl, Aminocarbonyl, $C_1$-$C_4$-Alkyl, $C_1$-$C_4$-Alkoxycarbonyl, $C_3$-$C_6$-Cycloalkylaminocarbonyl oder gegebenenfalls Hydroxy substituiertes $C_1$-$C_6$-Alkylaminocarbonyl steht, wobei Alkyl substituiert ist mit einem Substituenten, wobei der Substituent ausgewählt wird aus der Gruppe bestehend aus Hydroxy, Hydroxycarbonyl, Aminocarbonyl, $C_1$-$C_4$-Alkoxy-carb- onyl und 2-Oxopyrrolidin-1-yl,
$R^5$ und $R^6$ unabhängig voneinander an die Position 3, 4 oder 5 ge- bunden sind und unabhängig voneinander für Wasserstoff, Hydroxy, Methyl oder Ethyl stehen,
und
Y für eine Methylengruppe oder ein Sauerstoffatom steht,

und ihre Salze, ihre Solvate und die Solvate ihrer Salze.

Erfindungsgemäße Verbindungen sind die Verbindungen der Formel (I) und (Ia) und deren Salze, Solvate und Solvate der Salze, sowie die von Formel (I) und (Ia) umfassten, nachfolgend als Ausführungsbeispiel(e) genannten Verbindungen und deren Salze, Solvate und Solvate der Salze, soweit es sich bei den von Formel (I) und (Ia) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von

Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin, *N*-Methylpiperidin und Cholin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Außerdem umfasst die vorliegende Erfindung auch Prodrugs der erfindungsgemäßen Verbindungen. Der Begriff "Prodrugs" umfasst Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:

<u>Alkyl per se und "Alk" und "Alkyl" in Alkoxy, Alkylamino. Alkylcarbonyl, Alkoxycarbonyl und Alkylaminocarbonyl</u> stehen für einen linearen oder verzweigten Alkylrest mit in der Regel 1 bis 6, vorzugsweise 1 bis 4, besonders bevorzugt 1 bis 3 Kohlenstoffatomen, beispielhaft und vorzugsweise für Methyl, Ethyl, n-Propyl, Isopropyl, n-Butyl, *tert*-Butyl, n-Pentyl und n-Hexyl.

<u>Alkoxy</u> steht beispielhaft und vorzugsweise für Methoxy, Ethoxy, n-Propoxy, Isopropoxy, n-Butoxy, *tert*-Butoxy, n-Pentoxy und n-Hexoxy.

<u>Alkylamino</u> steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, n-Butylamino, *tert*-Butylamino, n-Pentylamino, n-Hexylamino, *N*,*N*-Dimethylamino, *N*,*N*-Diethylamino, *N*-Ethyl-*N*-methylamino, *N*-Methyl-*N*-n-propylamino, *N*-Isopropyl-*N*-n-propylamino, *N*-Methyl-*N*-n-butylamino, *N*-*tert*-Butyl-*N*-methylamino, *N*-Ethyl-*N*-n-pentylamino und *N*-n-Hexyl-*N*-methylamino. $C_1$-$C_3$-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstitüent.

<u>Alkylcarbonyl</u> steht beispielhaft und vorzugsweise für Acetyl und Propanoyl.

<u>Alkoxycarbonyl</u> steht beispielhaft und vorzugsweise für Methoxycarbonyl, Ethoxycarbonyl, n-Propoxycarbonyl, Isopropoxycarbonyl, n-Butoxycarbonyl, *tert*-Butoxycarbonyl, n-Pentoxycarbonyl und n-Hexoxycarbonyl.

<u>Alkylaminocarbonyl</u> steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, Isopropylaminocarbonyl, *tert*-Butylaminocarbonyl, n-Pentylaminocarbonyl, n-Hexylaminocarbonyl, *N*, *N*-Dimethylaminocarbonyl, *N*,*N*-Diethylaminocarbonyl, *N*-Ethyl-*N*-methylaminocarbonyl, *N*-Methyl-*N*-n-propytaminocarbonyl, *N*-Isopropyl-*N*-n-pröpylaminocarbonyl, *N*-Methyl-*N*-n-butylaminocarbonyl, *N*-*tert*-Butyl-*N*-methylamino-carbonyl, *N*-Ethyl-*N*-n-pentylaminocarbonyl und *N*-n-Hexyl-*N*-methylaminocarbonyl. $C_1$-$C_3$-Alkylaminocarbonyl steht beispielsweise für einen Monoalkylaminocarbonylrest mit 1 bis 3 Kohlenstoffatomen oder für einen Dialkylaminocarbonylrest mit jeweils 1 bis 3 Kohlenstoffatomen pro Alkylsubstituent.

<u>Cycloalkyl</u> steht für eine Cycloalkylgruppe mit in der Regel 3 bis 8, bevorzugt 3 bis 5 Kohlenstoffatomen. Beispielhaft und vorzugsweise für Cycloalkyl sind genannt Cyclopropyl, Cyclobutyl, Cyclopentyl, Cyclohexyl und Cycloheptyl.

<u>Cycloalkylaminocarbonyl</u> steht für eine Cycloalkylgruppe mit in der Regel 3 bis 6 Kohlenstoffatomen, die über eine Aminocarbonyl-Gruppe gebunden ist. Beispielhaft und vorzugsweise für Cycloalkylaminocarbonyl sind genannt Cyclopropylaminocarbonyl, Cyclobutylaminocarbonyl, Cyclopentylaminocarbonyl und Cyclohexylaminocarbonyl.

<u>Halogen</u> steht für Fluor, Chlor, Brom und Jod, bevorzugt Fluor und Chlor.

In der Formel der Gruppe, die für $R^3$ und $R^4$ stehen kann, steht der Endpunkt der Linie, neben der jeweils ein * oder # steht, nicht für ein Kohlenstoffatom beziehungsweise eine $CH_2$-Gruppe sondern ist Bestandteil der Bindung zu dem Atom, an das $R^3$ und $R^4$ gebunden sind.

In den Formeln der Gruppe, die für $R^{10}$ stehen kann, steht der Endpunkt der Linie, neben der jeweils ein * steht, nicht für ein Kohlenstoffatom beziehungsweise eine $CH_2$-Gruppe sondern ist Bestandteil der Bindung zu dem Atom, an das $R^{10}$ gebunden ist.

Bevorzugt sind solche Verbindungen der Formel (I), welche der Formel

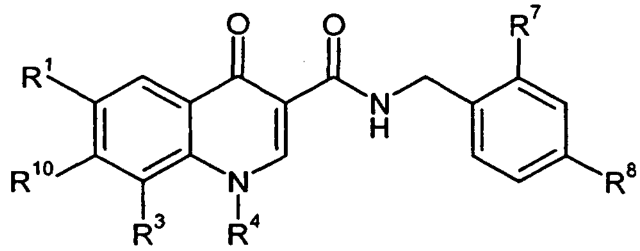 (Ia),

entsprechen,
in welcher

$R^1$ für Wasserstoff, Fluor, Chlor oder Trifluormethyl steht,

$R^3$ für Halogen, Hydroxy, $C_1$-$C_4$-Alkoxy, Cyano, Trifluormethyl, Monofluor- methoxy, Difluormethoxy, Trifluormethoxy oder Ethinyl steht,

$R^4$ für $C_1$-$C_6$-Alkyl oder $C_3$-$C_8$-Cycloalkyl steht, wobei Alkyl substituiert sein kann mit 1 bis 3 Substituenten, wobei die Substi- tuenten unabhängig voneinander ausgewählt werden aus der Gruppe beste- hend aus Halogen, Hydroxy, Amino, Cyano, Trifluormethyl, Hydroxycarbonyl, Aminocarbonyl, $C_1$-$C_6$-Alkoxy, $C_1$-$C_6$-Alkylamino, $C_1$-$C_6$-Alkylcarbonyl und $C_1$-$C_6$-Alkoxycarbonyl, und
wobei Cycloalkyl substituiert sein kann mit 1 bis 3 Substituenten, wobei die Substituenten unabhängig voneinander ausgewählt werden aus der Gruppe bestehend aus Halogen, Hydroxy, Amino, Cyano, Trifluormethyl, Hydroxycarbonyl, Aminocarbonyl, $C_1$-$C_6$-Alkyl, $C_1$-$C_6$-Alkoxy, $C_1$-$C_6$-Alkylamino, $C_1$-$C_6$- Alkylcarbonyl und $C_1$-$C_6$-Alkoxycarbonyl,

oder

$R^3$ und $R^4$ bilden gemeinsam mit den Atomen an die sie gebunden sind einen Ring durch eine Gruppe der Formel

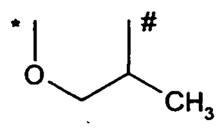

wobei
* die Anknüpfstelle an das Kohlenstoffatom ist,
und
# die Anknüpfstelle an das Stickstoffatom ist,

$R^7$ und $R^8$ unabhängig voneinander für Halogen, Hydroxy, Cyano, Trifluor- methyl, Monofluormethoxy, Difluormethoxy, Trifluormethoxy, $C_1$-$C_3$-Alkyl oder $C_1$-$C_3$-Alkoxy stehen,

$R^{10}$ für eine Gruppe der Formel

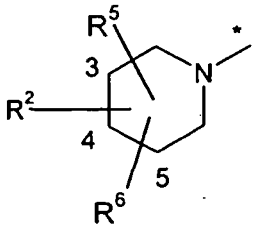 , 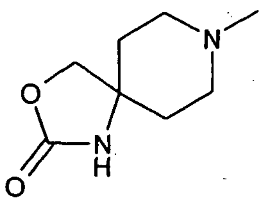 oder 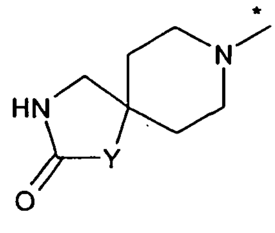

steht,
wobei
* die Anknüpfstelle an das Kohlenstoffatom ist,
$R^2$ an die Position 3 oder 4 gebunden ist und für Hydroxy, Hydro- xycarbonyl, Aminocarbonyl, $C_1$-$C_4$-Alkyl oder $C_1$-$C_4$-Alkoxy- carbonyl steht,
wobei Alkyl substituiert ist mit einem Substituenten, wobei der Substituent ausgewählt wird aus der Gruppe bestehend aus Hydroxy, Hydroxycarbonyl, Aminocarbonyl und $C_1$-$C_4$-Alkoxy- carbonyl,
$R^5$ und $R^6$ unabhängig voneinander an die Position 3, 4 oder 5 ge- bunden sind und unabhängig voneinander für Wasserstoff, Hydroxy, Methyl oder Ethyl stehen,
und
Y für eine Methylengruppe oder ein Sauerstoffatom steht,

und ihre Salze, ihre Solvate und die Solvate ihrer Salze.

Bevorzugt sind auch solche Verbindungen der Formel (I) und (Ia), in welcher

$R^1$ für Wasserstoff, Fluor oder Chlor steht,

$R^3$ für Halogen, Hydroxy, $C_1$-$C_3$-Alkoxy, Cyano, Trifluormethyl, Monofluor- methoxy, Difluormethoxy oder Trifluormethoxy steht,

$R^4$ für $C_1$-$C_6$-Alkyl oder $C_3$-$C_6$-Cycloalkyl steht, wobei Alkyl substituiert sein kann mit 1 bis 3 Substituenten, wobei die Substi- tuenten unabhängig voneinander ausgewählt werden aus der Gruppe beste- hend aus Halogen, Hydroxy, Amino, Cyano, Trifluormethyl und $C_1$-$C_4$- Alkoxy, und
wobei Cycloalkyl substituiert sein kann mit 1 bis 3 Substituenten, wobei die Substituenten unabhängig voneinander ausgewählt werden aus der Gruppe bestehend aus Halogen, Hydroxy, Amino, Cyano, Trifluormethyl, $C_1$-$C_4$-Alkyl und $C_1$-$C_4$-Alkoxy,

oder

$R^3$ und $R^4$ bilden gemeinsam mit den Atomen an die sie gebunden sind einen Ring durch eine Gruppe der Formel

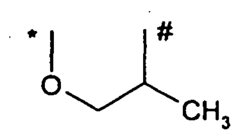

wobei
* die Anknüpfstelle an das Kohlenstoffatom ist,
und
# die Anknüpfstelle an das Stickstoffatom ist,

$R^7$ und $R^8$ unabhängig voneinander für Halogen, Cyano, Trifluormethyl, Mono- fluormethoxy, Difluormethoxy, Trifluormethoxy, $C_1$-$C_3$-Alkyl oder $C_1$-$C_3$- Alkoxy stehen,

$R^{10}$ für eine Gruppe der Formel

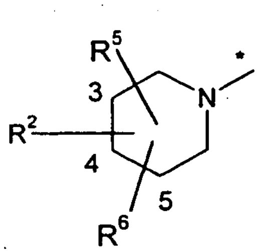 , 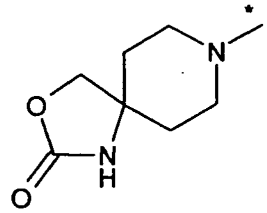 oder 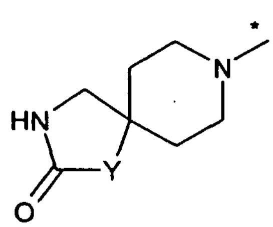

steht,
wobei
* die Anknüpfstelle an das Kohlenstoffatom ist,
$R^2$ an die Position 3 oder 4 gebunden ist und für Hydroxy, Hydro- xycarbonyl, Aminocarbonyl, $C_1$-$C_4$-Alkyl oder $C_1$-$C_4$-Alkoxy- carbonyl steht,
$R^5$ und $R^6$ wobei Alkyl substituiert ist mit einem Substituenten, wobei der Substituent ausgewählt wird aus der Gruppe bestehend aus Hydroxycarbonyl und $C_1$-$C_4$-Alkoxycarbonyl, unabhängig voneinander an die Position 3, 4 oder 5 ge- bunden sind und unabhängig voneinander für Wasserstoff, Hydroxy, Methyl oder Ethyl stehen,
und
Y für eine Methylengruppe oder ein Sauerstoffatom steht,

und ihre Salze, ihre Solvate und die Solvate ihrer Salze.

Bevorzugt sind auch solche Verbindungen der Formel (I) und (Ia), in welcher

$R^1$ für Wasserstoff oder Fluor steht,

$R^3$ für Chlor, Hydroxy, Methoxy, Ethoxy, Cyano, Trifluormethyl, Monofluor- methoxy, Difluormethoxy oder Trifluormethoxy steht,

$R^4$ für $C_1$-$C_4$-Alkyl, Cyclopropyl, Cyclobutyl oder Cyclopentyl steht, wobei Alkyl substituiert sein kann mit 1 bis 3 Substituenten, wobei die Substi- tuenten unabhängig voneinander ausgewählt werden aus der Gruppe bestehend aus Halogen, Trifluormethyl und $C_1$-$C_4$-Alkoxy, und wobei Cyclopropyl, Cyclobutyl und Cyclopentyl substituiert sein können mit 1 bis 3 Substituenten, wobei die Substituenten unabhängig voneinander ausgewählt werden aus der Gruppe bestehend aus Halogen, Trifluormethyl, Me- thyl, Ethyl, Methoxy und Ethoxy,

oder

$R^3$ und $R^4$ bilden gemeinsam mit den Atomen an die sie gebunden sind einen Ring durch eine Gruppe der Formel

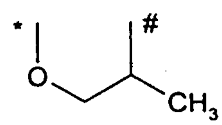

wobei
* die Anknüpfstelle an das Kohlenstoffatom ist,
und
\# die Anknüpfstelle an das Stickstoffatom ist,

$R^7$ und $R^8$ unabhängig voneinander für Chlor, Brom, Trifluormethyl, Monofluor- methoxy, Difluormethoxy, Trifluormethoxy, Methyl oder Methoxy stehen,

$R^{10}$ für eine Gruppe der Formel

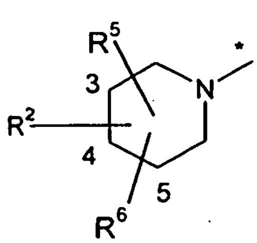 , 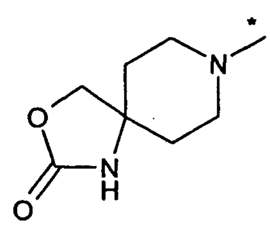 oder 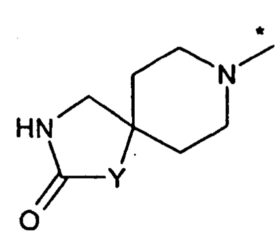

steht,
wobei
* die Anknüpfstelle an das Kohlenstoffatom ist,
$R^2$ an die Position 3 oder 4 gebunden ist und für Hydroxy, Hydro- xycarbonyl, Aminocarbonyl, Methyl, Ethyl oder $C_1$-$C_4$-Alkoxy- carbonyl steht, wobei Methyl und Ethyl substituiert sind mit einem Substituenten, wobei der Substituent ausgewählt wird aus der Gruppe be- stehend aus Hydroxycarbonyl und $C_1$-$C_4$-Alkoxycarbonyl,
$R^5$ an die Position 3 gebunden ist und für Wasserstoff, Hydroxy oder Methyl steht,
$R^6$ an die Position 5 gebunden ist und für Wasserstoff, Hydroxy oder Methyl steht,
und
Y für eine Methylengruppe oder ein Sauerstoffatom steht, und ihre Salze, ihre Solvate und die Solvate ihrer Salze.

Bevorzugt sind auch solche Verbindungen der Formel (I) und (Ia), in welcher

$R^1$ für Fluor steht,

$R^3$ für Chlor, Hydroxy, Methoxy oder Ethoxy steht,

$R^4$ für $C_1$-$C_3$-Alkyl, Cyclopropyl oder Cyclobutyl steht, wobei Alkyl substituiert sein kann mit 1 bis 2 Substituenten, wobei die Substi- tuenten unabhängig voneinander ausgewählt werden aus der Gruppe bestehend aus Fluor und Trifluormethyl,
und
wobei Cyclopropyl und Cyclobutyl substituiert sein können mit 1 bis 3 Substi- tuenten Fluor,

$R^7$ und $R^8$ unabhängig voneinander für Chlor, Trifluormethyl, Trifluormethoxy oder Methyl stehen,

$R^{10}$ für eine Gruppe der Formel

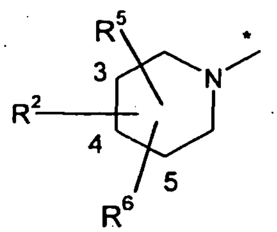

oder

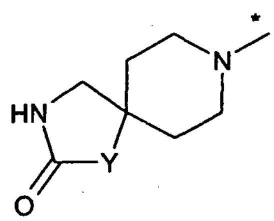

steht,
wobei
* die Anknüpfstelle an das Kohlenstoffatom ist,
$R^2$ an die Position 3 oder 4 gebunden ist und für Hydroxy, Hydro- xycarbonyl, Aminocarbonyl, Methyl oder Ethyl steht, wobei Methyl und Ethyl substituiert sind mit einem Substituen- ten Hydroxycarbonyl,
$R^5$ an die Position 3 gebunden ist und für Wasserstoff oder Methyl steht,
$R^6$ an die Position 5 gebunden ist und für Wasserstoff oder Methyl steht,
und
Y für eine Methylengruppe steht,

und ihre Salze, ihre Solvate und die Solvate ihrer Salze.

Bevorzugt sind auch solche Verbindungen der Formel (I) und (Ia), in welcher $R^1$ für Fluor steht.

Bevorzugt sind auch solche Verbindungen der Formel (I) und (Ia), in welcher $R^2$ an die Position 3 oder 4 gebunden ist und für Hydroxy, Hydroxycarbonyl, Aminocarbonyl oder Methyl steht, wobei Methyl substituiert ist mit einem Substituenten Hydroxycarbonyl.

Bevorzugt sind auch solche Verbindungen der Formel (I) und (Ia), in welcher $R^2$ für Hydroxycarbonyl oder Hydroxycarbonylmethyl steht.

Bevorzugt sind auch solche Verbindungen der Formel (I) und (Ia), in welcher $R^3$ für Halogen, Hydroxy, $C_1$-$C_3$-Alk-

oxy, Cyano, Trifluormethyl, Monofluormethoxy, Difluormethoxy, Trifluormethoxy oder Ethinyl steht.

Bevorzugt sind auch solche Verbindungen der Formel (I) und (Ia), in welcher $R^3$ für Halogen, Cyano, Methoxy, Trifluormethyl, Monofluormethoxy, Difluormethoxy, Trifluormethoxy oder Ethinyl steht.

Bevorzugt sind auch solche Verbindungen der Formel (I) und (Ia), in welcher $R^3$ für Halogen, Cyano, Methoxy, Trifluormethyl, Monofluormethoxy, Difluormethoxy oder Trifluormethoxy steht.

Bevorzugt sind auch solche Verbindungen der Formel (I) und (Ia), in welcher $R^3$ für Chlor, Cyano, Methoxy, Trifluormethyl, Monofluormethoxy, Difluormethoxy oder Trifluormethoxy steht.

Bevorzugt sind auch solche Verbindungen der Formel (I) und (Ia), in welcher $R^3$ für Chlor, Methoxy, Trifluormethyl oder Difluormethoxy steht.

Bevorzugt sind auch solche Verbindungen der Formel (I) und (Ia), in welcher $R^3$ für Chlor oder Methoxy steht.

Bevorzugt sind auch solche Verbindungen der Formel (I) und (Ia), in welcher $R^3$ für Chlor, Hydroxy, Methoxy oder Ethoxy steht.

Bevorzugt sind auch solche Verbindungen der Formel (I) und (Ia), in welcher $R^4$ für Cyclopropyl oder 2-Fluorcycloprop-1-yl steht.

Bevorzugt sind auch solche Verbindungen der Formel (I) und (Ia), in welcher $R^4$ für 2,2,2-Trifluorethyl, 2,2-Difluorethyl, 2-Fluorethyl, 1-Fluorprop-2-yl oder 1,1,1-Trifluorprop-2-yl steht.

Bevorzugt sind auch solche Verbindungen der Formel (I) und (Ia), in welcher $R^4$ für 2,2,2-Trifluorethyl steht.

Bevorzugt sind auch solche Verbindungen der Formel (I) und (Ia), in welcher $R^5$ und $R^6$ für Wasserstoff oder Methyl stehen.

Bevorzugt sind auch solche Verbindungen der Formel (I), in welcher $R^7$ und $R^8$ unabhängig voneinander für Halogen, Trifluormethyl, Monofluormethoxy, Difluormethoxy, Trifluormethoxy, Methyl oder Methoxy stehen, und $R^9$ für Wasserstoff oder Methyl steht.

Bevorzugt sind auch solche Verbindungen der Formel (I) und (Ia), in welcher $R^7$ für Chlor oder Methyl und $R^8$ für Chlor, Trifluormethyl oder Trifluormethoxy steht.

Bevorzugt sind auch solche Verbindungen der Formel (I), in welcher $R^9$ für Wasserstoff steht.

Bevorzugt sind auch solche Verbindungen der Formel (I) und (Ia), in welcher $R^{10}$ für eine Gruppe der Formel

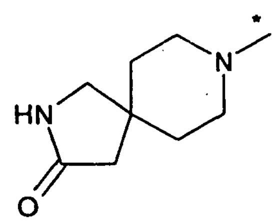

steht, wobei * die Anknüpfstelle an das Kohlenstoffatom ist.

Die in den jeweiligen Kombinationen bzw. bevorzugten Kombinationen von Resten im einzelnen angegebenen Restedefinitionen werden unabhängig von den jeweiligen angegebenen Kombinationen der Reste beliebig auch durch Restedefinitionen anderer Kombination ersetzt.

Ganz besonders bevorzugt sind Kombinationen von zwei oder mehreren der oben genannten Vorzugsbereiche.

Gegenstand der Erfindung ist weiterhin ein Verfahren zur Herstellung der Verbindungen der Formel (I), wobei

[A] Verbindungen der Formel

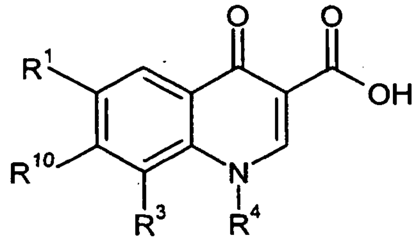

(II),

in welcher

$R^1$, $R^3$, $R^4$ und $R^{10}$ die oben angegebene Bedeutung aufweisen,
mit Verbindungen der Formel

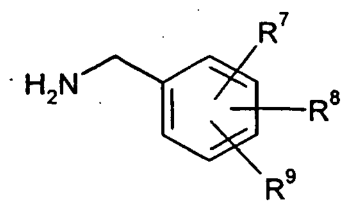 (III),

in welcher
$R^7$, $R^8$ und $R^9$ die oben angegebene Bedeutung aufweisen,
umgesetzt werden,
oder
[B] Verbindungen der Formel

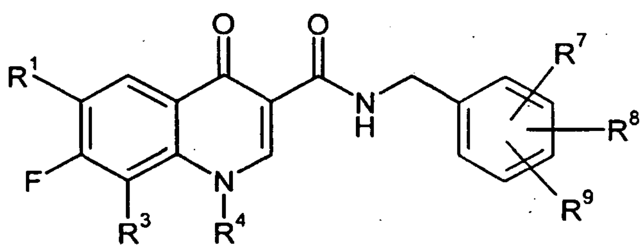 (IV),

in welcher
$R^1$, $R^3$, $R^4$, $R^7$, $R^8$ und $R^9$ die oben angegebene Bedeutung aufweisen,
mit Verbindungen der Formel

$R^{10}$-H (V),

in welcher
$R^{10}$ die oben angegebene Bedeutung aufweist,
umgesetzt werden,
oder
[C] Verbindungen, die nach Verfahren [A] oder [B] entstehen und eine beliebige Ester-Gruppe im Rest $R^{10}$ tragen, mit einer Base zu der entsprechenden Säure verseift werden. (Diese Ester-Gruppe kann, aber muss nicht der Definition von $R^{10}$ entsprechen.)

Die Umsetzung nach Verfahren [A] erfolgt im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, *N*-Methylpiperidin, 4-Dimethylaminopyridin oder Diisopropylethylamin.

Als Dehydratisierungsreagenzien eignen sich hierbei beispielsweise Carbodiimide wie z.B. *N*,*N*'-Diethyl-, *N*,*N*,'-Dipropyl-, *N*,*N*'-Diisopropyl-, *N*,*N*'-Dicyclohexylcarbodiimid, *N*-(3-Dimethylaminoisopropyl)-*N*'-ethylcarbodiimid-hydrochlorid (EDC), *N*-Cyclohexylcarbodiimid-*N*'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert*-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid,oder*O*-(Benzotriazol-1-yl)-*N*,*N*,*N*',*N*'-tetramethyluroniumhexafluorophosphat(HBTU),2-(2-Oxo-1-(2H)-pyridyl)-1,1,3,3-tetramethyluroniumtetrafluoroborat (TPTU) oder *O*-(7-Azabenzotriazol-1-yl)-*N*,*N*,*N*',*N*'- tetramethyluroniumhexafluorophosphat (HATU), oder 1-Hydroxybenzotriazol (HOBt), oder Benzotriazol-1-yloxytris(dimethylamino)-phosphoniumhexafluorophosphat (BOP), oder Benzotriazol-1-yloxytris(pyrrolidino)-phosphoniumhexafluorophosphat (PyBOP), oder *N*-Hydroxysuccinimid, oder Mischungen aus diesen, mit Basen.

Vorzugsweise wird die Kondensation mit HATU, Benzotriazol-1-yloxytris(pyrrolidino)-phosphoniumhexafluorophosphat (PyBOP) oder mit EDC in Gegenwart von HOBt durchgeführt.

Alternativ kann die Umsetzung nach Verfahren [A] über die Aktivierung der Säure in Formel (II) als Säurechlorid oder gemischtes Anhydrid erfolgen.

Die Umsetzung nach Verfahren [B] kann nach den in A. Da Silva, M. De Almeida, V. De Souza, M. Couri, Current Medicinal Chemistry, 2003, 10, 21-39 beschriebenen Verfahren durchgeführt werden.

Die Verseifung nach Verfahren [C] erfolgt im Allgemeinen in Wasser oder inerten Lösungsmitteln oder Mischungen von Wasser und inerten Lösungsmitteln, in Gegenwart einer Base, bevorzugt in einem Temperaturbereich von -30°C bis 100°C bei Normaldruck.

Inerte Lösungsmittel sind beispielsweise Halogenkohlenwasserstoffe wie Dichlormethan oder Trichlormethan, Kohlenwasserstoff wie Benzol, oder andere Lösungsmittel wie Nitromethan, Dioxan, Methanol, Tetrahydrofuran, Dimethylformamid oder Acetonitril. Ebenso ist es möglich, Gemische der Lösemittel einzusetzen. Besonders bevorzugt sind Dioxan, Methanol, Tetrahydrofuran oder Dimethylformamid.

Basen sind beispielsweise Alkalihydroxide oder -carbonate, wie z.B. Natrium-, Kaliumoder Lithiumhydroxid, Natrium- oder Kaliumcarbonat, oder -hydrogencarbonat.

Die Verbindungen der Formel (III) und (V) sind bekannt oder lassen sich nach bekannten Verfahren aus den entsprechenden Edukten synthetisieren.

Die Verbindungen der Formel (II) sind bekannt oder können hergestellt werden, indem Verbindungen der Formel

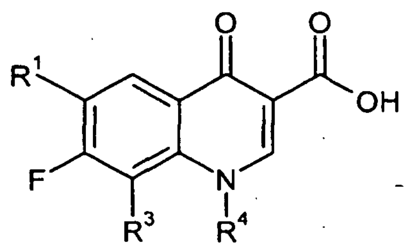

(VI),

in welcher

$R^1$, $R^3$ und $R^4$ die oben angegebene Bedeutung aufweisen,

mit Verbindungen der Formel (V) nach Verfahren [B] umgesetzt werden.

In den Verbindungen der Formel (VI) wird gegebenenfalls vor der Umsetzung mit Verbindungen der Formel (V) die Carbonsäure-Gruppe durch Bildung eines Bor-Esters aktiviert.

Die Verbindungen der Formel (VI) sind bekannt oder lassen sich nach bekannten Verfahren aus den entsprechenden Edukten synthetisieren, wie z.B. in A. Da Silva, M. De Almeida, V. De Souza, M. Couri, Current Medicinal Chemistry, 2003, 10, 21-39 beschrieben.

Die Verbindungen der Formel (IV) sind bekannt oder können hergestellt werden, indem Verbindungen der Formel (VI) mit Verbindungen der Formel (III) nach Verfahren [A] umgesetzt werden.

Die Herstellung der erfindungsgemäßen Verbindungen kann durch das folgende Syntheseschema verdeutlicht werden.

Syntheseschema:

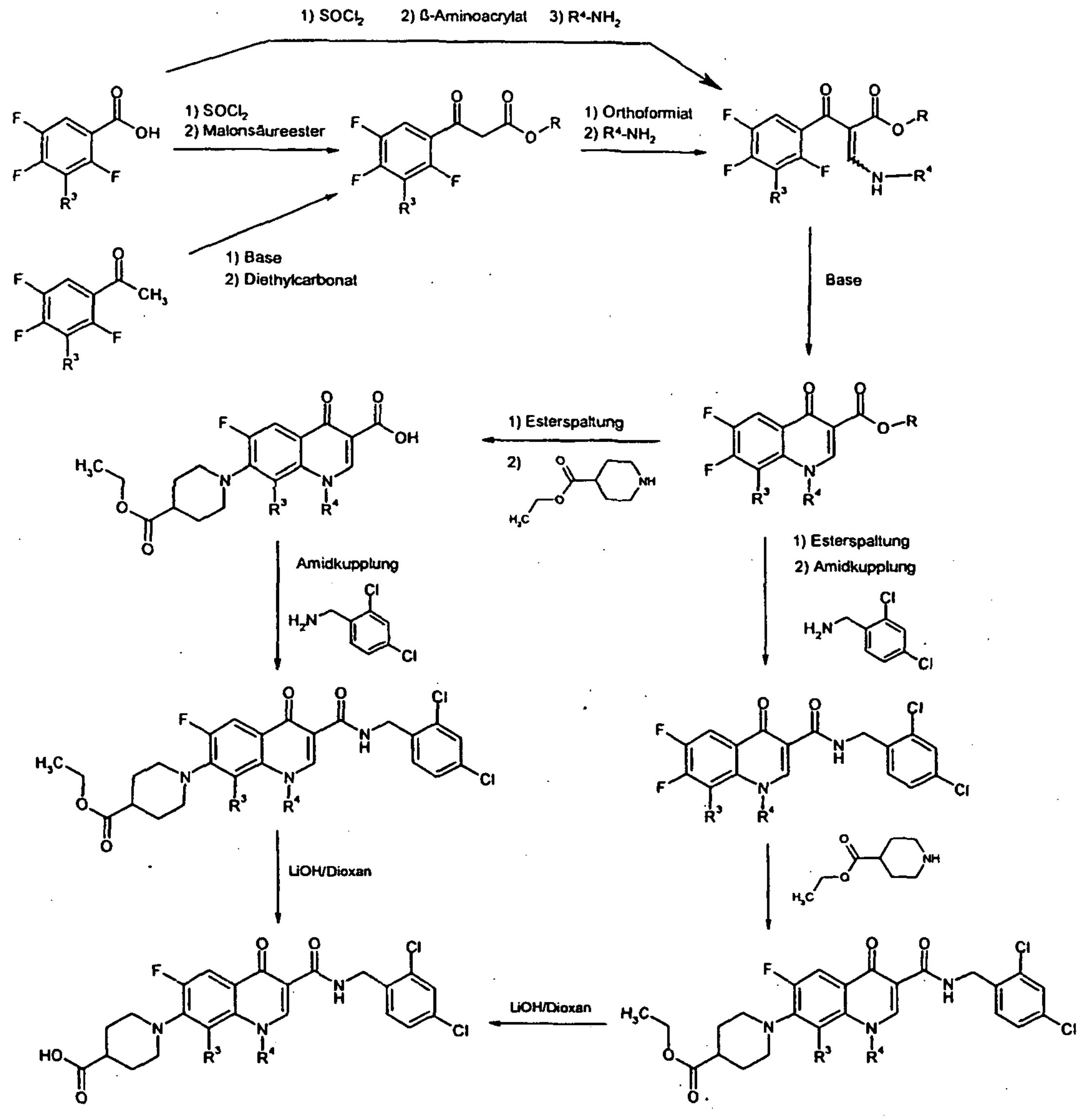


Die erfindungsgemäßen Verbindungen zeigen ein nicht vorhersehbares, überraschendes Wirkspektrum. Sie zeigen eine antivirale Wirkung gegenüber Vertretern der Gruppe der Herpes viridae (Herpesviren), vor allem gegenüber Cytomegaloviren (CMV), insbesondere gegenüber dem humanen Cytomegalovirus (HCMV).

Als Indikationsgebiete können beispielsweise genannt werden:

1) Behandlung und Prophylaxe von HCMV-Infektionen bei AIDS-Patienten (Retinitis, Pneumonitis, gastrointestinale Infektionen).

2) Behandlung und Prophylaxe von Cytomegalovirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.

3) Behandlung und Prophylaxe von HCMV-Infektionen bei Neugeborenen und Kleinkindern.

4) Behandlung einer akuten HCMV-Infektion bei Schwangeren.

5) Behandlung der HCMV-Infektion bei immunsupprimierten Patienten bei Krebs und Krebs-Therapie.

6) Behandlung von HCMV-positiven Krebspatienten mit dem Ziel, HCMVvermittelte Tumorprogression zu verringern (vgl. J. Cinatl , et al., FEMS Microbiology Reviews 2004, 28, 59-77).

Weiterer Gegenstand der vorliegenden Erfindung ist der Einsatz der erfindungsgemäßen Verbindungen zur Behandlung und/oder Prophylaxe von Krankheiten, vor allem von Infektionen mit Viren, insbesondere den vorstehend genannten Viren, und den dadurch hervorgerufenen Infektionskrankheiten. Unter einer Virusinfektion wird nachfolgend sowohl eine Infektion mit einem Virus als auch eine durch eine Infektion mit einem Virus hervorgerufene Krankheit verstanden.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Bevorzugt werden die erfindungsgemäßen Verbindungen zur Herstellung von Arzneimitteln verwendet, die zur Prophylaxe und/oder Behandlung von Infektionen mit einem Vertreter der Gruppe der Herpes viridae, besonders einem Cytomegalovirus, insbesondere dem humanen Cytomegalovirus, geeignet sind.

Weiterer Gegenstand der vorliegenden Erfindung ist die Verwendung einer antiviral wirksamen Menge der erfindungsgemäßen Verbindungen in einem Verfahren zur Behandlung und/oder Prophylaxe von Erkrankungen, insbesondere der zuvor genannten Erkrankungen.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt: antivirale Wirkstoffe wie Valganciclovir, Ganciclovir, Aciclovir, Cidofovir oder Foscarnet.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch, topisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nicht überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophilisate, Kapseln (beispielsweise Hartoder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme, Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei intravenöser Applikation Mengen von etwa 0.001 bis

10 mg/kg, vorzugsweise etwa 0.01 bis 5 mg/kg Körpergewicht zur Erzielung wirksamer Ergebnisse zu verabreichen, und bei oraler Applikation beträgt die Dosierung etwa 0.01 bis 25 mg/kg, vorzugsweise 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Die Prozentangaben der Ausbeuten der Beispiel-Verbindungen beziehen sich auf Mol.

A. Beispiele

Abkürzungen:

| | |
|---|---|
| ca. | circa |
| Boc | *tert*-Butoxycarbonyl |
| $CDCl_3$ | Deuterochloroform |
| DCI | direkte chemische Ionisation (bei MS) |
| DIEA | *N*,*N*-Diisopropylethylamin |
| DMSO | Dimethylsulfoxid |
| DMF | *N*,*N*-Dimethylformamid |
| d. Th. | der Theorie |
| EDC | *N*-(3-Dimethylaminoisopropyl)-*N*'-ethylcarbodiimid- hydrochlorid |
| EI | Elektronenstoß-Ionisation (bei MS) |
| ESI | Elektrospray-Ionisation (bei MS) |
| ges. | gesättigt |
| h | Stunde |
| HPLC | Hochdruck-, Hochleistungsflüssigchromatographie |
| HV | Hochvakuum |
| konz. | konzentriert |
| LC-MS | Flüssigchromatographie-gekoppelte Massenspektroskopie |
| LDA | Lithium-Diisopropylamid |
| min | Minuten |
| MS | Massenspektroskopie |
| MTBE | Methyl-*tert*-butylether |
| NMR | Kernresonanzspektroskopie |
| Pd-C | Palladium auf Kohle |
| proz. | prozentig |
| PyBOP | 1-Benzotriazolyloxy- tripyrrolidinophosphoniumhexafluorophosphat |
| quant. | quantitativ |
| RP-HPLC | Reverse Phase HPLC |
| RT | Raumtemperatur |
| $R_t$ | Retentionszeit (bei HPLC) |
| TFA | Trifluoressigsäure |
| THF | Tetrahydrofuran |

**Allgemeinen Methoden LC-MS und HPLC:**

**Methode 1 (LC-MS):** Instrument: Micromass Quattro LCZ mit HPLC Agilent Serie 1100; Säule: Phenomenex Synergi 2μ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50˚C; UV-Detektion: 208- 400 nm.

**Methode 2 (LC-MS):** Gerätetyp MS: Micromass ZQ; Gerätetyp HPLC: Waters Alliance 2795; Säule: Pheno-

menex Synergi 2μ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

**Methode 3 (LC-MS):** Gerätetyp MS: Micromass ZQ; Gerätetyp HPLC: HP 1100 Series; UV DAD; Säule: Phenomenex Synergi 2μ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

**Methode 4 (LC-MS):** Instrument: Micromass Platform LCZ mit HPLC Agilent Serie 1100; Säule: Thermo Hypersil GOLD 3μ 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 100%A → 0.2 min 100%A → 2.9 min 30%A → 3.1 min 10%A → 5.5 min 10%A; Ofen: 50°C; Fluss: 0.8 ml/min; UV-Detektion: 210 nm.

**Methode 5 (präparative HPLC, Ameisensäure):** Säule: Grom-Sil 120 ODS-4HE, 10 μm, SNr. 3331, 250 mm x 30 mm. Eluent A: Ameisensäure 0.1% in Wasser, Eluent B: Acetonitril; Fluss: 50 ml/min. Programm: 0-3 min: 10% B; 3-27 min: Gradient bis 95% B; 27-34 min: 95% B; 34.01-38 min: 10% B.

**Methode 6 (präparative HPLC, Salzsäure):** Säule: Grom-Sil 120 ODS-4HE, 10 μm, SNr. 3331, 250 mm x 30 mm. Eluent A: Salzsäure 0.1% in Wasser, Eluent B: Acetonitril; Fluss: 50 ml/min. Programm: 0-2 min 10% B, 3-43 min: Gradient bis 100% B, 43.01-45 min: 100% B.

**Methode 7 (analytische HPLC):** Instrument: HP 1100 mit DAD-Detektion; Säule: Kromasil 100 RP-18, 60 mm x 2.1 mm, 3.5 μm; Eluent A: 5 ml Perchlorsäure (70%ig) /l Wasser, Eluent B: Acetonitril; Gradient: 0 min 2%B, 0.5 min 2%B, 4.5 min 90%B, 9 min 90%B, 9.2 min 2%B, 10 min 2%B; Fluss: 0.75 ml/min; Säulen-Temperatur: 30°C; UV-Detektion: 210 nm.

**Methode 8 (analytische HPLC):** Instrument: HP 1100 mit DAD-Detektion; Säule: Kromasil 100 RP-18, 60 mm x 2.1 mm, 3.5 μm; Eluent A: 5 ml Perchlorsäure (70%ig) /l Wasser, Eluent B: Acetonitril; Gradient: 0 min 2%B, 0.5 min 2%B, 4.5 min 90%B, 6.5 min 90%B, 6.7 min 2%B, 7.5 min 2%B; Fluss: 0.75 ml/min; Säulen-Temperatur: 30°C; UV-Detektion: 210 nm.

Ausgangsverbindungen

**Beispiel 1A**

2-Brom-4-chlor-benzonitril

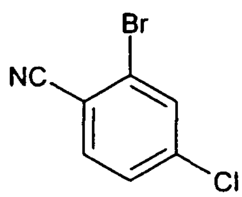

588 mg (2.5 mmol) 2-Brom-4-chlorbenzoesäure und 300 mg Harnstoff werden in Dichlormethan / Methanol gelöst und auf 364 mg Aluminiumoxid (neutral) einrotiert. Der Rückstand wird in der Mikrowelle bei 150°C insgesamt 60 min bestrahlt. Nach Abkühlung wird mit Essigsäureethylester und Wasser verrührt, abfiltriert und die wässrige Phase abgetrennt. Die organische Phase wird mit Natriumhydrogencarbonat-Lösung gewaschen, über Natriumsulfat getrocknet, einrotiert und dann im Hochvakuum getrocknet. Das Produkt (383 mg, 80% rein, 57% d. Th.) wird ohne zusätzliche Reinigung weiter umgesetzt.

$^1$H-NMR (300 MHz, $CDCl_3$): δ = 7.72 (d, 1H), 7.60 (d, 1H), 7.42 (dd, 1H).

**Beispiel 2A**

2-Chlor-4-(trifluormethoxy)-phenyl-trifluormethylsulfonat

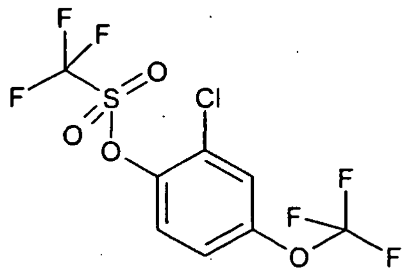

4.00 g 2-Chlor-4-trifluormethoxy-phenol in 50 ml Toluol und 50 ml einer 30%igen Kaliumphosphat-Lösung in Wasser werden bei 0°C vorgelegt, langsam mit 3.82 ml Trifluormethansulfonsäureanhydrid versetzt und 1.5 h bei RT gerührt. Die wässrige Phase wird abgetrennt und die organische Phase mit Wasser gewaschen, über Natriumsulfat getrocknet und eingeengt. Das Rohprodukt (6,2 g) wird ohne Reinigung zu Beispiel 3A weiter umgesetzt.

**Beispiel 3A**

2-Chlor-4-trifluormethoxy-benzonitril

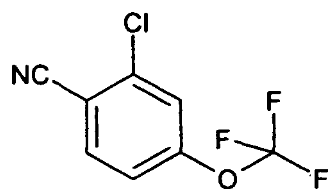

3.00 g der Verbindung aus Beispiel 2A werden in 12 ml entgastem DMF mit 2.04 g Zinkcyanid und 1.00 g Tetrakis(triphenylphosphin)palladium gelöst und unter Argon bei 120°C 2 h erhitzt. Nach Abkühlung wird die Reaktionsmischung mit Essigsäureethylester verdünnt und zweimal mit gesättigter Natriumhydrogencarbonat-Lösung, dann mit gesättigter Natriumchlorid-Lösung ausgeschüttelt. Die organische Phase wird über Natriumsulfat getrocknet und eingeengt. Der Rückstand wird durch Kieselgel-Chromatographie (Cyclohexan / Essigsäureethylester 10:1) gereinigt. Man erhält 880 mg (44% d. Th.) der Titelverbindung.

$^1$H-NMR (300 MHz, DMSO-$d_6$): δ = 7.62 (dd, 1H), 7.95 (d, 1H), 8.18 (d, 1H).

**Beispiel 4A**

2-Methyl-4-(trifluormethoxy)benzamid

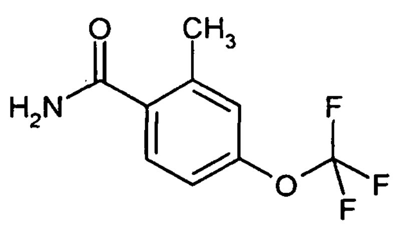

795 mg (3.61 mmol) 2-Methyl-4-(trifluorrnethoxy)benzoesäure werden mit 4 ml (54.8 mmol) Thionylchlorid und

einem Tropfen DMF 30 min unter Rückfluss erhitzt. Nach dem Abkühlen wird die Reaktionslösung langsam in eisgekühlte konz. wässrige Ammoniaklösung getropft. Der entstandene Niederschlag wird abgesaugt, in 30 ml Wasser aufgenommen und 1h bei 60°C gerührt. Man lässt abkühlen, filtriert den Feststoff ab und trocknet ihn im Vakuum. Ausbeute: 562 mg (71% d. Th.)

LC-MS (Methode 2): $R_t$ = 1.61 min.

MS (ESI$^+$): m/z = 220 $(M+H)^+$

$^1$H-NMR (400MHz, DMSO-$d_6$): δ = 7.79 (bs, 1H), 7.42-7.50 (m, 2H), 7.19-7.28 (m, 2H), 2.39 (s, 3H).

**Beispiel 5A**

2-Methyl-4-(trifluormethoxy)-benzylamin

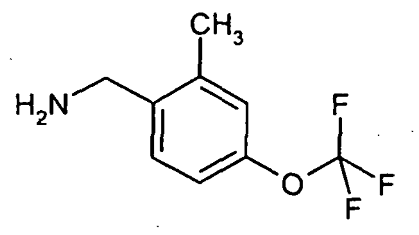

18.8 ml (18.8 mmol) Boran-THF-Komplex (1M) wird unter Argon und Eiskühlung vorgelegt. Es wird eine Lösung von 823 mg (3.76 mmol) 2-Methyl-4-(trifluormethoxy)benzamid (Beispiel 4A) in 80 ml THF zugetropft und anschließend 8 h unter Rückfluss gerührt. Unter Eiskühlung werden 80 ml 1N Salzsäure (bis zur Beendigung der Gasentwicklung) zugetropft und 1 h unter Rückfluss erhitzt. Anschließend wird das Reaktionsgemisch mit 1N Natronlauge alkalisch gestellt, dreimal mit Dichlormethan extrahiert, die vereinigten organischen Phasen über Natriumsulfat getrocknet und das Lösungsmittel im Vakuum entfernt. Man erhält ein Öl, das ohne weitere Reinigung weiter umgesetzt wird. Ausbeute: 732 mg (95% d. Th.).

LC-MS (Methode 3): $R_t$ = 1.41 min.

MS (ESI$^+$): m/z = 206 $(M+H)^+$

$^1$H-NMR (400MHz, $CDCl_3$): δ = 7.32-7.40 (m, 1H), 6.99-7.11 (m, 2H), 3.95-4.01 (m, 2H), 2.40 (s, 3H).

Durch Zugabe von überschüssigem HCl in Dioxan (4N) und Entfernung der flüchtigen Komponenten am Rotationsverdampfer erhält man das entsprechende Hydrochlorid.

**Beispiel 6A**

2-Brom-4-chlor-benzylamin

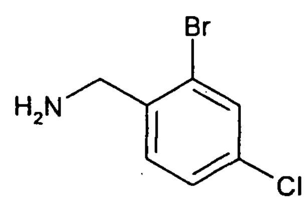

13.9 ml (13.9 mmol) Boran-THF Komplex (1 M) werden unter Eiskühlung vorgelegt. Langsam wird eine Lösung von 604 mg (2.8 mmol) 2-Brom-4-chlorbenzonitril (Beispiel 1A) in 60 ml THF dazugegeben. Danach wird die Reaktionsmischung 1 h unter Rückfluss erhitzt, abgekühlt und unter Eiskühlung mit 20 ml 1N Salzsäure tropfenweise versetzt. Zur Aufarbeitung wird die Lösung mit 1N Natronlauge alkalisch gestellt und mit Dichlormethan extrahiert. Die organische Phase wird über Natriumsulfat getrocknet und einrotiert. Das Rohprodukt (450 mg, ca. 73 % rein) wird ohne Reinigung weiter umgesetzt.

$^1$H-NMR (300 MHz, $CDCl_3$): δ = 3.89(s, 2H), 7.35-7.45 (m [ABM], 2H), 7.55 (d, 1H).

**Beispiel 7A**

2-Chlor-4-trifluormethoxy-benzylamin - Hydrochlorid

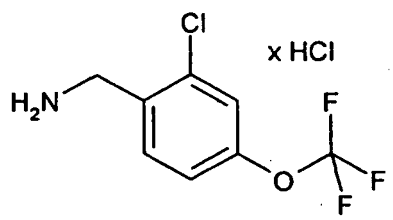

Die Herstellung erfolgt analog zu Beispiel 6A aus der Verbindung aus Beispiel 3A mit anschließender Behandlung mit 4N Salzsäure in Dioxan und Entfernung der flüchtigen Komponenten am Rotationsverdampfer.

$^1$H-NMR (300 MHz, DMSO-$d_6$): $\delta$ = 4.15 (s, 2H), 7.52 (d, 1H), 7.70 (s, 1H), 7.78 (d, 1H), 8.56 (bs, 3H).

**Beispiel 8A**

2,4-Dichlor-6-methyl-benzylamin - Hydrochlorid

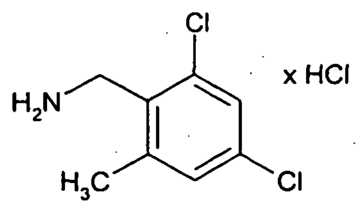

Die Herstellung erfolgt analog zu Beispiel 6A aus 2,4-Dichlor-6-methyl-benzonitril mit anschließender Behandlung mit 4N Salzsäure in Dioxan und Entfernung der flüchtigen Komponenten am Rotationsverdampfer.

$^1$H-NMR (300 MHz, DMSO-$d_6$): $\delta$ = 2.5 (s, 3H), 4.10 (s, 2H), 7.40 (s, 1H), 7.60 (s, 1H), 8.40 (bs, 3H).

LC-MS (Methode 4): $R_t$ = 2.44 min, MS (ES+) = 190 $(M+H)^+$.

**Beispiele 9A**

2-Methyl-4-trifluormethyl-benzylamin - Hydrochlorid

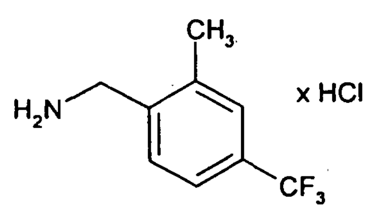

Die Herstellung erfolgt analog zu Beispiel 6A aus 2-Methyl-4-trifluormethylbenzonitril mit anschließender Behandlung mit 4N Salzsäure in Dioxan und Entfernung der flüchtigen Komponenten am Rotationsverdampfer.

$^1$H-NMR (300 MHz, DMSO-$d_6$): δ = 2.43 (s, 3H), 4.09 (s, 2H), 7.63 (s, 3H), 8.56 (br s, 3H).

**Beispiel 10A**

(all-cis)-N-Benzyl-3,5-dimethyl-4-hydroxy-piperidin

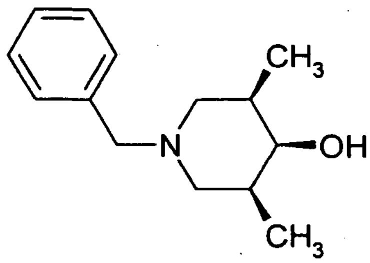

200 mg (0.60 mmol) des TFA-Salzes aus N-Benzyl-3,5-dimethyl-piperidin-4-on (Herstellung siehe: Journal of Medicinal Chemistry (1964), 7 (6), 726-728) werden in 2 ml Ethanol bei RT vorgelegt, mit 46 mg (1.21 mmol) Natriumborhydrid versetzt und über Nacht gerührt. 2 ml Wasser werden zugegeben und die Mischung zwischen Essigsäureethylester und einer gesättigten Natriumchlorid-Lösung ausgeschüttelt. Die wässrige Phase wird noch einmal mit Essigsäureethylester extrahiert, und die vereinigten organischen Phasen werden über Natriumsulfat getrocknet. Nach Entfernen des Lösungsmittels am Rotationsverdampfer erhält man 130 mg (98% d. Th.) der Titelverbindung, die direkt weiter umgesetzt wird.

**Beispiel 11A**

(all-cis)-3,5-Dimethyl-4-hydroxy-piperidin Hydrochlorid

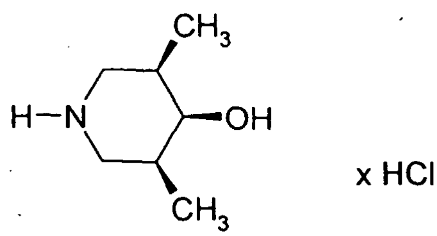

130 mg der Verbindung aus Beispiel 10A werden mit Pd 10%ig auf Kohle als Katalysator in 10 ml Methanol und 0.5 ml einer 4M Chlorwasserstoff-Lösung in Dioxan unter Normaldruck 24 h hydriert. Der Katalysator wird abfiltriert und das Filtrat im Vakuum von den Lösungsmitteln befreit. Der Rückstand wird im Hochvakuum getrocknet. Man erhält 98 mg (quant.) der Titelverbindung, die ohne weitere Reinigung eingesetzt wird.

MS (DCI ($NH_3$)): m/ z =147 (27) [M+NH4]$^+$, 130 (100) [M+H]$^+$.

**Beispiel 12A**

(4-Hydroxy-piperidin-4-yl)-essigsäureethylester Hydrochlorid

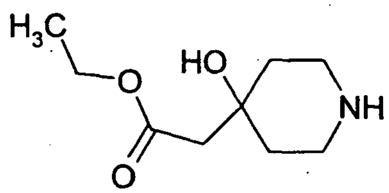

x HCl

3.01 ml (6.02 mmol) einer 2M Lösung von LDA in THF werden in 7 ml THF verdünnt und auf -78°C gekühlt. 540 µl (5.52 mmol) Essigsäureethylester werden zugegeben und die Lösung wird 30 min. bei -78°C gerührt. Eine Lösung von 1.00 g (5.01 mmol) N-*tert*-Butoxycarbonyl-piperidin-4-on in 10 ml THF wird zugetropft. Die Mischung wird noch 1 h bei -78°C gerührt und dann über Nacht langsam auf RT erwärmt. Eine gesättigte Ammoniumchloridlösung wird zugegeben und das Produkt mit Dichlormethan extrahiert. Nach Entfernen des Lösungsmittels erhält man den N-*tert* Butoxycarbonyl-4-hydroxypiperidin-4-yl)-essigsäureethylester. Dieses Rohprodukt wird durch HPLC (Methode 6) chromatographiert, wobei die *tert*-Butoxycarbonyl-Schutzgruppe durch die im Laufmittel enthaltene Salzsäure gespalten wird. Man erhält 478 mg (42% d. Th.) der Titelverbindung.

$^1$H-NMR (400 MHz, DMSO-$d_6$): $\delta$ = 1.20 (t, J = 7.1 Hz, 3H), 1.69-1.86 (m, 4H), 2.48 (s, 2H), 2.96-3.18 (m, 4H), 4.07 (q, J = 7.1 Hz, 2H), 5.05 (br. s, 1H).

**Beispiel 13A**

3-Oxo-2,8-diazaspiro[4,5]decan Hydrochlorid

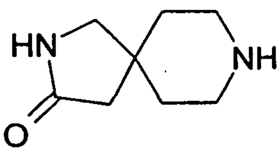

x HCl

Die Titelverbindung wird mit quantitativer Ausbeute durch Behandlung von 310 mg (1.22 mmol) 8-*tert*-Butoxycarbonyl-3-oxo-2,8-diazaspiro[4,5]decan (Herstellung siehe: Journal of Medicinal Chemistry (1995), 38(19), 3772-3780) mit 8 ml einer 4M Chlorwasserstoff-Lösung in Dioxan für 2 h bei RT und anschließender Entfernung der flüchtigen Komponenten am Rotationsverdampfer und im Hochvakuum erhalten.

MS (ES+): m/z = 155 $[M+H]^+$.

$^1$H-NMR (400 MHz, DMSO-$d_6$): $\delta$ = 1.71 (t, J = 7.1 Hz, 3H), 2.13 (s, 2H), 2.95-3.11 (m, 4H), 3.09 (s, 2H), 7.60 (br. s, 1H), 8.78 (br. s, 2H).

**Beispiel 14A**

8-Benzyl-2-oxa-4,8-diaza-spiro[4,5]decan-3-on

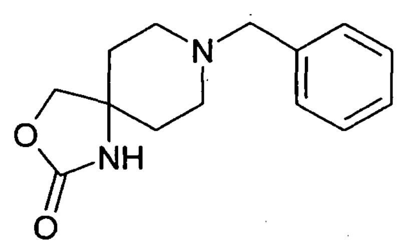

1.04 g (4.72 mmol) 4-Amino-1-benzyl-4-hydroxymethyl-piperidin (Herstellung siehe: Eur. J. Med. Chim. Ther. (1974) 9, 424-433) werden in 16 ml Dichlormethan suspendiert und mit 842 mg (5.2 mmol) Carbonyldiimidazol versetzt. Eine Lösung bildet sich mit fortschreitender Reaktion, die nach kompletter Umsetzung mit Dichlormethan verdünnt wird und zuerst mit Wasser, dann mit einer 5-prozentigen Natriumbicarbonat-Lösung und noch einmal mit Wasser gewaschen wird. Die organische Phase wird über Natriumsulfat getrocknet und am Rotationsverdampfer vom Lösungsmittel befreit. Man erhält 1.04 g der Titelverbindung als Rohprodukt, das so weiter umgesetzt wird.

LC-MS (Methode 4): $R_t$= 1.80 min, MS (ES+): m/z = 247 $(M+H)^+$

$^1$H-NMR (400 MHz, $CDCl_3$): δ = 1.74-1.84 (m, 4H), 2.40 (br.s, 4H), 3.50 (s, 2H), 4.12 (s, 2H), 5.90 (br.s, 1H), 7.22-7.35 (m, 5H).

**Beispiel 15A**

2-Oxa-4,8-diaza-spiro[4,5]decan-3-on

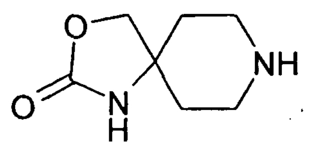

500 mg (1.61 mmol) der Verbindung aus Beispiel 14A werden mit 10 mg Pd (10% auf Kohle) in Methanol und 100 μl 4N Chlorwasserstoff in Dioxan bei Normaldruck und RT über Nacht hydriert. Der Katalysator wird abfiltriert und das Filtrat am Rotationsverdampfer vom Lösungsmittel befreit. Die freie Base lässt sich nicht durch Extraktion zwischen Essigsäureethylester und einer Natriumbicarbonat-Lösung reinigen. Deswegen wird die wässrige Phase am Rotationsverdampfer eingeengt und getrocknet und der Rückstand mit Methanol verrührt. Die Salze werden größtenteils abfiltriert. Nach Entfernen des Lösungsmittels aus dem Filtrat erhält man 360 mg Rohprodukt, das ohne weitere Reinigung eingesetzt wird.

MS (DCI ($NH_3$)): m/z = 174 $(M+NH_4)^+$, 157 $(M+H)^+$.

$^1$H-NMR (400 MHz, MeOD): δ = 1.68-1.80 (m, 4H), 2.73 (m, 2H), 2.90 (m, 2H), 4.19 (s, 2H).

**Beispiel 16A**

(*S*)-(1-*tert*-Butoxycarbonyl-piperidin-3-yl)-essigsäureethylester

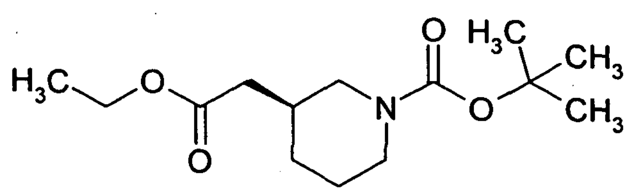

1 g (5.84 mmol) racemischer Piperidin-3-yl-essigsäureethylester werden in Dichlormethan vorgelegt und mit 1.4 g (6.42 mmol) Di-*tert*-butyl-dicarbonat versetzt. Die Lösung wird bei RT gerührt bis zum Ende der Gasentwicklung und am Rotationsverdampfer vom Lösungsmittel befreit. Die beiden Enantiomeren werden mittels chiraler HPLC (Daicel Chirapak AD-H, 5 μm, 250 mm x 20 mm, Laufmittel Isohexan / 2-Propanol 95 : 5) getrennt. Das zuerst eluierte Produkt ($R_t$ = 5.10 min) ist das (S)-Enantiomer (Beispiel 16A) (311 mg, 20% d. Th.). Das später eluierte Produkt ($R_t$ = 5.34 min) ist das (R)-Enantiomer (Beispiel 17A) (290 mg, 18% d. Th.). Die absolute Stereochemie ist nachträglich durch Röntgenstruktur von Beispiel 73 zugeordnet worden.

**Beispiel 17A**

*(R)*-(1-*tert*-Butoxycarbonyl-piperidin-3-yl)-essigsäureethylester

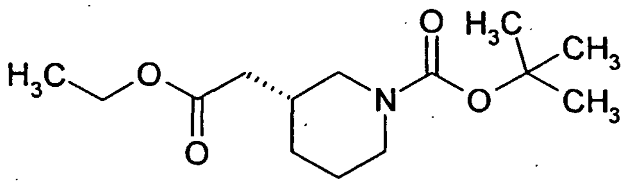

Herstellung: siehe unter Beispiel 16A.

**Beispiel 18A**

*(S)*-Piperidin-3-yl-essigsäureethylester Hydrotrifluoracetat

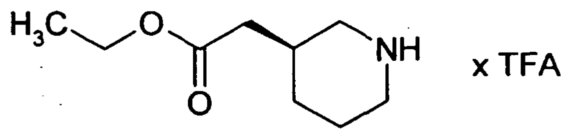

280mg (1.03 mmol) *(S)*-(1-*tert*-Butoxycarbonyl-piperidin-3-yl)-essigsäureethylester (Beispiel 16A) werden mit 2 ml Dichlormethan und 2 ml Trifluoressigsäure 1 h bei RT verrührt. Die flüchtigen Komponenten werden am Rotationsverdampfer entfernt und der Rückstand am Hochvakuum getrocknet. Das erhaltene Öl (290 mg, 99% d. Th.) wird so weiter umgesetzt.

MS (ES+): m/z = 172 $[M+H]^+$.

$^1$H-NMR (400 MHz, $CDCl_3$): δ = 1.28 (t, 3H), 1.30 (m, 1H), 1.80-2.00 (m, 3H), 2.28-2.35 (m, 3H), 2.70 (br.q, 1H), 2.87 (br.q, 1H), 3.42 (d, 1H), 3.51 (d, 1H), 4.13 (q, 2H), 8.50 (br s, 1H), 9.10 (br s, 1H).

**Beispiel 19A**

*(R)*-Piperidin-3-yl-essigsäureethylester Hydrotrifluoracetat

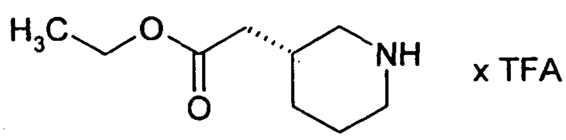

290 mg (1.07 mmol) *(R)*-(1-*tert*-Butoxycarbonyl-piperidin-3-yl)-essigsäureethylester (Beispiel 17A) werden mit 2 ml Dichlormethan und 2 ml Trifluoressigsäure 1 h bei RT verrührt. Die flüchtigen Komponenten werden am Rotationsverdampfer entfernt und der Rückstand am Hochvakuum getrocknet. Das erhaltene Öl (301 mg, 99% d. Th.) wird so weiter umgesetzt.

MS (ES+): m/z = 172 $[M+H]^+$.

$^1$H-NMR (400 MHz, $CDCl_3$): δ = 1.28 (t, 3H), 1.30 (m, 1H), 1.80-2.00 (m, 3H), 2.32 (br s, 3H), 2.70 (m, 1H), 2.87 (m, 1H), 3.42 (d, 1H), 3.50 (d, 1H), 4.13 (q, 2H), 8.72 (br s, 1H), 9.30 (br s, 1H).

**Beispiel 20A**

3-[(2,2,2-Trifluorethyl)amino]-2-(2,4,5-trifluor-3-methoxybenzoyl)acrylsäureethylester (E+Z)

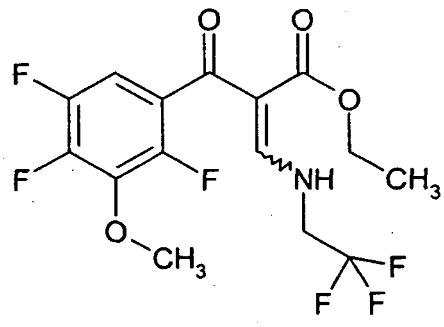

2.00 g (5.79 mmol) 3-Oxo-3-(2,4,5-trifluor-3-methoxyphenyl)-propansäureethylester (Herstellung siehe Journal of Medicinal Chemistry (1995), 38 (22), 4478-87) werden in 3.8 ml (4.14 g, 40.55 mmol) Essigsäureanhydrid und 4.82 ml (4.29 g, 28.96 mmol) Orthoameisensäuretriethylester für 2 h unter Rückfluss gerührt. Das Lösungsmittel wird anschließend am Rotationsverdampfer vollständig entfernt und der Rückstand in 10 ml Ethanol gelöst. Zur eisgekühlten Lösung werden 1.03 g (10.43 mmol) 2,2,2-Trifluor-1-aminoethan zugetropft. Die Mischung wird auf Raumtemperatur gebracht und bei dieser Temperatur über Nacht nachgerührt. Zur Aufarbeitung wird das Lösungsmittel entfernt und der Rückstand ohne Reinigungsschritte als Rohprodukt weiter umgesetzt (Ausbeute als quantitativ angenommen).

LC-MS (Methode 2): $R_t$= 2.37 min, MS (ES+) = 386 $(M+H)^+$.

Die folgenden Beispiele 21A bis 25A werden analog zu Beispiel 20A aus den entsprechenden Aminen hergestellt.

| Beispiel-Nr. | Struktur | Analytikdaten LC-MS (Methode)/ Meßwerte |
|---|---|---|
| 21A<br>(R)-Enantiomer | 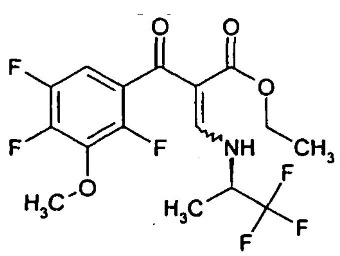 | LC-MS (Methode 1): $R_t$ = 2.46 min MS (ES+): m/z = 400 $(M+H)^+$ |
| 22A<br>racemisch | 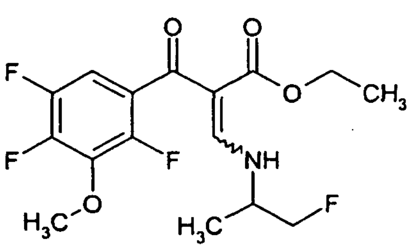 | LC-MS (Methode 2): $R_t$ = 2.28 min MS (ES+): m/z = 364 $(M+H)^+$ |

(fortgesetzt)

| Beispiel-Nr. | Struktur | Analytikdaten LC-MS (Methode)/ Meßwerte |
|---|---|---|
| 23A | 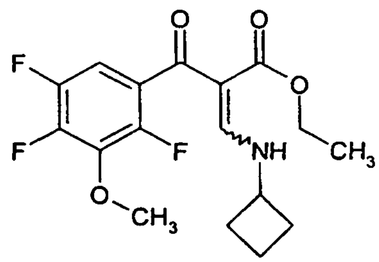 | LC-MS (Methode 3): $R_t$ = 2.72 min MS (ES+): m/z = 358 $(M+H)^+$ |
| 24A | 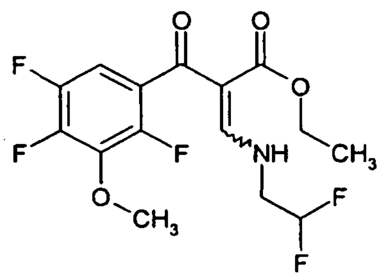 | LC-MS (Methode 2): $R_t$ = 2.22 min MS (ES+): m/z = 368 $(M+H)^+$ |
| 25A (1S,2R)-Enantiomer | 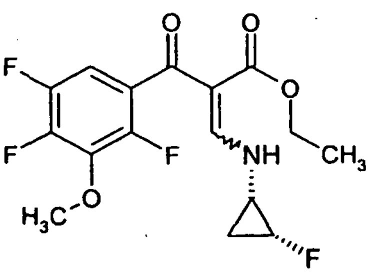 | LC-MS (Methode 1): $R_t$ = 2.40 min MS (ES+): m/z = 382 $(M+H)^+$ |

**Beispiel 26A**

6,7-Difluor-8-methoxy-4-oxo-1-(2,2,2-trifluorethyl)-1,4-dihydrochinolin-3-carbonsäureethylester

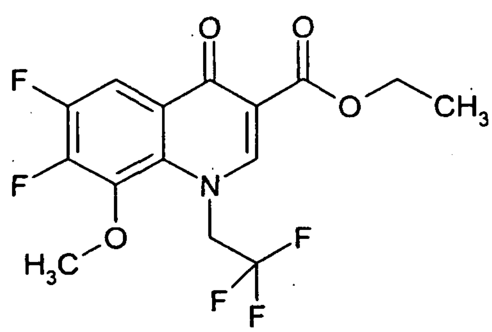

Unter Argon-Atmosphäre und Eiskühlung werden 0.32 g (8.11 mmol) 60%iges Natriumhydrid in 5 ml Tetrahydrofuran vorgelegt und eine Lösung von 2.23 g (5.79 mmol) der Verbindung aus Beispiel 20A in 15 ml Tetrahydrofuran wird langsam zugetropft. Die Mischung wird anschließend auf Raumtemperatur erwärmt, bei dieser Temperatur 2 h nachgerührt und über Nacht stehen gelassen. Zur Aufarbeitung werden 2 ml Essigsäure zugetropft, 5 min nachgerührt,

mit Essigsäureethylester verdünnt, mehrmals mit Wasser und einmal mit gesättigter Natriumhydrogencarbonat-Lösung gewaschen, die organische Phase über Magnesiumsulfat getrocknet, filtriert und das Lösungsmittel wird vollständig am Rotationsverdampfer entfernt. Das Rohprodukt wird säulenchromatographisch über Kieselgel 60 (Laufmittel: Dichlormethan/Methanol 100/1 → 100/2) vorgereinigt. Zur Feinreinigung wird die Hälfte des Rohprodukts über die präparative RP-HPLC (Methode 5) gereinigt (0.83 g reines Produkt). Die andere Hälfte wird aus Acetonitril umkristallisiert (1.02 g). Die Gesamtausbeute beträgt damit 1.85 g (87% d. Th.)

HPLC (Methode 8): $R_t$= 4.34 min,

MS (DCI ($NH_3$)) = 366 $(M+H)^+$.

$^1$H-NMR (300 MHz, $CDCl_3$): δ = 1.41 (t, 3H), 4.15 (s, 3H), 4.41 (q, 2H), 5.23 (q, 2H), 8.11 (dd, 1H), 8.33 (s, 1H).

Die in der folgenden Tabelle aufgeführten Beispiele 27A bis 31A werden analog zu Beispiel 26A hergestellt.

| **Beispiel-Nr.** | **Struktur** | **Edukt Beispiel-Nr.** | **Analytikdaten LC-MS (Methode)/ Meßwerte HPLC (Methode)/ Meßwerte MS (Methode)/ Meßwerte** |
|---|---|---|---|
| 27A (R)-Enantiomer | 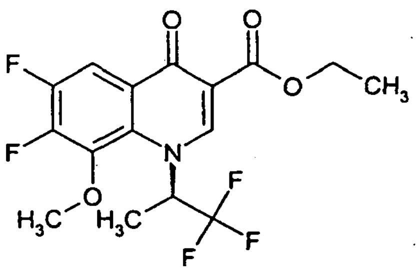 | 21A | LC-MS (Methode 1): $R_t$= 2.22 min MS (ES+): m/z = 380 $(M+H)^+$ |
| 28A racemisch | 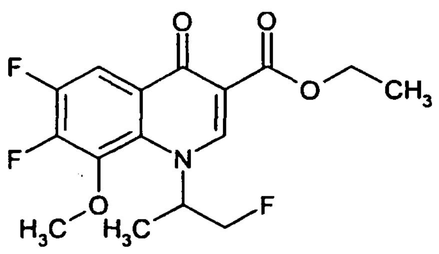 | 22A | HPLC (Methode 8): $R_t$=4.11min MS (DCI ($NH_3$)): m/z = 344 $(M+H)^+$ |
| 29A | 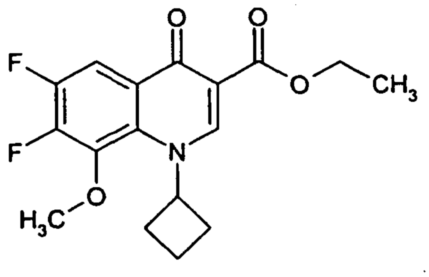 | 23A | LC-MS (Methode 3): $R_t$ = 2.33 min MS (ES+): m/z = 338 $(M+H)^+$ |
| 30A | 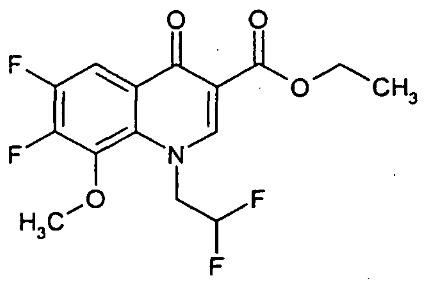 | 24A | LC-MS (Methode 2): $R_t$= 1.83 min MS (ES+): m/z = 348 $(M+H)^+$ |

(fortgesetzt)

| Beispiel-Nr. | Struktur | Edukt Beispiel-Nr. | Analytikdaten LC-MS (Methode)/ Meßwerte HPLC (Methode)/ Meßwerte MS (Methode)/ Meßwerte |
|---|---|---|---|
| 31A (1S,2R)-Enantiomer | 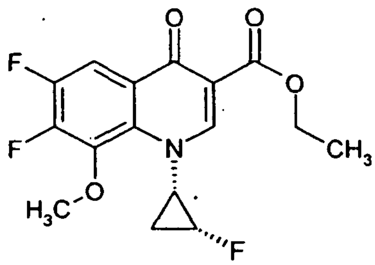 | 25A | LC-MS (Methode 2): $R_t$=1.76 min MS (ES+): m/z = 342 $(M+H)^+$ |

**Beispiel 32A**

6,7-Difluor-8-methoxy-4-oxo-1-(2,2,2-trifluorethyl)-1,4-dihydrochinolin-3-carbonsäure

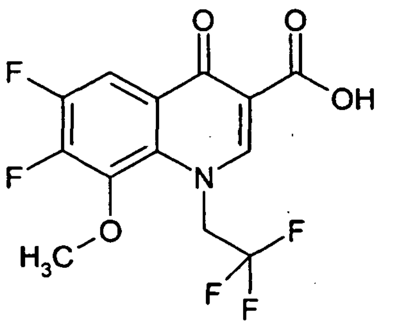

800 mg (2.19 mmol) der Verbindung aus Beispiel 26A werden in einer Mischung aus 25 ml Essigsäure-Wasser-Schwefelsäure 12:8:1 vorgelegt und über Nacht unter Rückfluss gerührt. Zur Aufarbeitung wird das Lösungsmittel am Rotationsverdampfer größtenteils entfernt, der Rückstand unter Eiskühlung vorsichtig mit gesättigter Natriumhydrogencarbonat-Lösung auf pH 3 gestellt, die Suspension mit Wasser verdünnt und der Niederschlag abgesaugt. Nach dem Trocknen des Filterrückstandes im Hochvakuum werden 575 mg der Titelverbindung erhalten.

LC-MS (Methode 3): $R_t$ = 2.41 min, MS (ES+) = 338 $(M+H)^+$.

$^1$H-NMR (300 MHz, $CDCl_3$): $\delta$ = 4.21 (s, 3H), 5.37 (q, 2H), 8.11 (dd, 1H), 8.62 (s, 1H), 14.05 (bs, 1H).

Die folgenden Beispiele 33A bis 37A werden analog zu Beispiel 32A hergestellt.

| Beispiel-Nr. | Struktur | Edukt | Analytikdaten LC-MS (Methode)/ Meßwerte HPLC (Methode)/ Meßwert MS (Methode)/ Meßwert |
|---|---|---|---|
| **33A** (R)-Enantiomer | 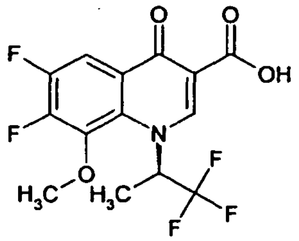 | 27A | LC-MS (Methode 3): $R_t$ = 2.47 min MS (ES+): m/z = 352 $(M+H)^+$ |
| **34A** racemisch | 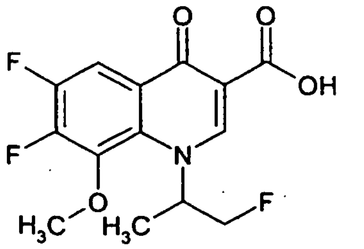 | 28A | HPLC (Methode 8): $R_t$ = 4.17 min MS (ESI+): m/z = 316 $(M+H)^+$ |
| **35A** | 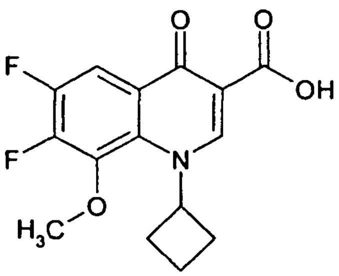 | 29A | LC-MS (Methode 3): $R_t$ = 2.35 min MS (ES+): m/z = 310 $(M+H)^+$ |
| **36A** | 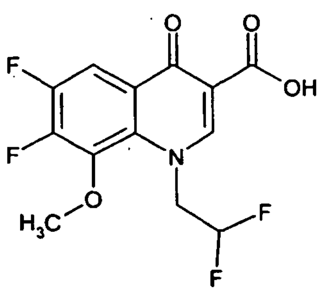 | 30A | HPLC (Methode 7): $R_t$ = 4.15 min MS (DCI ($NH_3$)): m/z = 337 $(M+NH_4)^+$ |
| **37A** (1S,2R)-Enantiomer | 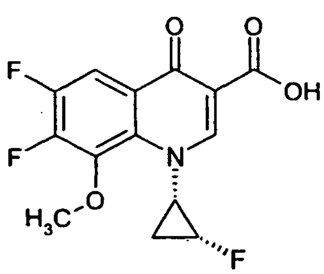 | 31A | LC-MS (Methode 2): $R_t$ = 1.84 min MS (ES+): m/z = 314 $(M+H)^+$ |

**Beispiel 38A**

[6,7-Difluor-8-methoxy-4-oxo-1-(2,2,2-trifluorethyl)-1,4-dihydrochinolin-3-yl]carbonyl-difluorborat

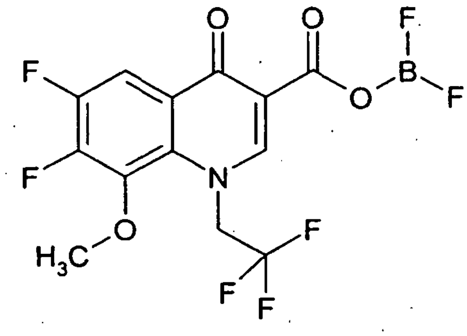

1.45 g (4.30 mmol) der Verbindung aus Beispiel 32A werden in 10 ml Tetrahydrofuran vorgelegt, anschließend mit 6.81 ml (7.63 g, 53.75 mmol) Bortrifluorid-Diethylether-Komplex versetzt und über Nacht bei 70˚C nachgerührt. Zur Aufarbeitung wird das auf Raumtemperatur abgekühlte Reaktionsgemisch mit 50 ml Diethylether versetzt, 20 min verrührt und der ausfallende Niederschlag wird abgesaugt. Nach dem Trocknen des Feststoffs im Hochvakuum werden 1150 mg der Titelverbindung erhalten, die ohne Reinigung weiter umgesetzt wird.

HPLC (Methode 7): $R_t$ = 4.25 min,

MS (DCI ($NH_3$)) = 402 $(M+NH_4)^+$.

$^1$H-NMR (300 MHz, DMSO-$d_6$): δ = 4.21 (s, 3H), 6.12 (q, 2H), 8.38 (dd, 1H), 9.66 (s, 1H).

Die folgenden Beispiele 39A bis 43A werden analog zu Beispiel 38A hergestellt.

| Beispiel-Nr. | Struktur | Edukt | Analytikdaten LC-MS (Methode)/ Meßwerte |
|---|---|---|---|
| **39A**<br>(R)-Enantiomer | 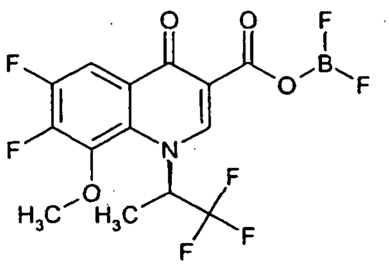 | 33A | LC-MS (Methode 2):<br>$R_t$ = 1.98 min<br>MS (ES+): m/z = 400 $(M+H)^+$ |
| 40A | 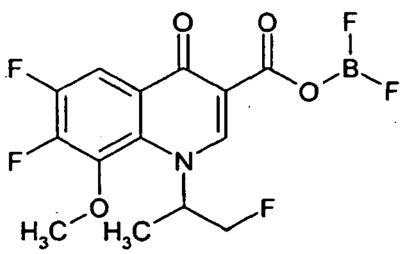 | 34A | LC-MS (Methode 1):<br>$R_t$ = 1.96 min<br>MS (ES+): m/z = 364 $(M+H)^+$ |
| **41A** | 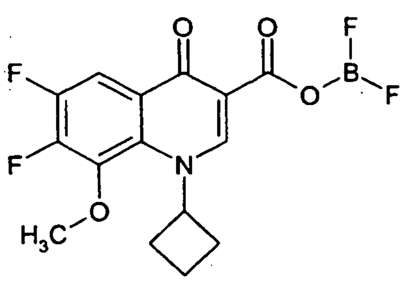 | 35A | LC-MS (Methode 1):<br>$R_t$ = 1.92 min<br>MS (ES+): m/z = 358 $(M+H)^+$ |

(fortgesetzt)

| Beispiel-Nr. | Struktur | Edukt | Analytikdaten LC-MS (Methode)/ Meßwerte |
|---|---|---|---|
| **42A** | 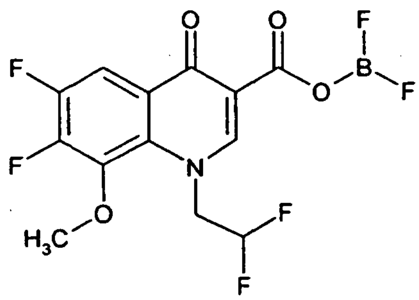 | 36A | LC-MS (Methode 3):<br>$R_t$ = 2.09 min<br>MS (ES+): m/z = 368 $(M+H)^+$ |
| **43A**<br>(1S,2R)-Enantiomer | 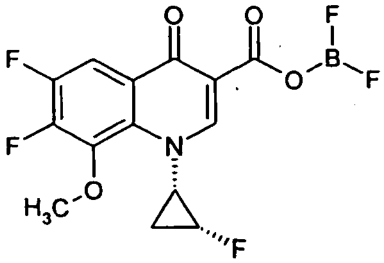 | 37A | LC-MS (Methode 2):<br>$R_t$ = 1.74 min<br>MS (ES+): m/z = 362 $(M+H)^+$ |

**Beispiel 44A**

[6,7-Difluor-1-{(1R,2*S*)-2-fluorcyclopropylamino}-8-methoxy-4-oxo-1,4-dihydrochinolin-3-yl]carbonyl-difluorborat

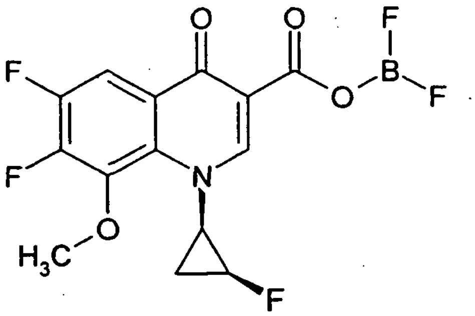

Aus 750 mg (2.39 mmol) 6,7-Difluor-1-{(1R,2S)-2-fluorcyclopropylamino}-8-methoxy-4-oxo-1,4-dihydrochinolin-3-carbonsäure (Herstellung: siehe WO 96/01262) und 4.08 g (29 mmol) $BF_3$-Etherat erhält man analog zu Beispiel 38A 582 mg der Titelverbindung.
LC-MS (Methode 2): $R_t$ = 1.74 min
MS (ES+): m/z = 362 $(M+H)^+$,
**[0185]** $^1$H-NMR (400 MHz, $CDCl_3$): δ = 9.17 (s, 1H), 8.15 (t, J = 8.5 Hz, 1H), 5.01 (dm, J = 63 Hz, 1H), 4.43 (m, 1H), 4.29 (s, 3H), 2.00-1.75 (m, 3H).

**Beispiel 45A**

8,9-Difluor-3-methyl-7-oxo-2,3-dihydro-7H-[1,4]oxazino[2,3,4-ij]chinolin-6-carbonyldifluorborat

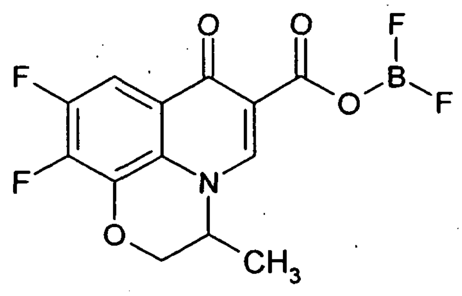

Aus 1.0 g 8,9-Difluor-3-methyl-7-oxo-2,3-dihydro-7H-[1,4]oxazino[2,3,4-ij]chinolin-6-carbonsäure (Herstellung: siehe Journal of Medicinal Chemistry 1992, 35 (4), 611) und 1.51 g (3 Äq.) $BF_3$-Etherat wird nach dem gleichem Verfahren wie für Beispiel 38A beschrieben 1.0 g (85% d. Th.) der Titelverbindung isoliert.
MS (ESI pos) : m/z = 330 $(M+H)^+$.
$^1$H-NMR (400 MHz, DMSO-$d_6$): δ = 9.64 (s, 1H), 8.15 (dd, J = 7.5, 10.0 Hz, 1H), 5.32 (m, 1H), 4.82 (d, J = 11.6 Hz, 1H), 4.57 (dd, J = 11.5 Hz, 1.8 Hz, 1H), 1.56 (d, J = 7.0 Hz, 3H).

**Beispiel 46A**

7-(4-Ethoxycarbonyl-piperidin-1-yl)-6-fluor-1-(2-fluorethyl)-8-methoxy-4-oxo-1,4-dihydrochinolin-3-carbonsäure

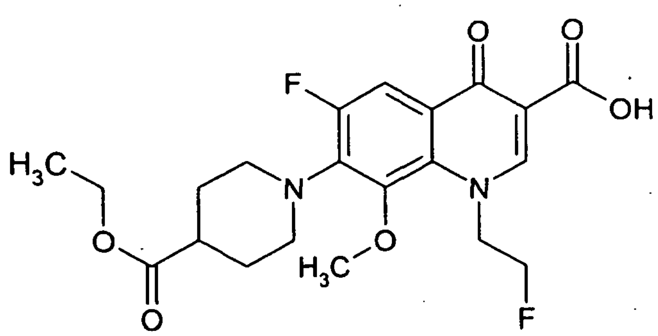

155 mg (0.38 mmol) 6,7-Difluor-1-(2-fluorethyl)-8-methoxy-4-oxo-1,4-dihydrochinolin-3-yl]-carbonyl-difluorborat (Herstellung: siehe EP0241206) und 120 mg (0.76 mmol, 2 Äq.) Piperidin-4-carbonsäureethylester werden in 3 ml Acetonitril 3 h bei 50°C gerührt. Das Lösungsmittel wird am Rotationsverdampfer entfernt und der Rückstand mit 0.56 ml Ethanol und 0.53 ml Triethylamin versetzt. Diese Lösung wird 2 h auf Rückfluss erhitzt. Die Lösungsmittel werden am Rotationsverdampfer entfernt, der Rückstand in wenig DMSO aufgenommen und über präparative HPLC (Methode 5) getrennt. Nach Einengen der entsprechenden Fraktionen am Rotationsverdampfer und Trocknung im Hochvakuum werden 100 mg (59% d. Th.) der Titelverbindung erhalten.
LC-MS (Methode 2): $R_t$ = 2.30 min, MS (ES+) : m/z = 439 $(M+H)^+$.
$^1$H-NMR (500 MHz, $CDCl_3$): δ = 14.67 (s, 1H), 8.59 (s, 1H), 7.98 (d, J = 12.1 Hz, 1H), 4.83 (dt, J = 25.6, 4 Hz, 2H), 4.71 (dt, J = 47 Hz, 4 Hz, 2H), 4.19 (q, J = 7.1 Hz, 2H), 3.83 (s, 3H), 3.51 (br. d, J = 12 Hz, 2H), 3.23 (br. t, J = 12 Hz, 2H), 2.54 (m, 1H), 2.05 (br.d, J = 10 Hz, 2H), 1.90 (m, 2H), 1.28 (t, J = 7.1 Hz, 3H).

**Beispiel 47A**

7-(4-Ethoxycarbonyl-piperidin-1-yl)-6-fluor-8-methoxy-4-oxo-1-(2,2,2-tritluorethyl)-1,4-dihydrochinolin-3-carbonsäure

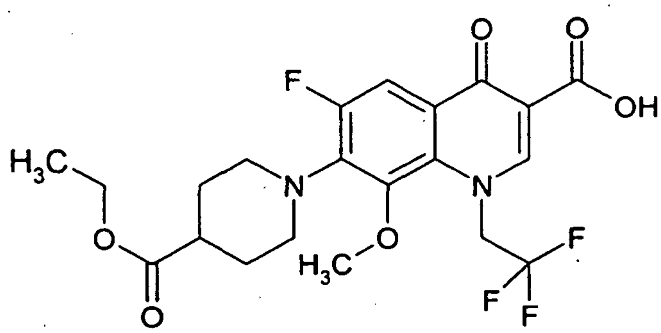

Nach dem gleichen Verfahren wie für Beispiel 46A erhält man aus 800 mg (2.08 mmol) [6,7-Difluor-8-methoxy-4-oxo-1-(2,2,2-trifluorethyl)-1,4-dihydrochinolin-3-yl]carbonyldifluorborat (Beispiel 38A) und 653 mg (4.15 mmol) Piperidin-4-carbonsäureethylester 625 mg (63% d. Th.) der Titelverbindung.

HPLC (Methode 8): $R_t$ = 4.97 min.

MS (ES+): m/z = 475 $[M+H]^+$.

$^1$H-NMR (400 MHz, $CDCl_3$): $\delta$ = 14.40 (s, 1H), 8.54 (s, 1H), 7.93 (d, J = 12.1 Hz, 1H), 5.31 (q, J = 7.9 Hz, 2H), 4.19 (q, J = 7.1 Hz, 2H), 3.86 (s, 3H), 3.53 (br. d, J = 12.5 Hz, 2H), 3.23 (br. t, J = 12 Hz, 2H), 2.54 (m, 1H), 2.09-2.01 (m, 2H), 1.97-1.85 (m, 2H), 1.29 (t, J = 7.1 Hz, 3H).

Alternatives Verfahren für größere Mengen: 15.5 g (40.3 mmol) der Verbindung aus Beispiel 38A und 12.66 g (80.52 mmol) Piperidin-4-carbonsäureethylester werden in 290 ml Acetonitril über Nacht bei 50°C gerührt. Das Lösungsmittel wird vollständig am Rotationsverdampfer entfernt, der Rückstand wird mit einer Mischung aus 250 ml Ethanol und 125 ml Triethylamin 1 h unter Rückfluss gerührt. Das Lösungsmittel wird am Rotationsverdampfer entfernt und der Rückstand in Methanol gelöst. Diese Lösung wird in 1000 ml 1N Salzsäure eingerührt. Das ausgefallene Produkt wird abgesaugt und im Hochvakuum getrocknet. Man erhält 19.1 g (74% d. Th.) der Titelverbindung.

Die folgenden Beispiele 48A bis 54A werden analog zur Arbeitsvorschrift von Beispiel 46A hergestellt. Wenn kein Edukt für den Piperidin-Teil eingegeben ist, ist das eingesetzte substituierte Piperidin kommerziell erhältlich.

| Beispiel-Nr. | Struktur | Edukte | Analytikdaten LC-MS (Methode)/ Meßwerte |
|---|---|---|---|
| **48A**<br>(R)-Enantiomer | 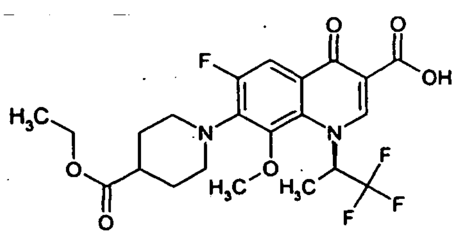 | 39A | LC-MS (Methode 3):<br>$R_t$ = 2.92 min.<br>MS (ES+): m/z = 489 $(M+H)^+$ |
| **49A**<br>racemisch | 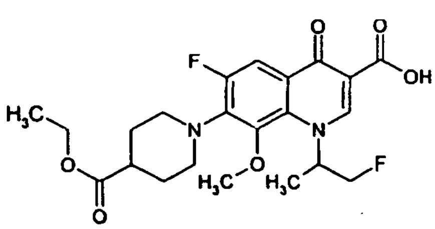 | 40A | LC-MS (Methode 1):<br>$R_t$ = 2.57 min.<br>MS (ES+): m/z = 453 $(M+H)^+$ |

(fortgesetzt)

| Beispiel-Nr. | Struktur | Edukte | Analytikdaten LC-MS (Methode)/ Meßwerte |
|---|---|---|---|
| **50A** | 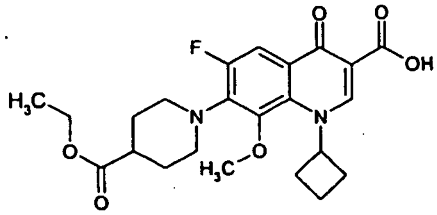 | 41A | LC-MS (Methode 1): $R_t$ = 2.71 min. MS (ES+): m/z = 447 $(M+H)^+$ |
| **51A** | 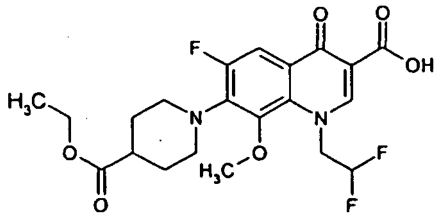 | 42A | LC-MS (Methode 3): $R_t$ = 2.69 min. MS (ES+): m/z = 457 $(M+H)^+$ |
| **52A** (1R, 2S)-Enantiomer | 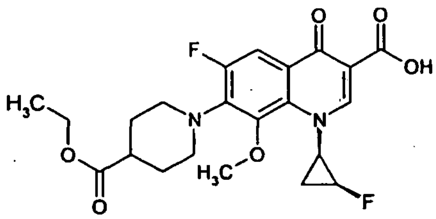 | 44A | LC-MS (Methode 3): $R_t$ = 2.74 min MS (ES+): m/z = 451 $(M+H)^+$ |
| **53A** racemisch | 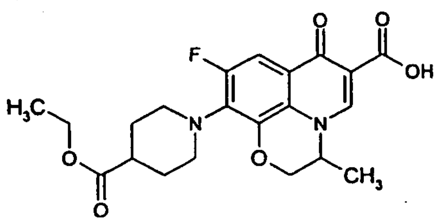 | 45A | LC-MS (Methode 1): $R_t$ = 2.44 min MS (ES+): m/z = 419 $(M+H)^+$ |
| **54A** | 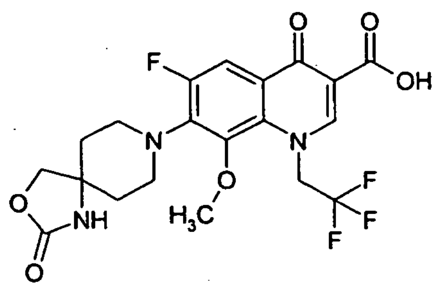 | 38A + 15A | LC-MS (Methode 1): $R_t$ = 2.03 min MS (ES+): m/z = 474 $(M+H)^+$ |

**Beispiel 55A**

7-[(*3S*)-3-(2-Ethoxy-2-oxoethyl)-piperidin-1-yl]-6-fluor-8-methoxy-4-oxo-1-(2,2,2-trifluorethyl)-1,4-dihydrochinolin-3-carbonsäure

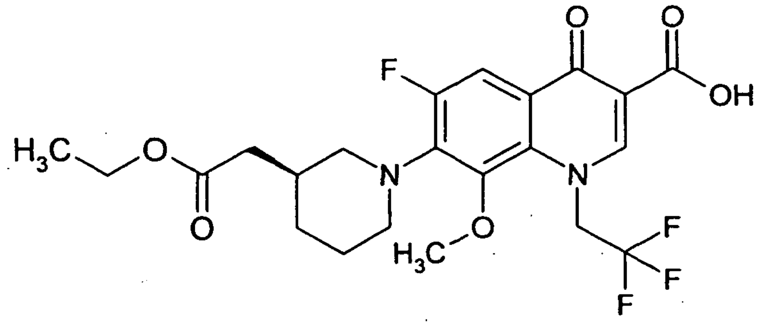

290 mg (1.05 mmol) der Verbindung aus Beispiel 18A (S-Enantiomer) werden in 8 ml Acetonitril bei RT vorgelegt, mit 177 µl N,N-Diisopropylethylamin (1.1 Äq.), dann mit 356 mg (0.92 mmol) der Verbindung aus Beispiel 38A versetzt. Die Mischung wird bei 50°C gerührt. Nach einer Stunde werden 80 µl N,N-Diisopropylethylamin (0.5 Äq.) und nach 2 Stunden erneut 80 µl N,N-Diisopropylethylamin (0.5 Äq.) zugegeben. Die Mischung wird weiter bei 50°C über Nacht rühren lassen, dann am Rotavapor von den flüchtigen Komponenten befreit. Der Rückstand wird mit 1.4 ml Ethanol und 1.4 ml Triethylamin 2 h gekocht und die Lösung abgekühlt auf RT. Nach Entfernung der flüchtigen Komponenten am Rotavapor wird der Rückstand in DMSO aufgenommen und über präparative HPLC (Methode 5) getrennt. Man erhält 243 mg (52 % d. Th.) der Titelverbindung.

LC-MS (Methode 2): $R_t$ = 2.65 min

MS (ES+): m/z = 489 $(M+H)^+$

$^1$H-NMR (400 MHz, $CDCl_3$): δ = 14.41 (s, 1H), 8.52 (s, 1H), 7.92 (d, J = 12 Hz, 1H), 5.31 (dq, J = 2.5, 7.9 Hz, 2H), 4.14 (q, J = 7.1 Hz, 2H), 3.84 (s, 3H), 3.50 (br. d, J = 12.1 Hz, 2H), 3.23 (br. d, J = 12.7 Hz, 2H), 3.14 (br. t, J = 11 Hz, 1H), 2.90 (br. t, J ~ 11 Hz, 1H), 2.30-2.20 (m, 3H), 1.96 (br. d, J ~ 8 Hz, 1H), 1.85-1.70 (m, 2H), 1.26 (t, J = 7.1 Hz, 3H).

**Beispiel 56A**

7-[(3R)-3-(2-Ethoxy-2-oxoethyl)-piperidin-1-yl]-6-fluor-8-methoxy-4-oxo-1-(2,2,2-trifluorethyl)-1,4-dihydrochinolin-3-carbonsäure

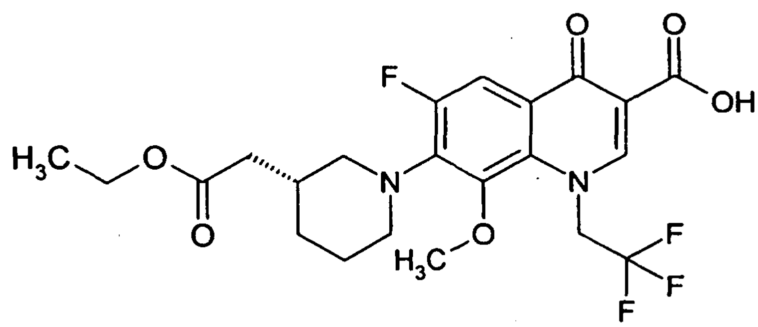

300 mg (1.05 mmol) der Verbindung aus Beispiel 19A (R-Enantiomer) werden in 8 ml Acetonitril bei RT vorgelegt, mit 183 µl N,N-Diisopropylethylamin (1.1 Äq.), dann mit 368 mg (0.96 mmol) der Verbindung aus Beispiel 38A versetzt. Die Mischung wird bei 50°C gerührt. Nach einer Stunde werden 83 µl N,N-Diisopropylethylamin (0.5 Äq.) und nach 2 Stunden erneut 83 µl N,N-Diisopropylethylamin (0.5 Äq.) zugegeben. Die Mischung wird weiter bei 50°C über Nacht rühren lassen, dann am Rotavapor von den flüchtigen Komponenten befreit. Der Rückstand wird mit 1.4 ml Ethanol und 1.4 ml Triethylamin 2 h gekocht und die Lösung abgekühlt auf RT. Nach Entfernung der flüchtigen Komponenten am Rotavapor wird der Rückstand in DMSO aufgenommen und über präparative HPLC (Methode 5) getrennt. Man erhält 243 mg (52 % d. Th.) der Titelverbindung.

LC-MS (Methode 2): $R_t$ = 2.65 min

MS (ES+): m/z = 489 $(M+H)^+$ $^1$H-NMR (400 MHz, $CDCl_3$): δ = 14.44 (s, 1H), 8.53 (s, 1H), 7.92 (d, J = 12 Hz, 1H), 5.31 (dq, J = 2.5, 7.9 Hz, 2H), 4.14 (q, J = 7.1 Hz, 2H), 3.84 (s, 3H), 3.50 (br. d, J = 12.1 Hz, 2H), 3.23 (br. d, J =

12.7 Hz, 2H), 3.14 (br. t, J = 11 Hz, 1 H), 2.90 (br. t, J ~ 11 Hz; 1H), 2.30-2.20 (m, 3H), 1.96 (br. d, J ~ 8 Hz, 1H), 1.85-1.70 (m, 2H), 1.26 (t, J = 7.1 Hz, 3H).

**Beispiel 57A**

7-[4-(2-Ethoxy-2-oxoethyl)-piperidin-1-yl]-6-fluor-8-meth oxy-4-oxo-1-(2,2,2-trifluorethyl)-1,4-dihydrochinolin-3-carbonsäure

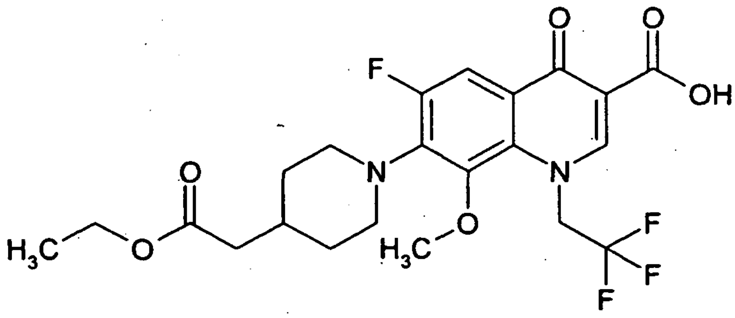

1100 mg (2.86 mmol) 6,7-Difluor-8-methoxy-1-(2,2,2-trifluorethyl)-4-oxo-1,4-dihydrochinolin-3-yl]-carbonyl-difluorborat (Beispiel 38A) und 979 mg (5.71 mmol, 2 Äq.) Piperidin-4-yl-essigsäureethylester werden in 20.6 ml Acetonitril 3 h bei 50°C gerührt. Das Lösungsmittel wird am Rotationsverdampfer entfernt und der Rückstand mit 14 ml Ethanol und 28 ml Triethylamin versetzt. Diese Lösung wird 1 h auf Rückfluss erhitzt. Die Lösungsmittel werden am Rotationsverdampfer entfernt, der Rückstand in DMSO / Acetonitril aufgenommen und über präparative HPLC (Methode 5) getrennt. Nach Einengen der entsprechenden Fraktionen am Rotationsverdampfer und Trocknung im Hochvakuum werden 358 mg (26% d. Th.) der Titelverbindung erhalten.

LC-MS (Methode 2): $R_t$ = 2.64 min

MS (ES+): m/z = 489 $(M+H)^+$

$^1$H-NMR (400 MHz, $CDCl_3$): δ = 14.48 (s, 1H), 8.54 (s, 1H), 7.90 (d, 1H), 5.32 (q, 2H), 4.17 (q, 2H), 3.83 (s, 3H), 3.50 (br. d, 2H), 3.22 (br. d, J = 12.7 Hz, 2H), 2.32 (d, 2H), 2.04 (m, 1H), 1.84 (br. d, 2H), 1.49 (dq, 2H), 1.28 (t, 3H).

**Beispiel 58A**

7-(4-Aminocarbonyl-piperidin-1-yl)-6-fluor-8-methoxy-4-oxo-1-(2,2,2-trifluorethyl)-1,4-dihydrochinolin-3-carbonsäure

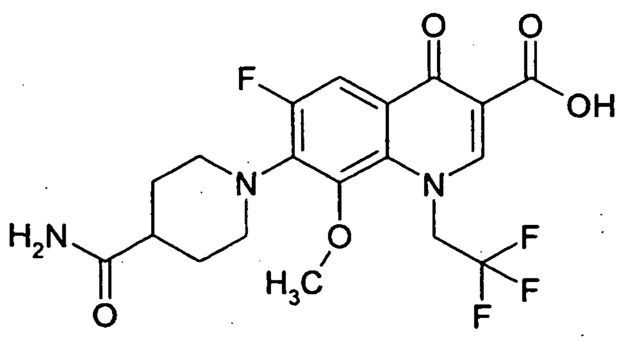

800 mg (2.08 mmol) der Verbindung aus Beispiel 38A und 533 mg 4-Aminocarbonylpiperidin (4.16 mmol) werden über Nacht in 15 ml Acetonitril bei 50°C verrührt. Das Lösungsmittel wird am Rotavapor entfernt und der Rückstand mit 20 ml Ethanol und 10 ml Triethylamin 1h verkocht. Nach Abkühlung werden die flüchtigen Komponenten am Rotavapor

entfernt. Der Rückstand wird mit Acetonitril verrührt, der Feststoff abfiltriert, mit Acetonitril gewaschen und im HV getrocknet. Man erhält 655 mg der Titelverbindung (71 % d. Th.).

LC-MS (Methode 1): $R_t$ = 1.90 min

MS (ES+): m/z = 446 $(M+H)^+$ $^1$H-NMR (400 MHz, DMSO-$d_6$): $\delta$ = 9.01 (s, 1H), 7.81 (d, J = 12.2 Hz, 1H), 7.31 (s, 1H), 6.82 (s, 1H), 5.78 (q, J = 8.7 Hz, 2H), 3.81 (s, 3H), 3.45 (br. d, J ~ 12.4 Hz, 2H), 3.16 (br. t, J = 12.2 Hz, 2H), 2.38-2.27 (m, 1H), 1.83-1.67 (m, 4H).

**Beispiel 59A**

1-Cyclopropyl-7-(4-ethoxycarbonyl-piperidin-1-yl)-6-fluor-8-methoxy-4-oxo-1,4-dihydrochinolin-3-carbonsäure

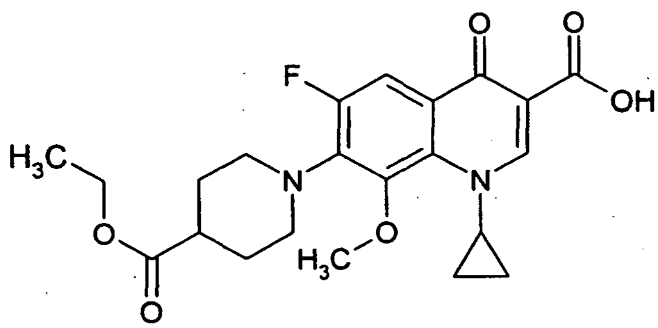

Eine Lösung von 275 mg (1.75 mmol) Piperidin-4-carbonsäureethylester und 250 mg (0.73 mmol) (T-4)-(1-Cyclopropyl-6,7-difluor-1,4-dihydro-8-methoxy-4-oxo-3-chinolincarboxylato-O3,O4)bordifluorid (Herstellung siehe: Journal of Medicinal Chemistry (1995), 38(22), 4478-87) in 5 ml Acetonitril wird über Nacht bei 50°C gerührt. Das Lösungsmittel wird am Rotationsverdampfer entfernt, der Rückstand in 5 ml Triethylamin und 50 ml Ethanol aufgenommen und 4 h auf Rückfluss erhitzt. Nach Abkühlung wird die Lösung am Rotationsverdampfer aufkonzentriert und das Produkt durch RP-HPLC (Methode 6) gereinigt. Man erhält 214 mg (68% d. Th.) der Titelverbindung.

$^1$H-NMR (300 MHz, DMSO-$d_6$): $\delta$ =1.00-1.06 (m, 2H), 1.09-1.16 (m, 2H), 1.21 (t, J = 7.1 Hz, 3H), 1.68-1.80 (m, 2H), 1.96 (br. d, J = 11 Hz, 2H), 2.59 (m, 1H), 3.22 (br. t, J = 12 Hz, 2H), 3.48 (br. d, J = 12.5 Hz, 2H), 3.75 (s, 3H), 4.10 (q, J = 7.1 Hz, 2H), 4.16 (m, 1H), 7.74 (d, J = 12.0 Hz, 1H), 8.69 (s, 1H), 14.95 (s, 1H).

Nach dem gleichen Verfahren wie für Beispiel 59A werden aus dem gleichen Edukt und den entsprechend substituierten Piperidinen die folgenden Beispiele 60A bis 62A hergestellt. Wenn keine Beispielnummer für den Piperidin-Teil angegeben ist, ist das eingesetzte substituierte Piperidin kommerziell erhältlich.

| Beispiel-Nr. | Struktur | Piperidin | Analytikdaten LC-MS (Methode)/ Meßwerte |
|---|---|---|---|
| 60A | 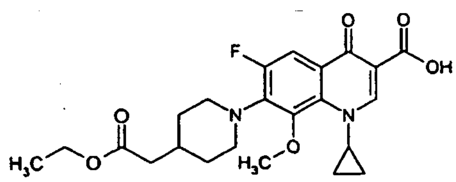 | | LC-MS (Methode 1):<br>$R_t$ = 2.67 min<br>MS (ES+): m/z = 447 $(M+H)^+$ |
| 61A | 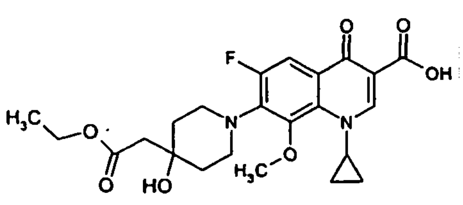 | 12A | LC-MS (Methode 3):<br>$R_t$ = 2.29 min<br>MS (ES+): m/z = 463 $(M+H)^+$ |

(fortgesetzt)

| Beispiel-Nr. | Struktur | Piperidin | Analytikdaten LC-MS (Methode)/ Meßwerte |
|---|---|---|---|
| **62A** | 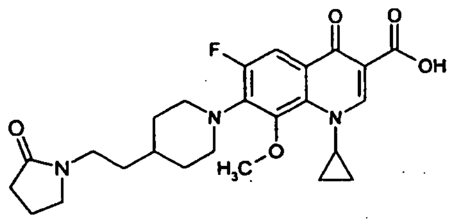 | | LC-MS (Methode 2):<br>$R_t$ = 2.06 min<br>MS (ES+): m/z = 472 $(M+H)^+$ |

**Beispiel 63A**

6-Fluor-7-(4-hydroxypiperidin-1-yl)-8-methoxy-4-oxo-1-(2,2,2-trifluorethyl)-1,4-dihydrochinolin-3-carbonsäure

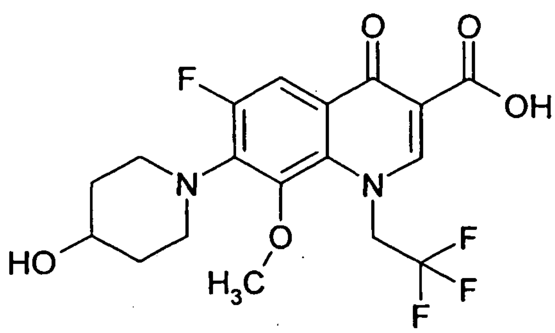

500 mg (1.30 mmol) der Verbindung aus Beispiel 38A und 394 mg (3.90 mmol) 4-Hydroxypiperidin werden in 5 ml Acetonitril bei 50°C über Nacht gerührt. Das Lösungsmittel wird am Rotationsverdampfer entfernt und der Rückstand in 5 ml Ethanol 2 h auf Rückfluss erhitzt. Die Suspension wird abgekühlt auf 0°C und filtriert. Der Feststoff wird mit Ethanol / Wasser 10 : 1 gewaschen und im Hochvakuum getrocknet. Man erhält 253 mg (47% d. Th.) der Titelverbindung.

LC-MS (Methode 3): $R_t$ = 2.21 min, MS (ES+) = 419 $(M+H)^+$.

$^1$H-NMR (400 MHz, DMSO=$d_6$): $\delta$ = 1.50-1.61 (m, 2H), 1.86-1.93 (m, 2H), 3.16 (br t, J = 11.5 Hz, 2H), 3.44 (br d, J = 12 Hz, 2H), 3.70 (m, 1H), 3.80 (s, 3H), 4.79 (d, J = 4.1 Hz, 1H), 5.78 (q, J = 8.6 Hz, 2H), 7.80 (d, J = 12.2 Hz, 1H), 9.01 (s, 1H), 14.66 (s, 1H).

Nach dem gleichen Verfahren wie für Beispiel 63A wird mit dem entsprechend substituierten Piperidin folgendes Beispiel 64A hergestellt.

| Beispiel-Nr. | Struktur | Edukt | Analytikdaten LC-MS (Methode)/ Meßwerte |
|---|---|---|---|
| **64A**<br>(1S,2R)-Enantiomer | 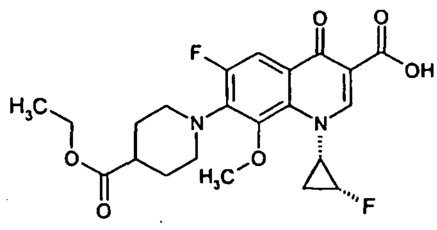 | 43A | LC-MS (Methode 2):<br>$R_t$ = 2.32 min<br>MS (ES+): m/z = 451 $(M+H)^+$ |

**Beispiel 65A**

7-[3-(2-Ethoxy-2-oxo-ethyl)-piperidin-1-yl]-6-fluor-8-methoxy-4-oxo-1-(2,2,2-trifluorethyl)-1,4-dihydrochinolin-3-carbonsäure (racemisch)

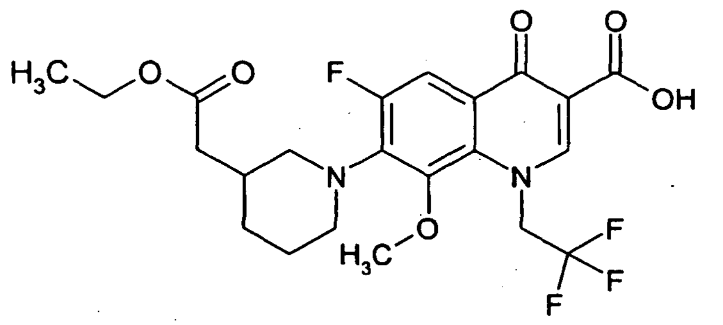

100 mg (0.26 mmol) der Verbindung aus Beispiel 38A und 80 mg (0.47 mmol) Piperidin-3-yl-essigsäureethylester werden in 1.5 ml Acetonitril bei 50°C über Nacht gerührt. Das Lösungsmittel wird am Rotationsverdampfer entfernt und der Rückstand in 3 ml Ethanol 1 h auf Rückfluss erhitzt. Ethanol wird am Rotationsverdampfer entfernt Der Rückstand wird mehrmals mit Ethanol verrührt und das Lösungsmittel abrotiert. Dann wird der Feststoff mit 4 ml Ethanol / Wasser 8 :2 gelöst, das Ethanol zum grössten Teil abdestilliert, wobei das Produkt ausfällt. Die Mischung wird abgekühlt auf 0°C für 20 min und das Produkt abfiltriert. Der Feststoff wird im Hochvakuum getrocknet. Man erhält 85 mg (67% d. Th.) der Titelverbindung.

LC-MS (Methode 2): $R_t$ = 2.62 min

MS (ES+): m/z = 489 $(M+H)^+$

$^1$H-NMR (400 MHz, DMSO-$d_6$): $\delta$ = 1.18 (t, 3H), 1.22 (m, 1H), 1.59-1.80 (m, 2H), 1.84 (br d, 1H), 2.09 (m, 1H), 2.30 (d, 2H), 3.11 (t, 1H), 3.39 (m, 2H), 3.79 (s, 3H), 4.05 (q, 2H), 5.78 (q, 2H), 7.80 (d, 1H), 9.01 (s, 1H), 14.6 (br s, 1H).

**Beispiel 66A**

6-Fluor-7-[(all-cis)-4-hydroxy-3,5-dimethylpiperidin-1-yl]-8-methoxy-4-oxo-1-(2,2,2-trifluorethyl)-1,4-dihydrochinolin-3-carbonsäure

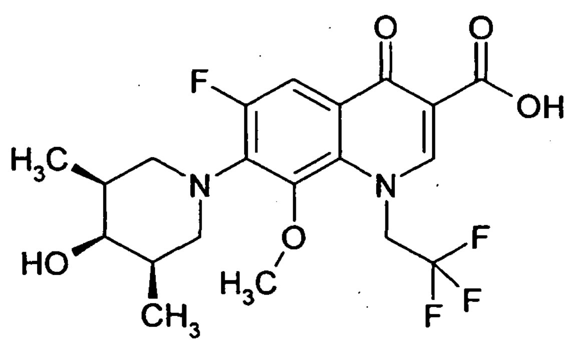

201 mg (0.52 mmol) der Verbindung aus Beispiel 38A und 95 mg (0.57 mmol) (allcis)-3,5-Dimethyl-4-hydroxypiperidin Hydrochlorid (Beispiel 11A) werden mit 109 µl (0.63 mmol) N,N-Diisopropylethylamin in 1.5 ml Acetonitril über Nacht bei 50°C gerührt. Das Lösungsmittel wird am Rotationsverdampfer entfernt, der Rückstand in 2 ml Triethylamin und 4 ml Ethanol aufgenommen und 1 h auf Rückfluss erhitzt. Nach Abkühlung wird die Lösung am Rotationsverdampfer von den Lösungsmitteln befreit und das Produkt durch RP-HPLC (Methode 5) gereinigt. Man erhält 36 mg (15% d. Th.) der Titelverbindung.

LC-MS (Methode 2): $R_t$ = 2.28 min, MS (ES+) = 447 $(M+H)^+$.
$^1$H-NMR (400 MHz, $CDCl_3$): δ = 1.01 (d, J = 6.9 Hz, 6H), 1.43 (br.s, 1H), 2.02 (m, 2H), 3.09 (dd, J = 4.2, 12.4 Hz, 2H), 3.22 (br t, J = 11.5 Hz, 2H), 3.76 (br s, 1H), 3.78 (s, 3H), 5.31 (q, J = 7.9 Hz, 2H), 7.91 (d, J = 12.1 Hz, 1H), 8.52 (s, 1H), 14.50 (s, 1H).

**Beispiel 67A**

6-Fluor-8-methoxy-4-oxo-7-{3-oxo-1-oxa-3,8-diazaspiro[4,5]dec-8-yl}-1-(2,2,2-trifluorethyl)-1,4-dihydrochinolin-3-carbonsäure

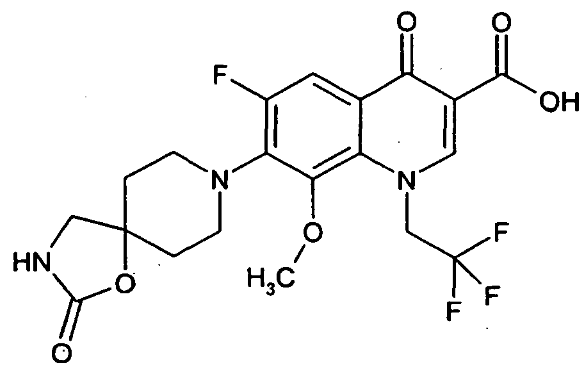

Aus 760 mg (4.87 mmol) 1-Oxa-3,8-diazaspiro[4,5]-decan-2-on (Herstellung siehe: Journal of Medicinal Chemistry (1981), 24, 1320-28) und 937 mg (2.43 mmol) Beispiel 38A werden analog zur Herstellung von Beispiel 66A 160 mg (6% d. Th.) der Titelverbindung isoliert.
LC-MS (Methode 3): $R_t$ = 2.30 min, MS (ES+) = 474 $(M+H)^+$.

**Beispiel 68A**

1-Cyclopropyl-6-fluor-8-methoxy-4-oxo-7-(3-oxo-2,8-diazaspiro[4,5]decan-8-yl)-1,4-dihydrochinolin-3-carbonsäure

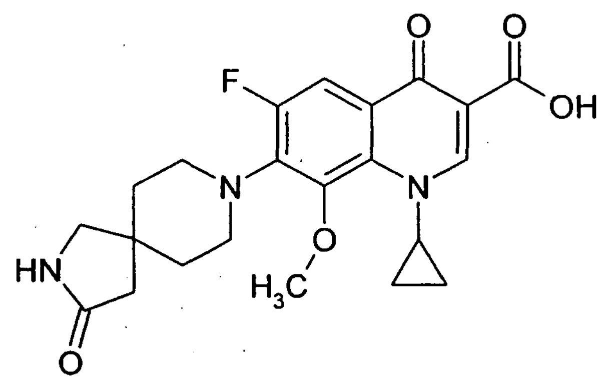

Aus 99 mg (0.52 mmol) 3-Oxo-2,8-diazaspiro[4,5]-decan Hydrochlorid (Beispiel 13A) wird die freie Base durch Verrühren mit 1 g Tris(aminoethyl)-polystyrol in Dichlormethan / Methanol 10:1 während 20 min und anschließender Filtration und Entfernung der Lösungsmittel am Rotationsverdampfer freigesetzt. Der Rückstand wird in 3 ml Acetonitril aufgenommen und mit 89 mg (0.26 mmol) (T-4)-(1-Cyclopropyl-6,7-difluor-1,4-dihydro-8-methoxy-4-oxo-3-chinolincarboxylato-O3,O4)bordifluorid (Herstellung siehe: Journal of Medicinal Chemistry (1995), 38(22), 4478-4487) über Nacht

bei 50°C gerührt. Das Lösungsmittel wird am Rotationsverdampfer entfernt, der Rückstand in 3 ml Triethylamin und 30 ml Ethanol aufgenommen und 1.5 h auf Rückfluss erhitzt. Nach Abkühlung werden die Lösungsmittel am Rotationsverdampfer entfernt, der Rückstand in wenig DMSO aufgenommen und durch RP-HPLC (Methode 5) gereinigt. Man erhält 56 mg (50% d. Th.) der Titelverbindung.

LC-MS (Methode 3): $R_t$ = 1.92 min, MS (ES+) = 430 $(M+H)^+$.

$^1$H-NMR (400 MHz, $CDCl_3$): δ = 0.97-1.03 (m, 2H), 1.18-1.27 (m, 2H, 1.86 (t, J = 5.3 Hz, 4H), 2.35 (s, 2H), 3.32 (s, 2H), 3.33-3.43 (m, 4H), 3.79 (s, 3H), 4.03 (m, 1H), 5.50 (s, 1H), 7.89 (d, J = 12.2 Hz, 1H), 8.82 (s, 1H), 14.73 (s, 1H)

**Beispiel 69A**

6-Fluor-8-methoxy-4-oxo-7-(3-oxo-2,8-diazaspiro[4.5]dec-8-yl)-1-(2,2,2-trifluorethyl)-1,4-dihydrochinolin-3-carbonsäure

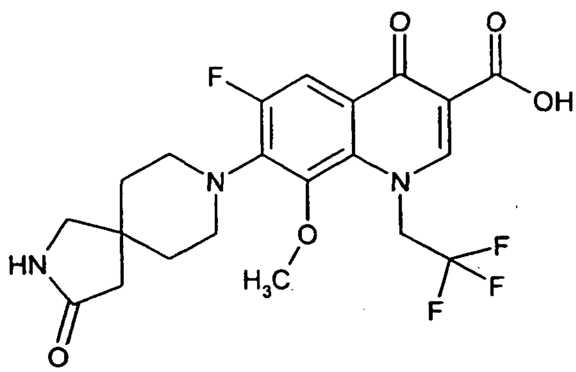

Analog zur Herstellung von Beispiel 68A werden aus 146 mg Beispiel 38A (0:38 mmol) und 145 mg (0.76 mmol) 3-Oxo-2,8-diazaspiro[4,5]-decan Hydrochlorid (Beispiel 13A) 73 mg (21% d. Th.) der Titelverbindung erhalten.

LC-MS (Methode 3): $R_t$ = 2.13 min, MS (ES+) = 472 $(M+H)^+$.

**Beispiel 70A**

8-Chlor-1-cyclopropyl-N-(2,4-dichlorbenzyl)-6,7-difluor-4-oxo-1,4-dihydrochinolin-3-carbonsäureamid

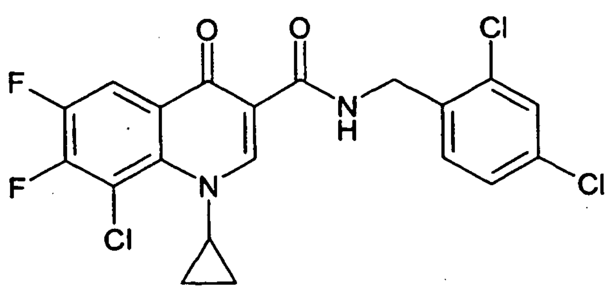

15.0 g 8-Chlor-1-cyclopropyl-6,7-difluor-4-oxo-1,4-dihydrochinolin-3-carbonsäure (Herstellung siehe DE 3420743 oder Y. Kimura et al. J. Med. Chem. 1994, 37 (20), 3344) werden in 500 ml DMF gelöst und mit 31.3 g PyBOP und 10.6 g 2,4-Dichlorbenzylamin versetzt. Nach einem Tag wird das Lösungsmittel einrotiert und der Rückstand durch Flashchromatographie über Kieselgel (Toluol / Essigsäureethylester 95:5) gereinigt. Man erhält 21.2 g (93% d. Th.) der Titelverbindung.

LC-MS (Methode 1): $R_t$ = 3.10 min, MS (ES+) = 457 $(M+H)^+$.

$^1$H-NMR (300 MHz, DMSO-$d_6$): $\delta$ = 1.05-1.16 (m, 2H), 1.18-1.29 (m, 2H), 4.32 (m, 1H), 4.99 (d, J = 6.0 Hz, 1H), 7.35-7.45 (m, 2H), 7.64 (d, J = 2.0 Hz, 1H), 8.22 (dd, J = 8.9, 10.0 Hz, 1H), 8.79 (s, 1H), 10.01 (t, J = 6.0 Hz, 1H).

## Ausführungsbeispiele

### Beispiel 1

1-[3-{[(2,4-Dichlorbenzyl)amino]carbonyl}-6-fluor-8-methoxy-4-oxo-1-(2,2,2-trifluorethyl)-1,4-dihydrochinolin-7-yl] piperidin-4-carbonsäureethylester

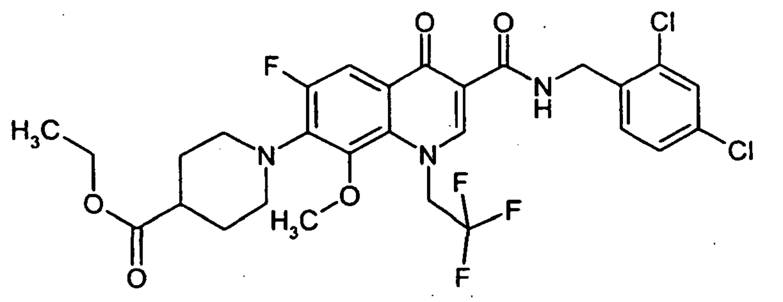

200.0 mg (0.42 mmol) der Verbindung aus Beispiel 47A und 111.3 mg (0.63 mmol) 2,4-Dichlorbenzylamin werden in 2.6 ml N,N-Dimethylformamid vorgelegt, mit 257 $\mu$l (1.48 mmol) N,N-Diisopropylethylamin und abschließend 438.8 mg (0.84 mmol) PyBOP versetzt. Die Reaktion wird bei Raumtemperatur 3 h gerührt. Zur Aufarbeitung wird die Mischung mit Essigsäureethylester verdünnt, zweimal mit Wasser gewaschen, die vereinigten wässrigen Phasen einmal mit Essigsäureethylester extrahiert, die vereinigten organischen Phasen über Natriumsulfat getrocknet und am Rotationsverdampfer vom Lösungsmittel vollständig befreit. Durch Feinreinigung des Rückstandes über die präparative RP-HPLC (Methode 5) wird die Titelverbindung mit 250.0 mg (94% d. Th.) erhalten.

$^1$H-NMR (400 MHz, $CDCl_3$): $\delta$ = 1.29 (t, J = 7 Hz, 3H), 1.83-1.96 (m, 2H), 2.03 (dd, J = 3, 13 Hz, 2H), 2.52 (tt, J = 3.8, 11.1 Hz, 1H), 3.21 (br t, J = 12 Hz, 2H), 3.49 (br d, J = 12 Hz, 2H), 3.84 (s, 3H), 4.19 (q, J = 7.1 Hz, 2H), 4.70 (d, J = 6.2 Hz, 2H), 5.24 (q, J = 8.1 Hz, 2H), 7.21 (dd, J = 2.0, 8.3 Hz, 1H), 7.390 (d, J = 8.1 Hz, 1H), 7.392 (d, J = 2 Hz, 1H), 7.91 (d, J = 12.5 Hz, 1H), 8.54 (s, 1H), 10.22 (t, J = 5.9 Hz, 1H).

HPLC (Methode 7): $R_t$ = 5.65 min.

MS (ES+): m/z = 632 $(M+H)^+$

### Beispiel 2

1-[6-Fluor-8-methoxy-3-({[2-methyl-4-(trifluormethoxy)benzyl]amino}carbonyl)-4-oxo-1-(2,2,2-trifluorethyl)-1,4-dihydrochinolin-7-yl]piperidin-4-carbonsäureethylester

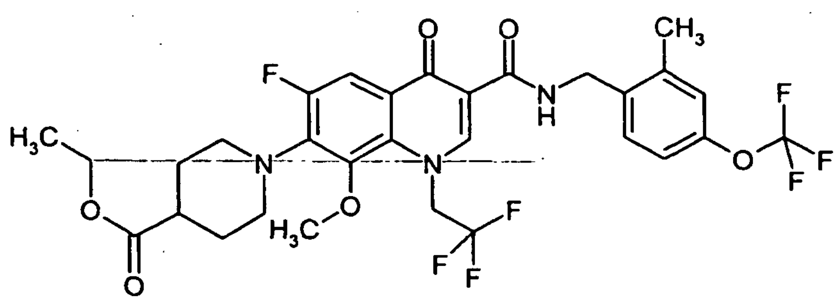

100.0 mg (0.21 mmol) der Verbindung aus Beispiel 47A und 157.2 mg (0.42 mmol) 2-Methyl-4-(trifluormethoxy) benzylamin (Beispiel 5A) werden in 3 ml N,N-Dimethylformamid vorgelegt, mit 202 μl (1.16 mmol) N,N-Diisopropylethylamin und abschließend 274.2 mg (0.84 mmol) PyBOP versetzt. Nach 3 h bei RT wird die ganze Reaktionsmischung über die präparative HPLC (Methode 5) getrennt. Man erhält 96.0 mg (69% d. Th.) der Titelverbindung.

$^1$H-NMR (400 MHz, $CDCl_3$): δ = 1.29 (t, J = 7.1 Hz, 3H), 1.83-1.96 (m, 2H), 2.03 (br dd, J = 3, 13 Hz, 2H), 2.40 (s, 3H), 2.51 (m, 1H), 3.21 (br. t, J = 12 Hz, 2H), 3.49 (br.d, J = 12 Hz, 2H), 3.84 (s, 3H), 4.19 (q, J = 7.1 Hz, 2H), 4.62 (d, J = 5.6 Hz, 2H), 5.24 (q, J = 8.0 Hz, 2H), 6.98-7.03 (m, 2H), 7.36 (d, J = 8.1 Hz, 1H), 7.88 (d, J = 12.5 Hz, 1H), 8.56 (s, 1H), 10.07 (t, J = 5.6 Hz, 1H).

HPLC (Methode 8): $R_t$ = 5.43 min

MS (ES+): m/z = 662 $(M+H)^+$

**Beispiel 3**

1-[3-({[2-Chlor-4-(trifluormethoxy)benzyl]amino}carbonyl)-6-fluor-8-methoxy-4-oxo-1-(2,2,2-trifluorethyl)-1,4-dihydrochinolin-7-yl]piperidin-4-carbonsäureethylester

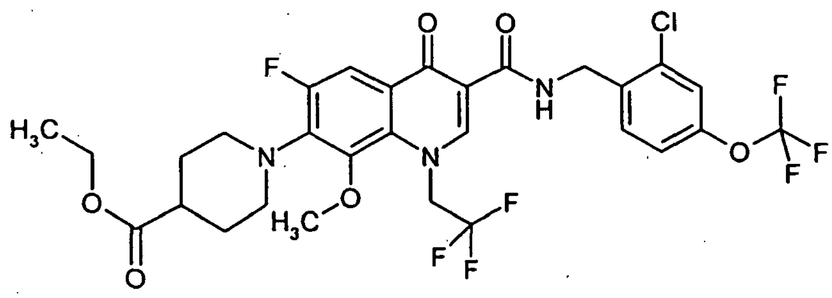

50.0 mg (0.105 mmol) der Verbindung aus Beispiel 47A und 55.2 mg (0.21 mmol) 2-Chlor-4-(trifluormethoxy)-benzylamin (Beispiel 7A) werden in 1.5 ml N,N-Dimethylformamid vorgelegt, mit 101 μl (0.58 mmol) N,N-Diisopropylethylamin und abschließend 137 mg (0.26 mmol) PyBOP versetzt. Nach 30 min. wird die ganze Reaktionsmischung über die präparative HPLC (Methode 5) getrennt. Man erhält 63 mg (87% d. Th.) der Titelverbindung.

LC-MS (Methode 3): $R_t$ = 3.43 min.

MS (ESI pos): m/z = 682 $(M+H)^+$

$^1$H-NMR (400 MHz, $CDCl_3$): δ = 1.29 (t, J = 7.1 Hz, 3H), 1.83-1.96 (m, 2H), 1.99-2.03 (m, 2H), 2.52 (m, 1H), 3.21 (br. t, J = 12 Hz, 2H), 3.49 (br.d, J = 12 Hz, 2H), 3.84 (s, 3H), 4.19 (q, J = 7.1 Hz, 2H), 4.72 (d, J = 6.0 Hz, 2H), 5.24 (q, J = 8.0 Hz, 2H), 7.10 (d, J = 8.7 Hz, 2H), 7.27 (unter $CHCl_3$ Signal, 1H), 7.49 (d, J = 8.7 Hz, 1H), 7.86 (d, J = 12.6 Hz, 1H), 8.55 (s, 1H), 10.77 (t, J = 6.0 Hz, 1H).

**Beispiel 4**

1-[6-Fluor-1-(2-fluorethyl)-8-methoxy-3-({[2-methyl-4-(trifluormethoxy)benzyl]-amino}carbonyl)-4-oxo-1,4-dihydrochinolin-7-yl]piperidin-4-carbonsäureethylester

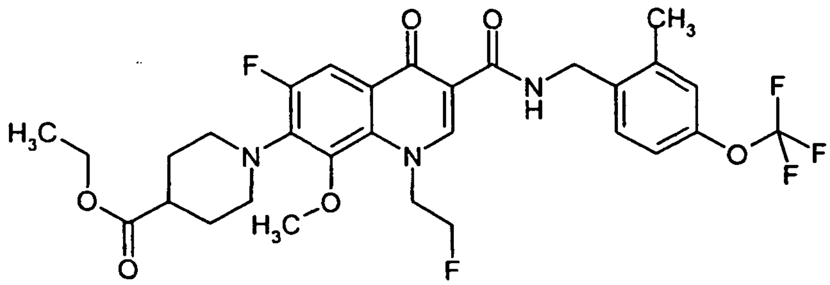

72.0 mg (0.164 mmol) der Verbindung aus Beispiel 46A und 47.6 mg (0.197 mmol) 2-Methyl-4-(trifluormethoxy)-benzylamin Hydrochlorid (Beispiel 5A) werden in 2.15 ml N,N-Dimethylformamid vorgelegt, mit 157 μl (0.90 mmol) N,N-Diisopropylethylamin und abschließend 170.9 mg (0.33 mmol) PyBOP versetzt. Es wird über Nacht bei RT gerührt, dann wird die ganze Reaktionsmischung über die präparative HPLC (Methode 5) getrennt. Man erhält 85 mg (83 % d. Th.) der Titelverbindung.

LC-MS (Methode 3): $R_t$ = 3.22 min.

MS (ESI pos): m/z = 626 $(M+H)^+$

$^1$H-NMR (400 MHz, $CDCl_3$): δ = 1.29 (t, J = 7.1 Hz, 3H), 1.83-1.96 (m, 2H), 2.03 (m, 2H), 2.41 (s, 3H), 2.51 (m, 1H), 3.21 (br. t, J = 12 Hz, 2H), 3.47 (br.d, J = 13 Hz, 2H), 3.82 (s, 3H), 4.18 (q, J = 7.1 Hz, 2H), 4.62 (d, J = 5.7 Hz, 2H), 4.69 (dt, J = 46, 4 Hz, 2H), 4.78 (dt, J = 31, 4 Hz, 2H), 7.01 (d, J = 8.0 Hz, 1H), 7.02 (s, 1H), 7.36 (d, J = 8.0 Hz, 1H), 7.93 (d, J = 12.6 Hz, 1H), 8.64 (s, 1H), 10.19 (t, J = 5.7 Hz, 1H).

## Beispiel 5

[6-Fluor-8-methoxy-3-({[2-methyl-4-(trifluormethoxy)benzyl)amino}carbonyl)-4-oxo-1-(2,2,2-trifluorethyl)-1,4-dihydrochinolin-7-yl]piperidin-4-yl-essigsäureethylester

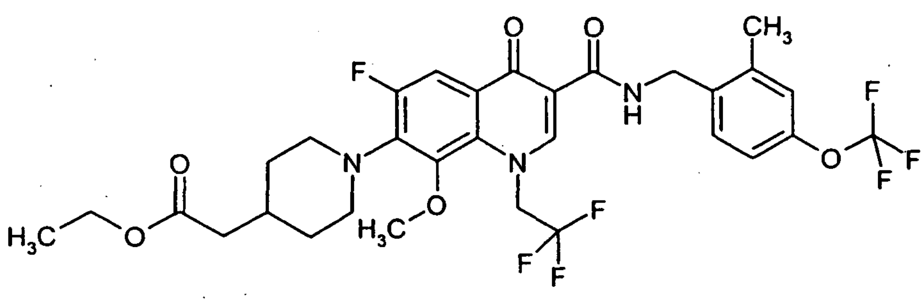

100.0 mg (0.18 mmol) der Verbindung aus Beispiel 57A und 46.7 mg (0.19 mmol) 2-Methyl-4-(trifluormethoxy) benzylamin Hydrochlorid (Beispiel 5A) werden in 1 ml N,N-Dimethylformamid vorgelegt, mit 177 μl (1.01 inmol) N,N-Diisopropylethylamin und abschließend 234.7 mg (0.46 mmol) PyBOP versetzt. Nach 1.5 h bei RT wird die ganze Reaktionsmischung über die präparative HPLC (Methode 5) getrennt. Man erhält 82.0 mg (66% d. Th.) der Titelverbindung.

LC-MS (Methode 2): $R_t$ = 3.22 min.

MS (ESI pos): m/z = 676 $(M+H)^+$

$^1$H-NMR (400 MHz, $CDCl_3$): δ = 1.28 (t, J = 7.1 Hz, 3H), 1.46 (m, 2H), 1.32 (br.d, J = 11 _ .,_ Hz, 2H), 2.20 (m, 1H), 2.32 (d, J = 7.1 Hz, 2H), 2.41 (s, 3H), 3.20 (br. t, J = 12 Hz, 2H), 3.45 (br.d, J = 12 Hz, 2H), 3.81 (s, 3H), 4.16 (q, J = 7.1 Hz, 2H), 4.62 (d, J = 5.6 Hz, 2H), 5.25 (q, J = 8.0 Hz, 2H), 7.00-7.04 (m, 2H), 7.36 (d, J = 8.1 Hz, 1H), 7.87 (d, J = 12.5 Hz, 1H), 8.56 (s, 1H), 10.08 (t, J = 5.6 Hz, 1H).

Beispiel 6

[3-{[(2,4-Dichlorbenzyl)amino]carbonyl}-6-fluor-8-methoxy-4-oxo-1-(2,2,2-trifluorethyl)-1,4-dihydrochinolin-7-yl]piperidin-3-yl-essigsäureethylester

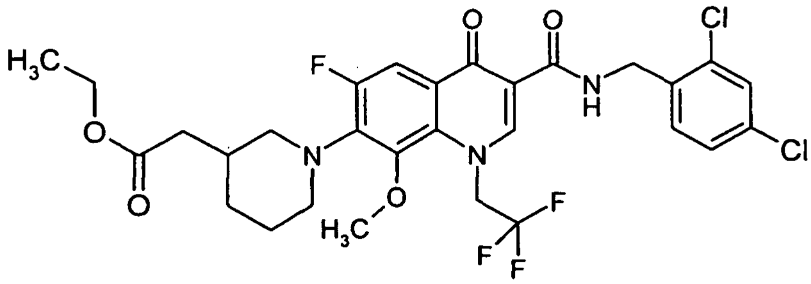

78 mg (0.16 mmol) der Verbindung aus Beispiel 65A, 116.3 mg (0.22 mmol) PyBOP und 9.7 mg DMAP (0.08 mmol) werden in 2 ml N,N-Dimethylformamid vorgelegt und mit 56.2 mg (0.32 mmol) 2,4-Dichlorbenzylamin versetzt. Die Mischung wird über Nacht bei RT gerührt dann über die präparative HPLC (Methode 5) getrennt. Man erhält 49.0 mg (47% d. Th.) der Titelverbindung.

LC-MS (Methode 2): $R_t$ = 3.21 min.

MS (ESI pos): m/z = 646 $(M+H)^+$

$^1$H-NMR (400 MHz, DMSO-$d_6$): δ = 1.16 (t, J = 7.1 Hz, 3H), 1.18-1.28 (m, 1H), 1.60-1.78 (m, 2H), 1.84 (m, 1H), 2.09 (m, 1H), 2.27-2.31 (m, 2H), 2.87 (br. t, J = 10.5 Hz, 1H), 3.08 (br. t, J = 11.5 Hz, 1H), 3.36 (m, teilweise unter Wassersignal, 1H ?), 3.78 (s, 3H), 4.04 (q, J = 7.1 Hz, 2H), 4.60 (d, J = 6.0 Hz, 2H), 5.69 (q, J = 8.7 Hz, 2H), 7.38-7.45 (m, 2H), 7.64 (d, J = 1.7 Hz, 1H), 7.77 (d, J = 12.1 Hz, 1H), 8.83 (s, 1H), 10.14 (t, J = 6.0 Hz, 1H).

Analog zum Beispiel 1 werden die folgenden Beispiele 7 bis 19 hergestellt. Wenn keine Beispielnummer für das Ausgangsamin eingegeben ist, ist dieses kommerziell erhältlich.

| Beispiel- Nr. | Struktur | Edukte Beispiel-Nr. | Analytikdaten LC-MS (Methode)/ Meßwerte HPLC (Methode)/ Meßwerte MS (Methode)/ Meßwerte |
|---|---|---|---|
| 7 | 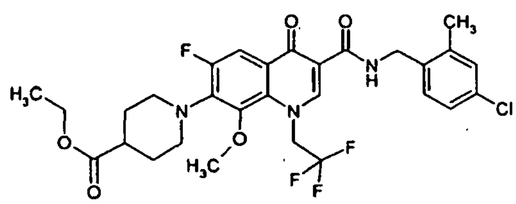 | 47A | HPLC (Methode 7): $R_t$ = 5.55 min MS (ESI+): m/z = 612 $(M+H)^+$ |
| 8 | 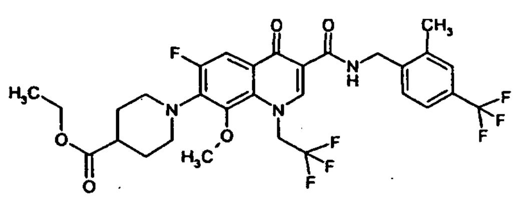 | 47A + 9A | LC-MS (Methode 3): $R_t$ = 3.36 min MS (ES+): m/z = 646 $(M+H)^+$ |

(fortgesetzt)

| Beispiel- Nr. | Struktur | Edukte Beispiel- Nr. | Analytikdaten LC-MS (Methode)/ Meßwerte HPLC (Methode)/ Meßwerte MS (Methode)/ Meßwerte |
|---|---|---|---|
| 9 | 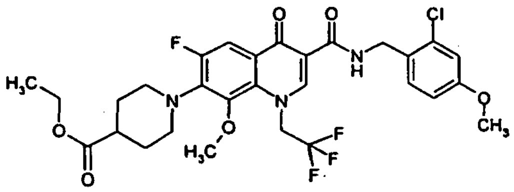 | 47A | LC-MS (Methode 3): $R_t$ = 3.28 min MS (ES+): m/z = 628 $(M+H)^+$ |
| 10 | 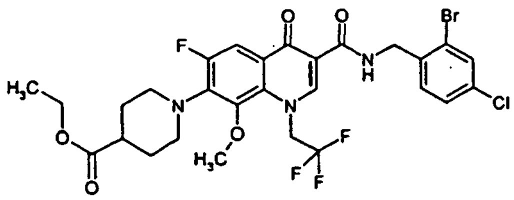 | 47A 47A + 6A | LC-MS (Methode 3): $R_t$ = 3.44 min MS (ES+): m/z = 676 / 678 $(M+H)^+$ |
| 11 | 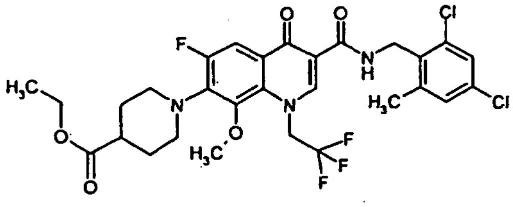 | 47A + 8A | LC-MS (Methode 1): $R_t$ = 3.40 min MS (ES+): m/z = 646 $(M+H)^+$ |
| 12 | 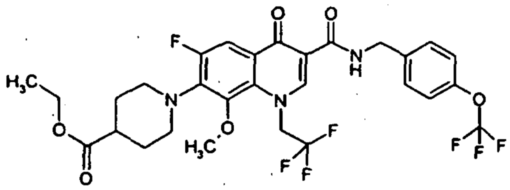 | 47A | LC-MS (Methode 3): $R_t$ = 3.31 min MS (ES+): m/z = 648 $(M+H)^+$ |
| 13 | 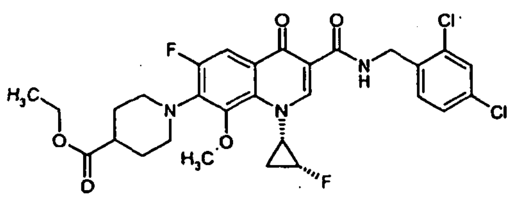 | 64A | LC-MS (Methode 2): $R_t$ = 3.04 min MS (ES+): m/z = 608 $(M+H)^+$ |
| 14 | 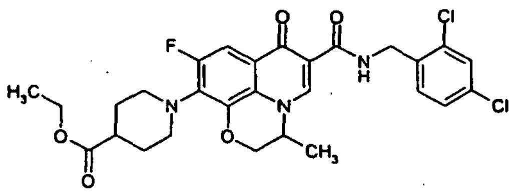 | 53A | LC-MS (Methode 1): $R_t$ = 3.14 min MS (ES+): m/z = 576 $(M+H)^+$ |

(fortgesetzt)

| Beispiel- Nr. | Struktur | Edukte Beispiel-Nr. | Analytikdaten LC-MS (Methode)/ Meßwerte HPLC (Methode)/ Meßwerte MS (Methode)/ Meßwerte |
|---|---|---|---|
| 15 | 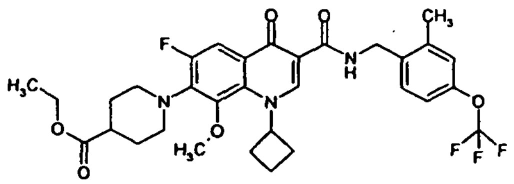 | 50A + 5A | LC-MS (Methode 2): $R_t$ = 3.21 min MS (ES+): m/z = 634 $(M+H)^+$ |
| 16 | 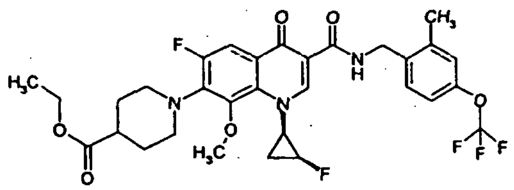 | 52A + 5A | LC-MS (Methode 1): $R_t$ = 3.21 min MS (ES+): m/z = 638 $(M+H)^+$ |
| 17 | 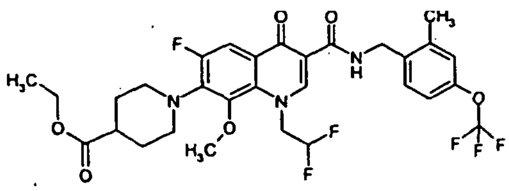 | 51A + 5A | LC-MS (Methode 2): $R_t$ = 3.07 min MS (ES+): m/z = 644 $(M+H)^+$ |
| 18 | 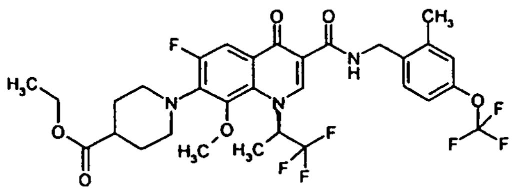 | 48A + 5A | LC-MS (Methode 2): $R_t$ = 3.22 min MS (ES+): m/z = 676 $(M+H)^+$ |
| 19 | 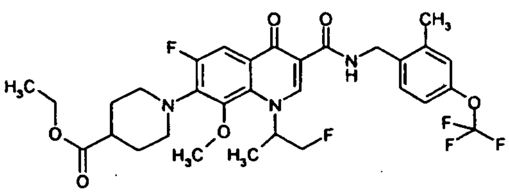 | 49A + 5A | LC-MS (Methode 2): $R_t$ = 3.10 min MS (ES+): m/z = 640 $(M+H)^+$ |

Beispiel 20

7-[(*3S*)-3-(2-Ethoxy-2-oxoethyl)-piperidin-1-yl]-6-fluor-8-methoxy-N-[2-methyl-4-(trifluormethoxy)-benzyl]-4-oxo-1-(2,2,2-trifluorethyl)-1,4-dihydrochinolin-3-carbonsäureamid

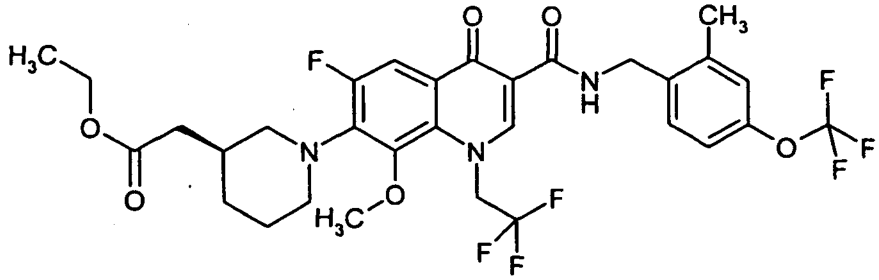

100.0 mg (0.21 mmol) der Verbindung aus Beispiel 55A und 59.4 mg (0.25 mmol) 2-Methyl-4-(trifluormethoxy)-benzylamin Hydrochlorid (Beispiel 5A) werden in 2.7 ml N,N-Dimethylformamid vorgelegt, mit 196 µl (1.13 mmol) N,N-Diisopropylethylamin und abschließend 213.1 mg (0.41 mmol) PyBOP versetzt. Die Reaktionsmischung wird über Nacht bei RT rühren lassen und anschließend als ganze über die präparative HPLC (Methode 5) getrennt. Man erhält 100.0 mg (72% d. Th.) der Titelverbindung.

LC-MS (Methode 2): $R_t$ = 3.24 min

MS (ES+): m/z = 676 $(M+H)^+$

$^1$H-NMR (400 MHz, $CDCl_3$): δ = 10.08 (t, J = 5.7 Hz, 1H), 8.55 (s, 1H), 7.87 (d, J = 12.4 Hz, 1H), 7.36 (d, J = 8.3 Hz, 1H), 6.98-7.03 (m, 2H), 5.25 (q, J = 7.9 Hz, 2H), 4.62 (d, J = 5.6 Hz, 2H), 4.14 (q, J = 7.1 Hz, 2H), 3.83 (s, 3H), 3.45 (br. d, J - 11 Hz, 1H), 3.38 (br. d, J ~ 12 Hz, 1H), 3.12 (br.t, J ~ 11 Hz, 1H), 2.88 (br.t, J ~ 11 Hz, 1H), 2.41 (s, 3H), 2.30-2.20 (m, 3H), 1.95 (br. d, J ~ 11 Hz, 1H), 1.85-1.70 (m, 2H), 1.25 (t, J = 7.1 Hz, 3H), 1.24 (m, 1H).

Beispiel 21

7-[(3R)-3-(2-Ethoxy-2-oxoethyl)-piperidin-1-yl]-6-fluor-8-methoxy-N-[2-methyl-4-(trifluormethoxy)-benzyl]-4-oxo-1-(2,2,2-trifluorethyl)-1,4-dihydrochinolin-3-carbonsäureamid

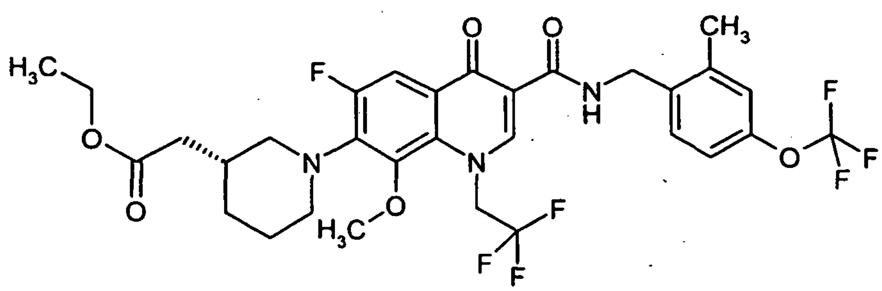

100.0 mg (0.21 mmol) der Verbindung aus Beispiel 56A und 59.4 mg (0.25 mmol) 2-Methyl-4-(trifluormethoxy)-benzylamin Hydrochlorid (Beispiel 5A) werden in 2.7 ml N,N-Dimethylformamid vorgelegt, mit 196 µl (1.13 mmol) N,N-Diisopropylethylamin und abschließend 213.1 mg (0.41 mmol) PyBOP versetzt. Die Reaktionsmischung wird über Nacht bei RT rühren lassen und anschließend als ganze über die präparative HPLC (Methode 5) getrennt. Man erhält 108 mg (78% d. Th.) der Titelverbindung,

LC-MS (Methode 2): $R_t$ = 3.23 min

MS (ES+): m/z = 676 $(M+H)^+$

$^1$H-NMR (400 MHz, $CDCl_3$): δ = 10.08 (t, J = 5.7 Hz, 1H), 8.55 (s, 1H), 7.87 (d, J = 12.4 Hz, 1H), 7.36 (d, J = 8.3 Hz, 1H), 6.98-7.03 (m, 2H), 5.25 (q, J = 7.9 Hz, 2H), 4.62 (d, J = 5.6 Hz, 2H), 4.14 (q, J = 7.1 Hz, 2H), 3.83 (s, 3H), 3.45 (br. d, J ~ 11 Hz, 1H), 3.38 (br. d, J ~ 12 Hz, 1H), 3.12 (br. t, J ~ 11 Hz, 1H), 2.88 (br. t, J ~ 11 Hz, 1H), 2.41 (s, 3H), 2.30-2.20 (m, 3H), 1.95 (br. d, J - 11 Hz, 1H), 1.85-1.70 (m, 2H), 1.25 (t, J = 7.1 Hz, 3H), 1.24 (m, 1H).

Analog zum Beispiel 1 werden auch die folgenden Beispiele 22 bis 30 hergestellt.

| Beispiel- Nr. | Struktur | Edukte Beispiel- Nr. | Analytikdaten LC-MS (Methode)/ Meßwerte HPLC (Methode)/ Meßwerte MS (Methode)/ Meßwerte |
|---|---|---|---|
| 22 | 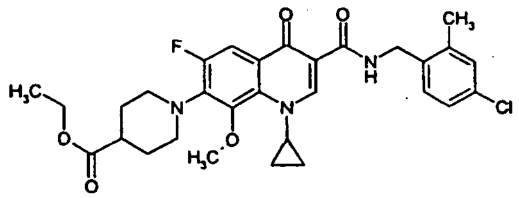 | 59A | LC-MS (Methode 2): $R_t$ = 3.05 min MS (ES+): m/z = 570 $(M+H)^+$ |
| 23 | 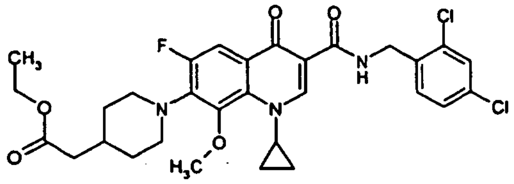 | 60A | LC-MS (Methode 1): $R_t$ = 3.17 min MS (ES+): m/z = 604 $(M+H)^+$ |
| 24 | 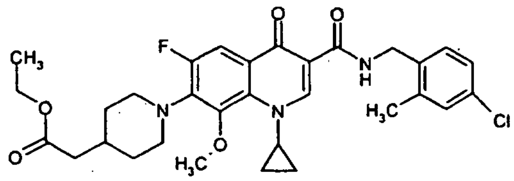 | 60A | LC-MS (Methode 2): $R_t$ = 3.12 min MS (ES+): m/z = 584 $(M+H)^+$ |
| 25 | 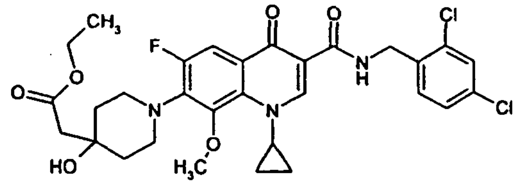 | 61A | LC-MS (Methode 3): $R_t$ = 3.17 min MS (ES+): m/z = 620 $(M+H)^+$ |
| 26 | 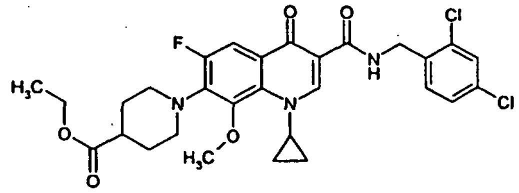 | 59A | LC-MS (Methode 2): $R_t$ = 3.12 min MS (ES+): m/z = 590 $(M+H)^+$ |
| 27 | 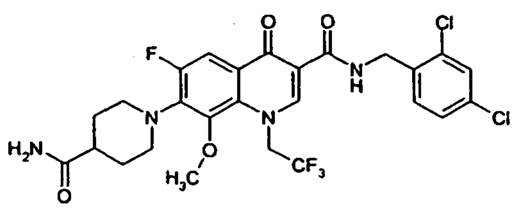 | 58A | HPLC (Methode 7): $R_t$ = 4.64 min MS (ES+): m/z = 603 $(M+H)^+$ |

(fortgesetzt)

| Beispiel- Nr. | Struktur | Edukte Beispiel- Nr. | Analytikdaten LC-MS (Methode)/ Meßwerte HPLC (Methode)/ Meßwerte MS (Methode)/ Meßwerte |
|---|---|---|---|
| 28 | 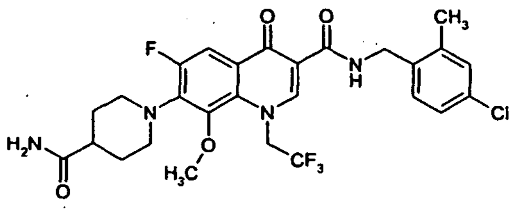 | 58A | HPLC (Methode 7): $R_t$ = 4.54 min MS (ES+): m/z = 583 $(M+H)^+$ |
| 29 | 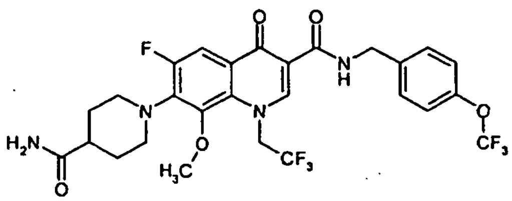 | 58A | MS (ES+): m/z = 619 $(M+H)^+$ |
| 30 | 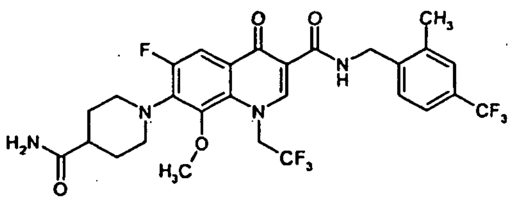 | 58A + 9A | LC-MS (Methode 1): $R_t$ = 2.59 min MS (ES+): m/z = 617 $(M+H)^+$ |

Beispiel 31

1-[6-Fluor-8-methoxy-3-({[2-methyl-4-(trifluormethoxy)benzyl]amino}carbonyl)-4-oxo-1-(2,2,2-trifluorethyl)-1,4-dihydrochinolin-7-yl]piperidin-4-carbonsäureamid

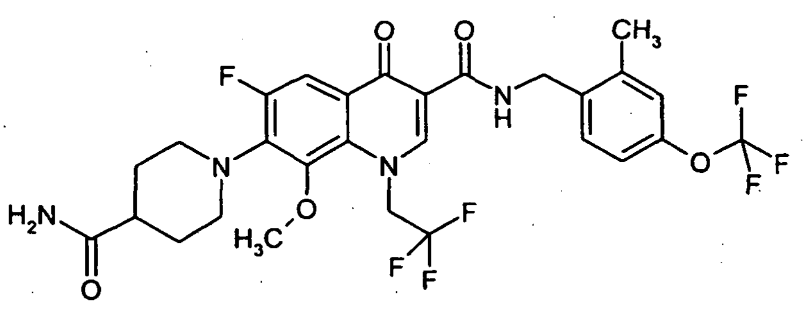

60.0 mg (0.14 mmol) der Verbindung aus Beispiel 58A und 46 mg (0.16 mmol) 2-Methyl-4-(trifluormethoxy) benzylamin Hydrochlorid (Beispiel 5A) werden in 1.7 ml N,N-Dimethylformamid vorgelegt, mit 129 µl (0.74 mmol) N,N-Diisopropylethylamin und abschließend 140.2 mg (0.27 mmol) PyBOP versetzt. Die Reaktionsmischung wird über Nacht bei RT rühren lassen dann als ganze über die präparative HPLC (Methode 5) getrennt. Man erhält 57 mg (67% d. Th.)

der Titelverbindung.

LC-MS (Methode 1): $R_t$ = 2.63 min

MS (ES+): m/z = 633 $(M+H)^+$.

$^1$H-NMR (400 MHz, $CDCl_3$): δ = 1.88-2.03 (m, 4H), 2.40 (m, 1H), 2.41 (s, 3H), 3.23 (br. t, J = 12 Hz, 2H), 3.53 (br.d, J = 12 Hz, 2H), 3.87 (s, 3H), 4.62 (d, J = 5.6 Hz, 2H), 5.26 (q, J = 8.0 Hz, 2H), 5.34 (br.s, 1H), 5.49 (br s, 1H), 6.98-7.04 (m, 2H), 7.34 (d, J = 8.1 Hz, 1H), 7.89 (d, J = 12.5 Hz, 1H), 8.56 (s, 1H), 10.06 (t, J - 5Hz, 1H).

Beispiel 32

6-Fluor-7-[(all-cis)-4-hydroxy-3,5-dimethylpiperidin-1-yl]-8-methoxy-N-[2-methyl-4-(trifluormethoxy)-benzyl]-4-oxo-1-(2,2,2-trifluorethyl)-1,4-dihydrochinolin-3-carbonsäureamid

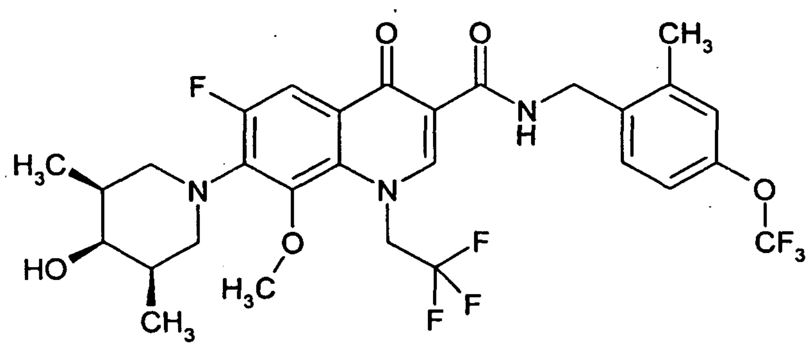

36 mg (0.081 mmol) der Verbindung aus Beispiel 66A und 21.4 mg (0.089 mmol) 2-Methyl-4-(trifluormethoxy) benzylamin Hydrochlorid (Beispiel 5A) werden in 0.7 ml N,N-Dimethylformamid vorgelegt, mit 77 μl (0.44 mmol) N,N-Diisopropylethylamin und abschließend 105 mg (0.20 mmol) PyBOP versetzt. Die Reaktionsmischung wird 1.5 h bei RT rühren lassen, mit 1 ml 1N Salzsäure versetzt und dann als ganze über die präparative HPLC (Methode 5) getrennt. Man erhält 36 mg (70% d. Th.) der Titelverbindung.

LC-MS (Methode 3): $R_t$ = 3.24 min

MS (ES+): m/z = 634 $(M+H)^+$.

$^1$H-NMR (400 MHz, $CDCl_3$): δ = 1.01 (d, J = 6.9 Hz, 6H), 2.02 (m, 2H), 2.41 (s, 3H), 3.05 (dd, J = 4.1, 12.4 Hz, 2H), 3.20 (t, J = 11.7 Hz, 2H), 3.74 (s, 1H), 3.77 (s, 3H), 4.62 (d, J = 5.6 Hz, 2H), 5.26 (q, J = 8.0 Hz, 2H), 6.99-7.04 (m, 2H), 7.36 (d, J = 8.1 Hz, 1H), 7.87 (d, J = 12.5 Hz, 1H), 8.55 (s, 1H), 10.10 (t, J - 5.4 Hz, 1H).

Beispiel 33

6-Fluor-8-methoxy-N-[2-methyl-4-(trifluormethoxy)-benzyl]-4-oxo-7-(3-oxo-2,8-diazaspiro[4.5]dec-8-yl)-1-(2,2,2-trifluorethyl)-1,4-dihydrochinolin-3-carbonsäureamid

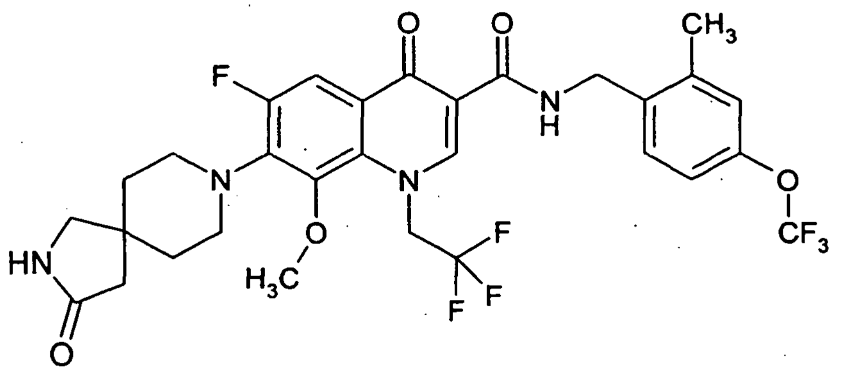

36 mg (0.076 mmol) der Verbindung aus Beispiel 69A und 22.1 mg (0.092 mmol) 2-Methyl-4-(trifluormethoxy) benzylamin Hydrochlorid (Beispiel 5A) werden in 1.0 ml N,N-Dimethylformamid vorgelegt, mit 73 $\mu$l (0.42 mmol) N,N-Diisopropylethylamin und abschließend 79.4 mg (0.15 mmol) PyBOP versetzt. Die Reaktionsmischung wird über Nacht bei RT rühren lassen dann als ganze über die präparative HPLC (Methode 5) getrennt. Man erhält 27 mg (54% d. Th.) der Titelverbindung.

LC-MS (Methode 1): $R_t$ = 2.73 min

MS (ES+): m/z = 659 $(M+H)^+$.

$^1$H-NMR (400 MHz, $CDCl_3$): $\delta$ = 1.84-1.88 (m, 4H), 2.34 (s, 2H), 2.41 (s, 3H), 3.31 (s, 2H), 3.32 (br.s, 4H), 3.84 (s, 3H), 4.62 (d, J = 5.6 Hz, 2H), 5.24 (q, J = 8.0 Hz, 2H), 6.99-7.03 (m, 2H), 7.36 (d, J = 7.7 Hz, 1H), 7.90 (d, J = 12.5 Hz, 1H), 8.57 (s, 1H), 10.05 (t, J ~ 5.5 Hz, 1H).

Beispiel 34

1-Cyclopropyl-6-fluor-8-methoxy-N-[2-methyl-4-(trifluormethoxy)-benzyl]-4-oxo-7-(3-oxo-2,8-diazaspiro[4.5]dec-8-yl)-1,4-dihydrochinolin-3-carbonsäureamid

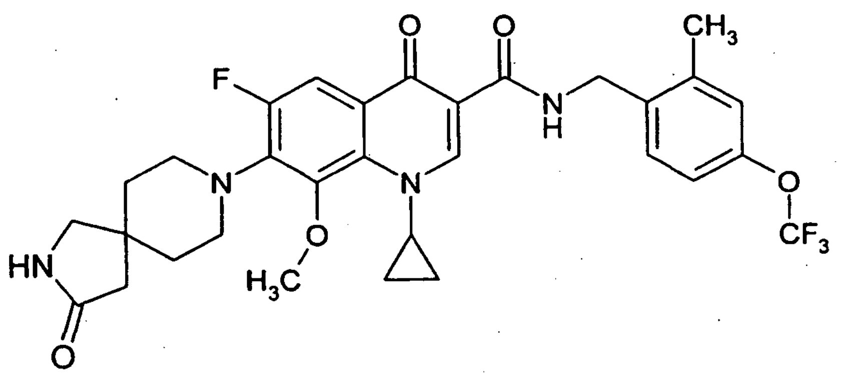

28 mg (0.065 mmol) der Verbindung aus Beispiel 68A und 18.9 mg (0.078 mmol) 2-Methyl-4-(trifluormethoxy) benzylamin Hydrochlorid (Beispiel 5A) werden in 0.8 ml N,N-Dimethylformamid vorgelegt, mit 62 $\mu$l (0.36 mmol) N,N-Diisopropylethylamin und abschließend 67.9 mg (0.13 mmol) PyBOP versetzt. Die Reaktionsmischung wird 30 min bei RT rühren lassen dann als ganze über die präparative HPLC (Methode 5) getrennt. Man erhält 27 mg (54% d. Th.) der Titelverbindung.

LC-MS (Methode 2): $R_t$ = 2.45 min

MS (ES+): m/z = 617 $(M+H)^+$.

$^1$H-NMR (400 MHz, $CDCl_3$): $\delta$ = 0.97 (m, 2H), 1.17 (m, 2H), 1.85 (m, 4H), 2.34 (s, 2H), 2.41 (s, 3H), 3.31 (s, 2H), 3.30-3.38 (m, 4H), 3.78 (s, 3H), 3.97 (m, 1H), 4.61 (d, J = 5.4 Hz, 2H), 5.58 (s, 1H), 7.005 (d, J - 8 Hz, 1H), 7.01 (s, 1H), 7.36 (d, J = 8.0 Hz, 1H), 7.85 (d, J = 12.4 Hz, 1H), 8.86 (s, 1H), 10.21 (br.s, 1H).

Analog zum Beispiel 1 werden auch die folgenden Beispiele 35 bis 42 hergestellt.

| Beispiel-Nr. | Struktur | Edukte Beispiel-Nr. | Analytikdaten LC-MS (Methode)/ Meßwerte HPLC (Methode)/ Meßwerte MS (Methode)/ Meßwerte |
|---|---|---|---|
| 35 | 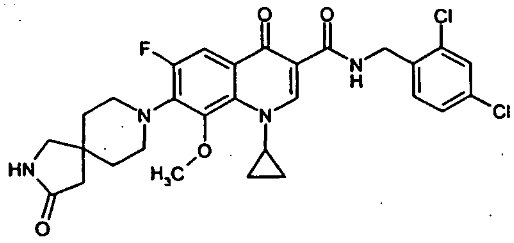 | 68A | LC-MS (Methode 2): $R_t$ = 2.43 min MS (ES+): m/z = 587 $(M+H)^+$ |
| 36 | 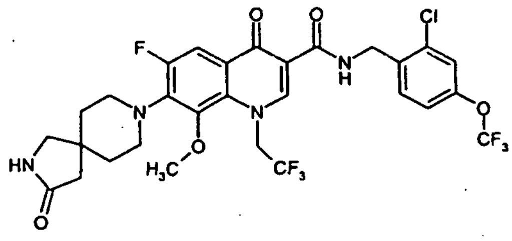 | 69A + 7A | LC-MS (Methode 3): $R_t$ = 2.86 min MS (ES+): m/z = 679 $(M+H)^+$ |
| 37 | 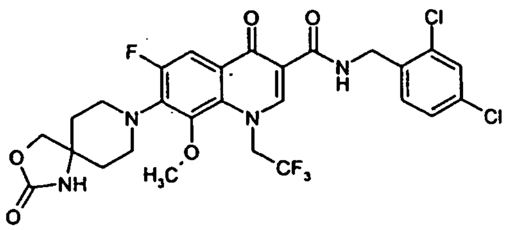 | 54A | LC-MS (Methode 3): $R_t$ = 2.90 min MS (ES+): m/z = 631 $(M+H)^+$ |
| **38** | 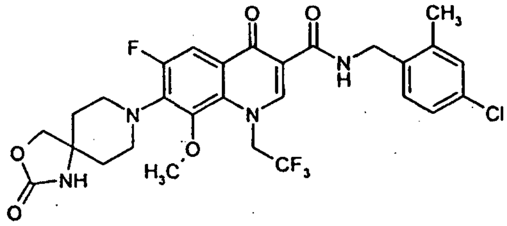 | 54A | LC-MS (Methode 1): $R_t$ = 2.67 min MS (ES+): m/z = 611 $(M+H)^+$ |
| **39** | 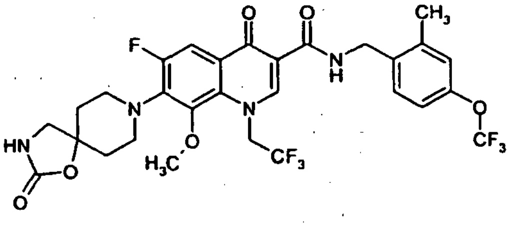 | 67A + 5A | LC-MS (Methode 2): $R_t$ = 2.57 min MS (ES+): m/z = 661 $(M+H)^+$ |

(fortgesetzt)

| Beispiel-Nr. | Struktur | Edukte Beispiel-Nr. | Analytikdaten LC-MS (Methode)/ Meßwerte HPLC (Methode)/ Meßwerte MS (Methode)/ Meßwerte |
|---|---|---|---|
| **40** | 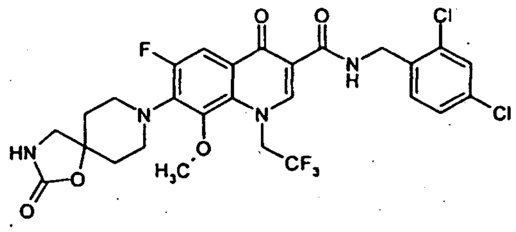 | 67A | LC-MS (Methode 1): $R_t$ = 2.72 min MS (ES+): m/z = 631 $(M+H)^+$ |
| **41** | 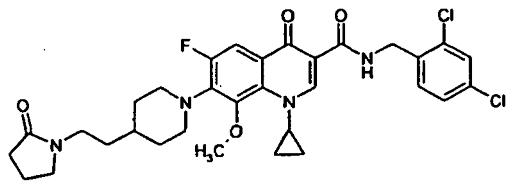 | 62A | LC-MS (Methode 3): $R_t$ = 3.29 min MS (ES+): m/z = 629 $(M+H)^+$ |
| **42** | 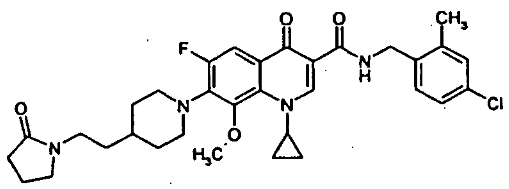 | 62A | LC-MS (Methode 3): $R_t$ = 3.24 min MS (ES+): m/z = 609 $(M+H)^+$ |

**Beispiel 43**

8-Chlor-7-{4-[(cyclohexylamino)carbonyl]piperidin-1-yl}-1-cyclopropyl-N-(2,4-dichlorbenzyl)-6-fluor-4-oxo-1,4-dihydrochinolin-3-carboxamid

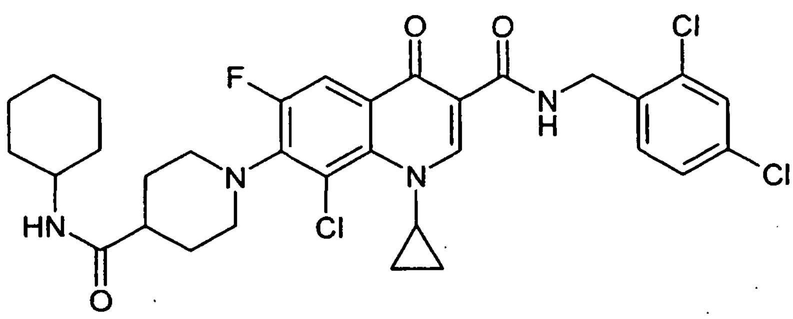

200 mg (0.44 mmol) der Verbindung aus Beispiel 70A und 138 mg (0.66 mmol) 4-(Cyclohexylamino)carbonylpiperidin (Herstellung: siehe WO 2003031397) werden mit 91 µl (0.66 mmol) Triethylamin in 4 ml DMSO 7 h bei 120°C

erhitzt. Nach Abkühlung wird die ganze Reaktionsmischung durch präparative HPLC (Methode 6) getrennt. Man erhält 30 mg der Titelverbindung.

LC-MS (Methode 3): $R_t$ = 3.24 min

MS (ES+): m/z = 647 $(M+H)^+$

**Beispiel 44**

8-Chlor-1-cyclopropyl-N-(2,4-dichlorbenzyl)-6-fluor-7-(4-{[(2-hydroxy-1,1-dimethylethyl)amino]carbonyl}piperidin-1-yl)-4-oxo-1,4-dihydrochinolin-3-carboxamid

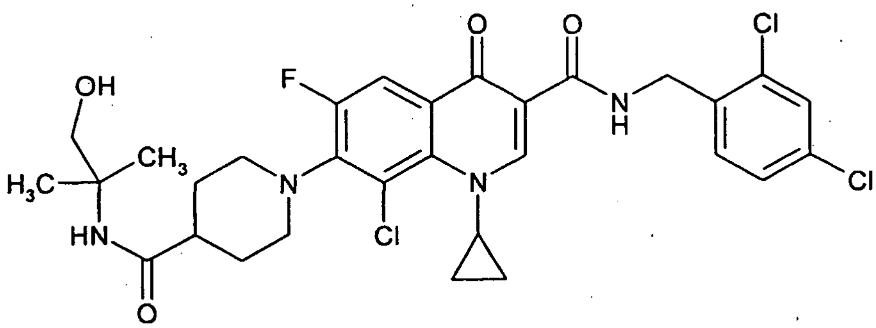

Nach dem gleichen Verfahren wie für Beispiel 43A werden aus 200 mg (0.44 mmol) der Verbindung aus Beispiel 70A und 131 mg (0.66 mmol) 4-{(2-Hydroxy-1,1-dimethyl-ethyl)-aminocarbonyl}-piperidin (Herstellung: siehe GB932487 (1960)) 23 mg (8% d. Th.) der Titelverbindung erhalten.

LC-MS (Methode 1): $R_t$ = 2.65 min

MS (ES+): m/z = 637 $(M+H)^+$

Analog zu Beispiel 44 werden die Beispiele 45 und 46 hergestellt.

| Beispiel-Nr. | Struktur | Edukt Beispiel- Nr. | Analytikdaten LC-MS (Methode)/ Meßwerte HPLC (Methode)/ Meßwerte MS (Methode)/ Meßwerte |
|---|---|---|---|
| 45 | 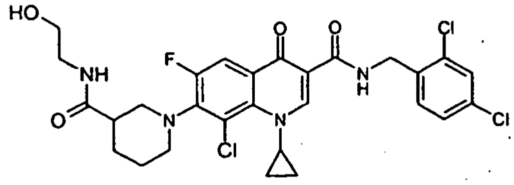 | 70A | LC-MS (Methode 3): $R_t$ = 2.73 min MS (ES+): m/z = 609 $(M+H)^+$ |
| 46 | 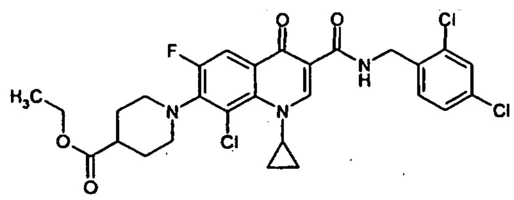 | 70A | LC-MS (Methode 1): $R_t$ = 3.35 min MS (ES+): m/z = 594 $(M+H)^+$ |

**Beispiel 47**

1-[6-Fluor-8-methoxy-3-({[2-methyl-4-(trifluormethoxy)benzyl]amino}carbonyl)-4-oxo-1-(2,2,2-trifluorethyl)-1,4-dihydrochinolin-7-yl]piperidin-4-carbonsäure

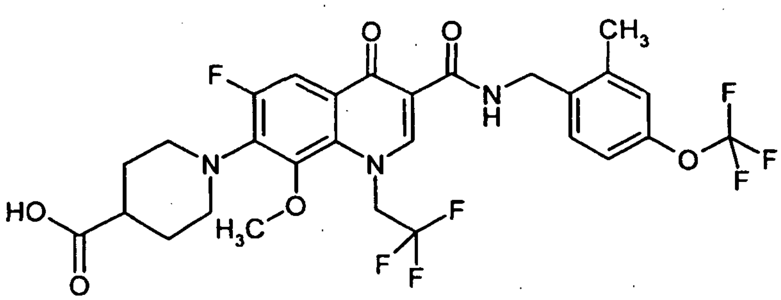

550 mg (0.698 mmol) der Verbindung aus Beispiel 2 werden in 10 ml Dioxan vorgelegt und mit 3.5 ml einer 1M Lithiumhydroxid-Lösung in Wasser versetzt und über Nacht gerührt. Die Reaktionsmischung wird mit 1N Salzsäure angesäuert und am Rotationsverdampfer von den Lösungsmitteln befreit. Der Rückstand wird in DMSO aufgenommen und durch präparative Chromatographie (Methode 5) getrennt. Man erhält 330 mg (72% d. Th.) der Titelverbindung.
HPLC (Methode 8): $R_t$ = 4.67 min.
MS (ES+): m/z = 634 $(M+H)^+$
$^1$H-NMR (400 MHz, $CDCl_3$): $\delta$ = 1.87-1.99 (m, 2H), 2.08 (br dd, J = 3, 13 Hz, 2H), 2.41 (s, 3H), 2.60 (tt, J = 4.0, 11.1 Hz, 1H), 3.23 (br. t, J = 12 Hz, 2H), 3.50 (br.d, J = 12 Hz, 2H), 3.85 (s, 3H), 4.63 (d, J = 5.7 Hz, 2H), 5.27 (q, J = 8.0 Hz, 2H), 7.00-7.50 (m, 2H), 7.36 (d, J = 8.1 Hz, 1H), 7.90 (d, J = 12.3 Hz, 1H), 8.62 (s, 1H), 10.10 (t, J = 5.7 Hz, 1H).

**Beispiel 48**

1-[3-(([2-Chlor-4-(trifluormethoxy)benzyl]amino)carbonyl)-6-fluor-8-methoxy-4-oxo-1-(2,2,2-trifluorethyl)-1,4-dihydrochinolin-7-yl]piperidin-4-carbonsäure

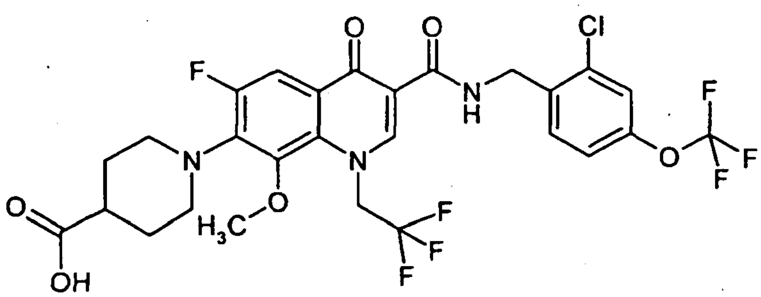

40 mg (0.059 mmol) der Verbindung aus Beispiel 3 werden in 2 ml Dioxan gelöst, mit 293 µl (5 Äq.) einer 1M Lithiumhydroxid-Lösung versetzt und die Mischung bis zur vollständigen Umsetzung (2 Tage) bei RT gerührt. Die Reaktionsmischung wird mit 1N Salzsäure angesäuert, mit wenig DMSO versetzt und die gesamte Rohlösung über präparative HPLC (Methode 5) getrennt. Man erhält 25 mg (65% d. Th.) der Titelverbindung.
LC-MS (Methode 1): $R_t$ = 2.95 min.
MS (ESI pos): m/z = 654 $(M+H)^+$
$^1$H-NMR (400 MHz, $CDCl_3$): $\delta$ = 1.87-1.99 (m, 2H), 2.04-2.13 (m, 2H), 2.60 (m, 1H), 3.23 (br. t, J = 12 Hz, 2H), 3.51 (br.d, J = 12 Hz, 2H), 3.84 (s, 3H), 4.73 (d, J = 5.9 Hz, 2H), 5.26 (q, J = 8.0 Hz, 2H), 7.10 (d, J = 8.5 Hz, 2H), 7.27 (unter $CHCl_3$ Signal, 1H), 7.49 (d, J = 8.6 Hz, 1H), 7.92 (d, J = 12.3 Hz, 1H), 8.58 (s, 1H), 10.27 (t, J = 5.9 Hz, 1H).

**Beispiel 49**

1-[6-Fluor-1-(2-fluorethyl)-8-methoxy-3-(((2-methyl-4-(trifluormethoxy)benzyl)-amino)carbonyl)-4-oxo-1,4-dihydrochinolin-7-yl]piperidin-4-carbonsäure

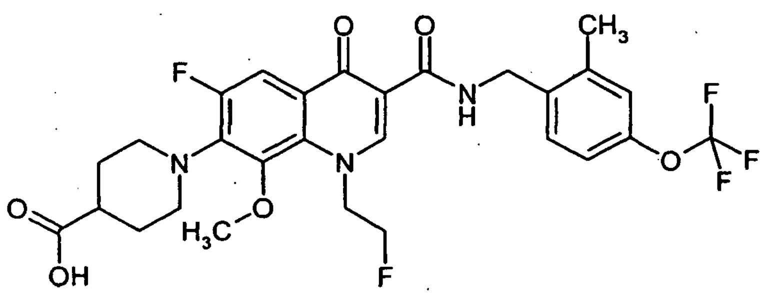

60 mg (0.096 mmol) der Verbindung aus Beispiel 4 werden in 2.35 ml Dioxan gelöst, mit 480 μl (5 Äq.) einer 1M Lithiumhydroxid-Lösung versetzt und die Mischung bis zur vollständigen Umsetzung (4 h) bei RT gerührt. Die Reaktionsmischung wird mit 1N Salzsäure angesäuert und mit Essigsäureethylester und Wasser verdünnt. Nach der Phasentrennung wird die organische Phase noch einmal mit Wasser und dann mit gesättigter Natriumchlorid-Lösung gewaschen, über Magnesiumsulfat getrocknet und am Rotationsverdampfer von Lösungsmitteln befreit. Der Rückstand wird im Hochvakuum getrocknet. Man erhält 54 mg (94% d. Th.) der Titelverbindung.

LC-MS (Methode 3): $R_t$ = 2.76 min.

MS (ESI pos): m/z = 598 $(M+H)^+$

$^1$H-NMR (400 MHz, DMSO-$d_6$): δ = 1.70 (br q, J = 11 Hz, 2H), 1.91 (br d, J = 11 Hz, 2H), 2.36 (s, 3H), 2.48 (m, 1H), 3.15 (br. t, J =11.5 Hz, 2H), 3.42 (br.d, J = 12 Hz, 2H), 3.76 (s, 3H), 4.53 (d, J = 5.7 Hz, 2H), 4.73 (br d, J = 47 Hz, 2H), 4.78 (br d, J = 38 Hz, 2H), 7.17 (br d, J = 8.5 Hz, 1H), 7.22 (br s, 1H), 7.36 (d, J = 8.5 Hz, 1H), 7.78 (d, J = 12.5 Hz, 1H), 8.71 (s, 1H), 10.19 (t, J = 5.7 Hz, 1H), 12.3 (br.s, 1H).

**Beispiel 50**

1-[3-{[(2,4-Dichlorbenzyl)amino]carbonyl}-6-fluor-8-methoxy-4-oxo-1-(2,2,2-trifluorethyl)-1,4-dihydrochinolin-7-yl]piperidin-4-carbonsäure

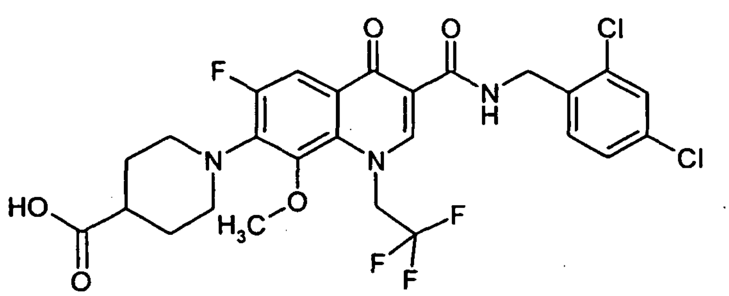

Analog zu Beispiel 49 werden aus 225 mg (0.356 mmol) der Verbindung aus Beispiel 1 durch Verseifung 200 mg (88% d. Th.) der Titelverbindung hergestellt.

HPLC (Methode 7): $R_t$ = 4.86 min.

MS (ES+): m/z = 604 $(M+H)^+$

$^1$H-NMR (400 MHz, $CDCl_3$): δ = 1.86-1.99 (m, 2H), 2.03-2.12 (m, 2H), 2.52 (m, 1H), 3.22 (br t, J = 12 Hz, 2H),

3.50 (br d, J = 12.3 Hz, 2H), 3.84 (s, 3H), 4.70 (d, J = 6.0 Hz, 2H), 5.27 (q, J = 8 Hz, 2H), 7.21 (dd, J = 2.0, 8.3 Hz, 1H), 7.385 (d, J = 8 Hz, 1H), 7.392 (d, J = 2 Hz, 1H), 7.92 (d, J = 12.4 Hz, 1H), 8.60 (s, 1H), 10.25 (t, J = 6.0 Hz, 1H).

**Beispiel 51**

[3-{[(2,4-Dichlorbenzyl)amino]carbonyl}-6-fluor-8-methoxy-4-oxo-1-(2,2,2-trifluorethyl)-1,4-dihydrochiriolin-7-yl] piperidin-3-yl-essigsäure

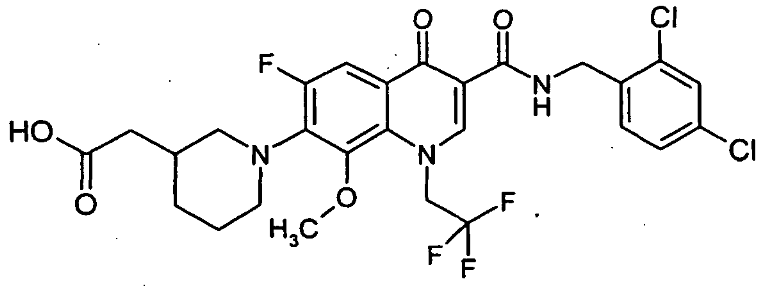

40 mg (0.062 mmol) der Verbindung aus Beispiel 6 wird in 3 ml THF / Wasser 5:1 vorgelegt, mit 7.4 mg LiOH (0.31 mmol, 5 Äq.) versetzt und die Reaktionsmischung 10 h bei 50°C verrührt. Die Lösungsmittel werden am Rotationsverdampfer entfernt und der Rückstand mit 1N HCl verrührt. Das ausgefallene Produkt wird abgesaugt und im HV getrocknet. Man erhält 39 mg der Titelverbindung (quant.)
LC-MS (Methode 1): $R_t$ = 2.99 min
MS (ES+): m/z = 618 $(M+H)^+$
$^1$H-NMR (400 MHz, DMSO-$d_6$): δ = 1.18-1.26 (m, 1H), 1.60-1.78 (m, 2H), 1.84 (m, 1H), 2.05 (m, 1H), 2.13-2.27 (m, 2H), 2.87 (br.t, J = 10.5 Hz, 1H), 3.08 (br.t, J = 11.5 Hz, 1H), 3.38 (1H ?, unter Wassersignal), 3.78 (s, 3H), 4.60 (d, J = 6.0 Hz, 2H), 5.70 (m, 2H), 7.38-7.45 (m, 2H), 7.64 (d, J = 1.7 Hz, 1H), 7.77 (d, J = 12.1 Hz, 1H), 8.83 (s, 1H), 10.14 (t, J = 6.0 Hz, 1H), 12.1 (br s, 1H).

**Beispiel 52**

1-[3-{[(2,4-Dichlorbenzyl)amino]carbonyl}-6-fluor-1-[(1S,2R)-2-fluorcyclopropyl]-8-methoxy-4-oxo-1,4-dihydrochinolin-7-yl]piperidin-4-carbonsäure

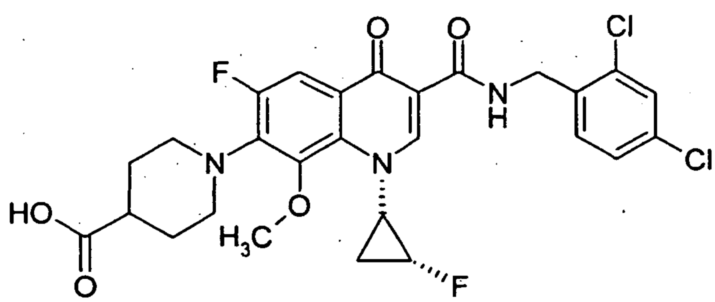

Diese Verbindung wird aus dem Beispiel 13 (32 mg 0.053 mmol) nach dem für Beispiel 51 beschriebenen Verfahren hergestellt. Man erhält 30 mg (98 % d. Th.) der Titelverbindung.
LC-MS (Methode 1): $R_t$ = 2.70 min
MS (ES+): m/z = 580 $(M+H)^+$
$^1$H-NMR (400 MHz, DMSO-$d_6$): δ = 1.45-1.65 (m, 2H), 1.65-1.80 (m, 2H), 1.95 (br.d, J = 12.5 Hz, 2H), 2.49

(m, 1H), 3.10-3.24 (m, 2H), 3.35-3.48 (m, 2H), 3.78 (s, 3H), 4.08 (m, 1H), 4.53-4.63 (m, 2H), 5.01 (dq, J = 65.2, ~3 Hz, 1H), 7.35-7.45 (m, 2H), 7.64 (d, J = 1.9 Hz, 1H), 7.73 (d, J = 12.5 Hz, 1H), 8.67 (s, 1H), 10.31 (t, J = 6.Hz, 1H), 12.3 (br s, 1H).

**Beispiel 53**

[6-Fluor-8-methoxy-3-({[2-methyl-4-(trifluormethoxy)benzyl]amino}carbonyl)-4-oxo-1-(2,2,2-trifluorethyl)-1,4-dihydrochinolin-7-yl]piperidin-4-yl-essigsäure

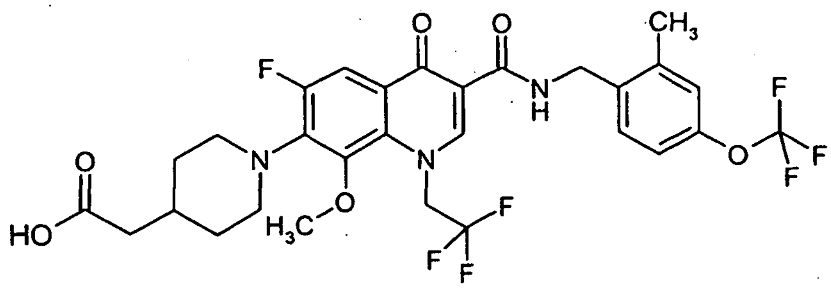

60 mg (0.089 mmol) der Verbindung aus Beispiel 5 werden in 2.2 ml Dioxan und 444 μl LiOH 1M (5 Äq.) in Wasser bei RT über Nacht verrührt. Die Mischung wird mit 1N-HCl angesäuert und mit Essigester verdünnt. Sie wird 2 mal mit Wasser und 1 mal mit ges. NaCl-Lösung ausgeschüttelt. Die organische Phase wird über Magnesiumsulfat getrocknet und am Rotationsverdampfer vom Lösungsmittel befreit. Der Rückstand wird im HV getrocknet. Man erhält 57 mg der Titelverbindung (94 % d. Th.).

LC-MS (Methode 3): $R_t$ = 3.01 min.

MS (ESI pos): m/z = 648 $(M+H)^+$

$^1$H-NMR (400 MHz, $CDCl_3$): δ = 1.49 (m, 2H), 1.87 (br.d, J = 11 Hz, 2H), 2.05 (m, 1H), 2.39 (d, J = 7.0 Hz, 2H), 2.40 (s, 3H), 3.21 (br. t, J = 12.2 Hz, 2H), 3.46 (br.d, J ~12.5 Hz, 2H), 3.81 (s, 3H), 4.62 (d, J = 5.6 Hz, 2H), 5.26 (q, J = 8.0 Hz, 2H), 7.00-7.04(m, 2H), 7.36 (d, J = 8.0 Hz, 1H), 7.88 (d, J = 12.5 Hz, 1H), 8.59 (s, 1H), 10.10 (t, J = 5.6 Hz, 1H).

Analog zu Beispiel 47 werden folgende Carbonsäuren der Beispiele 54 bis 71 aus den entsprechenden Estern hergestellt.

| Beispiel-Nr. | Struktur | Edukt Beispiel-Nr. | Analytikdaten LC-MS (Methode)/ Meßwerte HPLC (Methode)/ Meßwerte MS (Methode)/ Meßwerte |
|---|---|---|---|
| 54 | 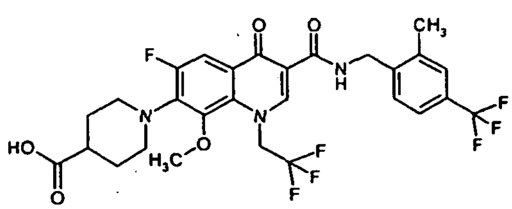 | 8 | LC-MS (Methode 1): $R_t$ = 2.82 min MS (ES+): m/z = 618 $(M+H)^+$ |

(fortgesetzt)

| Beispiel-Nr. | Struktur | Edukt Beispiel-Nr. | Analytikdaten LC-MS (Methode)/ Meßwerte HPLC (Methode)/ Meßwerte MS (Methode)/ Meßwerte |
|---|---|---|---|
| 55 | 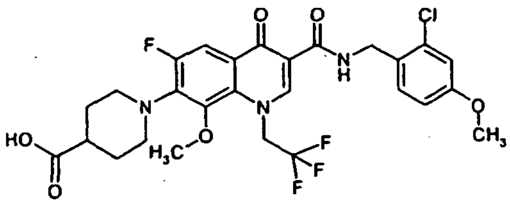 | 9 | LC-MS (Methode 1): $R_t$ = 2.65 min MS (ES+): m/z = 600 $(M+H)^+$ |
| 56 | 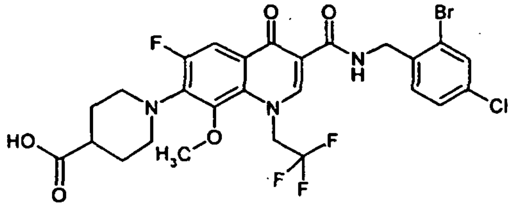 | 10 | LC-MS (Methode 1): $R_t$ = 2.92 min MS (ES+): m/z = 649 / 651 $(M+H)^+$ |
| 57 | 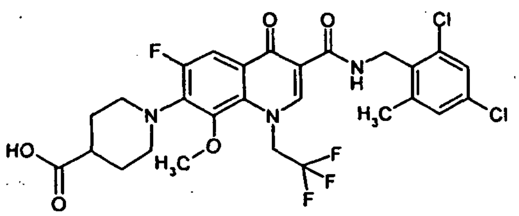 | 11 | LC-MS (Methode 1): $R_t$ = 2.92 min MS (ES+): m/z = 618 $(M+H)^+$ |
| 58 | 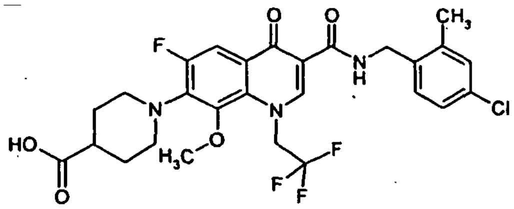 | 7 | HPLC (Methode 7): $R_t$ = 4.77 min MS (ESI+): m/z = 584 $(M+H)^+$ |
| 59 | 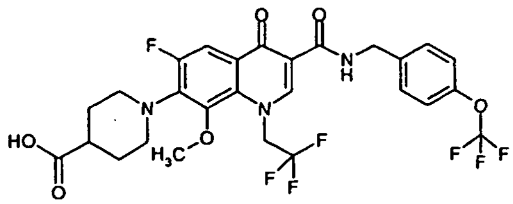 | 12 | LC-MS (Methode 2): $R_t$ = 2.61 min MS (ES+): m/z = 620 $(M+H)^+$ |

(fortgesetzt)

| Beispiel-Nr. | Struktur | Edukt Beispiel-Nr. | Analytikdaten LC-MS (Methode)/ Meßwerte HPLC (Methode)/ Meßwerte MS (Methode)/ Meßwerte |
|---|---|---|---|
| 60 | 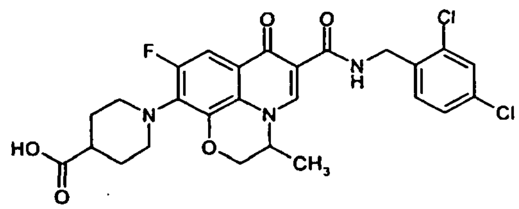 | 14 | LC-MS (Methode 3): $R_t$ = 2.67 min MS (ES+): m/z = 548 $(M+H)^+$ |
| 61 | 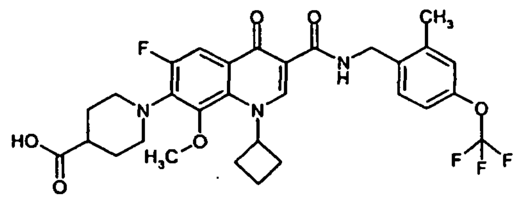 | 15 | LC-MS (Methode 3): $R_t$ = 2.97 min MS (ES+): m/z = 606 $(M+H)^+$ |
| 62 | 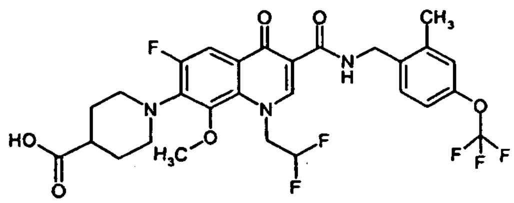 | 17 | LC-MS (Methode 2): $R_t$ = 2.59 min MS (ES+): m/z = 616 $(M+H)^+$ |
| 63 | 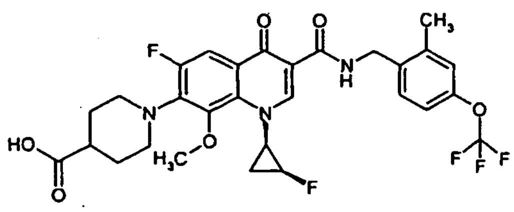 | 16 | LC-MS (Methode 3): $R_t$ = 2.81 min MS (ES+): m/z = 610 $(M+H)^+$ |
| 64 | 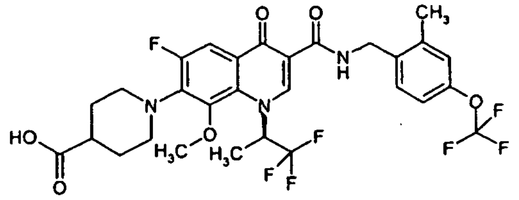 | 18 | LC-MS (Methode 2): $R_t$ = 2.79 min MS (ES+): m/z = 648 $(M+H)^+$ |
| 65 | 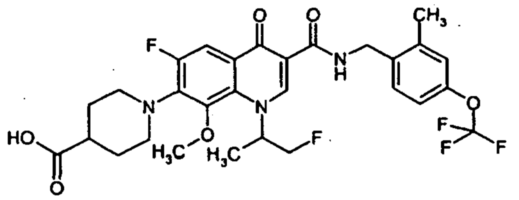 | 19 | LC-MS (Methode 2): $R_t$ = 2.60 min MS (ES+): m/z = 612 $(M+H)^+$ |

(fortgesetzt)

| Beispiel-Nr. | Struktur | Edukt Beispiel-Nr. | Analytikdaten LC-MS (Methode)/ Meßwerte HPLC (Methode)/ Meßwerte MS (Methode)/ Meßwerte |
|---|---|---|---|
| 66 | 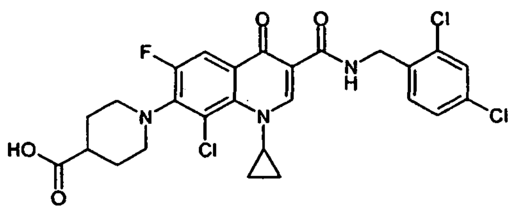 | 45 | LC-MS (Methode 2): $R_t$ = 2.67 min MS (ES+): m/z = 566 $(M+H)^+$ |
| 67 | 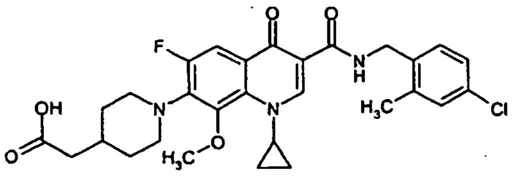 | 24 | LC-MS (Methode 1): $R_t$ = 2.75 min MS (ES+): m/z = 556 $(M+H)^+$ |
| 68 | 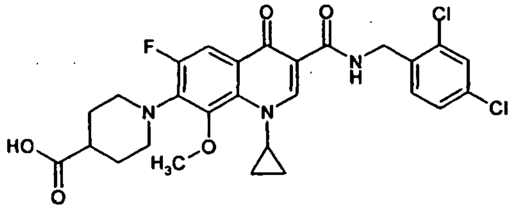 | 26 | LC-MS (Methode 1): $R_t$ = 2.76 min MS (ES+): m/z = 592 $(M+H)^+$ |
| 69 | 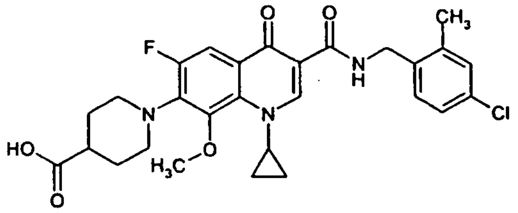 | 22 | LC-MS (Methode 1): $R_t$ = 2.67 min MS (ES+): m/z =542 $(M+H)^+$ |
| 70 | 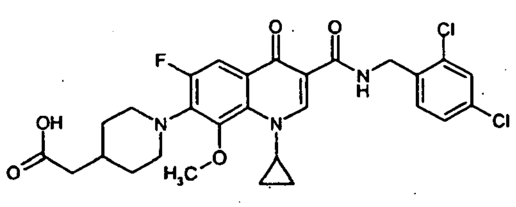 | 23 | LC-MS (Methode 1): $R_t$ = 2.84 min MS (ES+): m/z = 576 $(M+H)^+$ |

(fortgesetzt)

| Beispiel-Nr. | Struktur | Edukt Beispiel-Nr. | Analytikdaten LC-MS (Methode)/ Meßwerte HPLC (Methode)/ Meßwerte MS (Methode)/ Meßwerte |
|---|---|---|---|
| 71 | 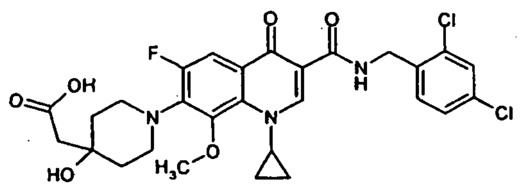 | 25 | LC-MS (Methode 2): $R_t$ = 2.37 min MS (ES+): m/z = 592 $(M+H)^+$ |

**Beispiel 72**

{(3S)-1-[6-Fluor-8-methoxy-3-({[2-methyt-4-(trifluormethoxy)-benzyl]amino}carbonyl)-4-oxo-1-(2,2,2-trifluorethyl)-1,4-dihydrochinolin-7-yl]piperidin-3-yl}essigsäure

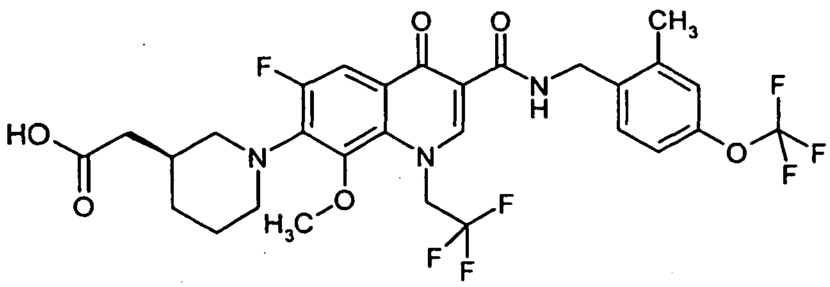

72 mg (0.107 mmol) der Verbindung aus Beispiel 20 werden mit 2.6 ml Dioxan und 533 µl LiOH (Lösung 1M in Wasser, 5 Äq.) bei RT über Nacht verrührt. Die Mischung wird mit 1N-HCl angesäuert und mit Essigester verdünnt. Sie wird 2 mal mit Wasser und 1 mal mit ges. NaCl-Lösung ausgeschüttelt. Die organische Phase wird über Magnesiumsulfat getrocknet und am Rotationsverdampfer vom Lösungsmittel befreit. Der Rückstand wird im HV getrocknet. Man erhält 70 mg der Titelverbindung (99 % d. Th.).

LC-MS (Methode 3): $R_t$ =3.07 min

MS (ES+): m/z = 648 $(M+H)^+$

$^1$H-NMR (400 MHz, $CDCl_3$): δ = 10.17 (t, J =5.6 Hz, 1H), 8.74 (s, 1H), 7.87 (d, J = 12.4 Hz, 1H), 7.35 (d, J = 8.1 Hz, 1H), 7.04-7.00 (m, 2H), 5.42-5.24 (m, 2H), 4.62 (d, J = 5.6 Hz, 2H), 3.87 (s, 3H), 3.53 (br. d, J ~ 11.5 Hz, 1H), 3.38 (br. d, J ~ 12 Hz, 1H), 3.17 (br.t, J ~ 12 Hz, 1H), 2.84 (br.t, J - 11 Hz, 1H), 2.41 (s, 3H), 2.36-2.31 (m, 2H), 2.31-2.22 (m, 1H), 2.00-1.92 (m, 1H), 1.85-1.72 (m, 2H), 1.30-1.20 (m, 1H).

**Beispiel 73**

{(3R)-1-[6-Fluor-8-methoxy-3-({[2-methyl-4-(trifluormethoxy)-benzyl]amino}carbonyl)-4-oxo-1-(2,2,2-trifluorethyl)-1,4-dihydrochinolin-7-yl]piperidin-3-yl}essigsäure

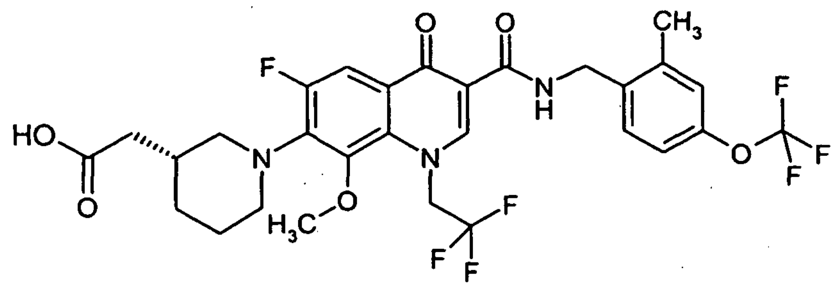

83 mg (0.123 mmol) der Verbindung aus Beispiel 21 werden mit 3.0 ml Dioxan und 614 µl LiOH (Lösung 1M in Wasser, 5 Äq.) bei RT über Nacht verrührt. Die Mischung wird mit 1N-HCl angesäuert und mit Essigester verdünnt. Sie wird 2 mal mit Wasser und 1 mal mit ges. Nacl-Lösung ausgeschüttelt. Die organische Phase wird über Magnesiumsulfat getrocknet und am Rotationsverdampfer vom Lösungsmittel befreit. Der Rückstand wird im HV getrocknet. Man erhält 73 mg der Titelverbindung (90%d.Th.).

LC-MS (Methode 3): $R_t$ = 3.07 min

MS (ES+): m/z = 648 $(M+H)^+$

$^1$H-NMR (400 MHz, $CDCl_3$): δ = 10.17 (t, J = 5.6 Hz, 1H), 8.74 (s, 1H), 7.87 (d, J = 12.4 Hz, 1H), 7.35 (d, J = 8.1 Hz, 1H), 7.04-7.00 (m, 2H), 5.42-5.24 (m, 2H), 4.62 (d, J = 5.6 Hz, 2H), 3.87 (s, 3H), 3.53 (br. d, J - 11.5 Hz, 1H), 3.38 (br. d, J ~ 12 Hz, 1H), 3.17 (br.t, J - 12 Hz, 1H), 2.84 (br.t, J - 11 Hz, 1H), 2.41 (s, 3H), 2.36-2.31 (m, 2H), 2.31-2.22 (m, 1H), 2.00-1.92 (m, 1H), 1.85-1.72 (m, 2H), 1.30-1.20 (m, 1H).

Die absolute Stereochemie wird durch eine Röntgenstrukturanalyse bestätigt.

**Beispiel 74**

8-Chlor-1-cyclopropyl-N-(2,4-dichlorbenzyl)-6-fluor-4-oxo-7-(3-oxo-2,8-diazaspiro[4.5]dec-8-yl)-1,4-dihydrochinolin-3-carboxamid

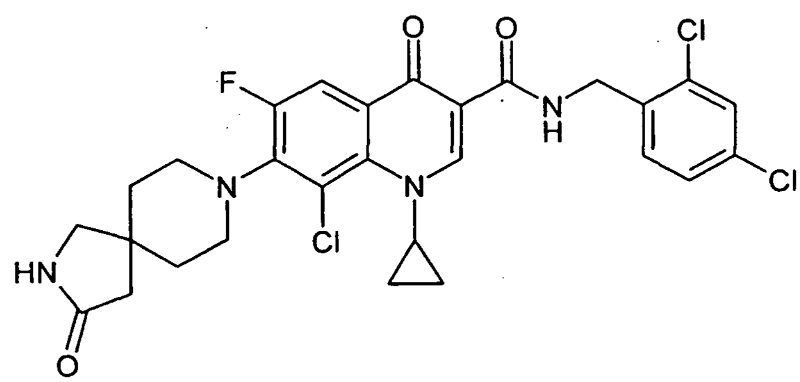

60 mg (0.13 mmol) der Verbindung aus Beispiel 70A und 37 mg (0.20 mmol) 3-Oxo-2,8-diazaspiro[4,5]decan Hydrochlorid (Beispiel 13A) werden mit 91 µl (0.52 mmol) N,N-Diisopropylethylamin in 2 ml DMSO 2 Tage bei 120°C verrührt. Nach Abkühlung wird die gesamte Reaktionsmischung über präparative HPLC (Methode 5) getrennt. Nach Einengen der geeigneten Fraktionen am Rotationsverdampfer und Trocknung im Hochvakuum erhält man 20 mg (26% d. Th.) der Titelverbindung.

LC-MS (Methode 3): $R_t$ = 2.73 min

MS (ES+): m/z = 592 $(M+H)^+$.

$^1$H-NMR (400 MHz, $CDCl_3$): δ = 0.88-0.95 (m, 3H), 1.20-1.26 (m, 2H), 1.85-1.91 (m, 4H), 2.34 (s, 2H), 3.31 (br.s, 6H), 4.27 (m, 1H), 4.69 (d, J = 6.2 Hz, 2H), 5.44 (br s, 1H), 7.21 (dd, J = 2.0, 8.3 Hz, 1H), 7.3 7-7.40 (m, 2H), 8.01 (d, J = 12.1 Hz, 1H), 8.92 (s, 1H), 10.20 (t, J = 6.2 Hz, 1H).

**Beispiel 75**

1-[6-Fluor-8-methoxy-3-({[2-methyl-4-(trifluormethoxy)benzyl]amino}carbonyl)-4-oxo-1-(2,2,2-trifluorethyl)-1,4-dihydrochinolin-7-yl]piperidin-4-carbonsäure Diethanolamin-Salz

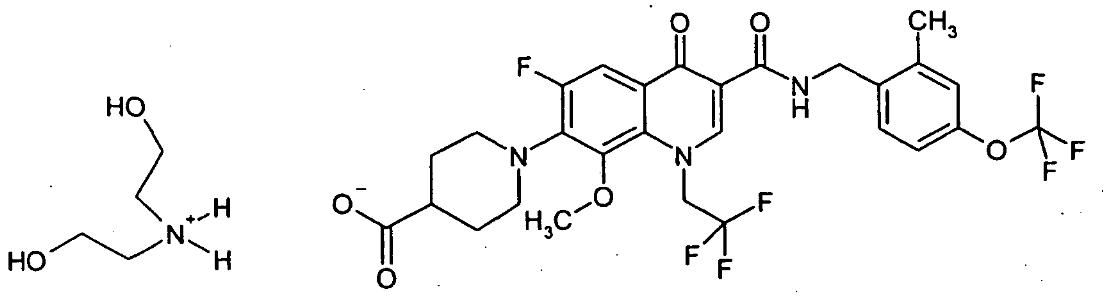

400 mg (0.63 mmol) 1-[6-Fluor-8=methoxy-3-({[2-methyl-4-(trifluormethoxy)benzyl]amino}carbonyl)-4-oxo-1-(2,2,2-trifluorethyl)-1,4-dihydrochinolin-7-yl]piperidin-4-carbonsäure (Beispiel 47) werden in 20 ml entionisiertem Wasser und 20 ml Acetonitril bei RT suspendiert. Dazu werden 60.5 μl (66.4 mg , 0.63 mmol) Diethanolamin zugegeben und die Mischung über Nacht bei RT verrührt. Die entstandene Lösung wird am Rotationsverdampfer vom Acetonitril befreit. Die verbleibende wässrige Lösung wurde eingefroren und lyophilisiert. Man erhält 475 mg (100% d. Th.) Rückstand, der analytisch der Titelverbindung entspricht.

$^1$H-NMR (400 MHz, $CDCl_3$): δ = 1.77-1.90 (m, 2H), 2.01 (br d, J = 13 Hz, 2H), 2.38 (m, 1H), 2.40 (s, 3H), 3.03-3.09 (m, 4H), 3.18 (br. t, J = 12 Hz, 2H), 3.49 (br.d, J = 12 Hz, 2H), 3.83 (s, 3H), 3.86-3.89 (m, 4H), 4.62 (d, J = 5.7 Hz, 2H), 5.27 (q, J = 8.0 Hz, 2H), 6.99-7.05 (m, 2H), 7.35 (d, J = 8.1 Hz, 1H), 7.86 (d, J = 12.5 Hz, 1H), 8.57 (s, 1H), 10.10 (t, J = 5.7 Hz, 1H).

**Beispiel 76**

1-[6-Fluor-8-methoxy-3-({[2-methyl-4-(trifluormethoxy)benzyl]amino}carbcnyl)-4-oxo-1-(2,2,2-trifluorethyl)-1,4-dihydrochinolin-7-yl]piperidin-4-carbonsäure Cholin-Salz

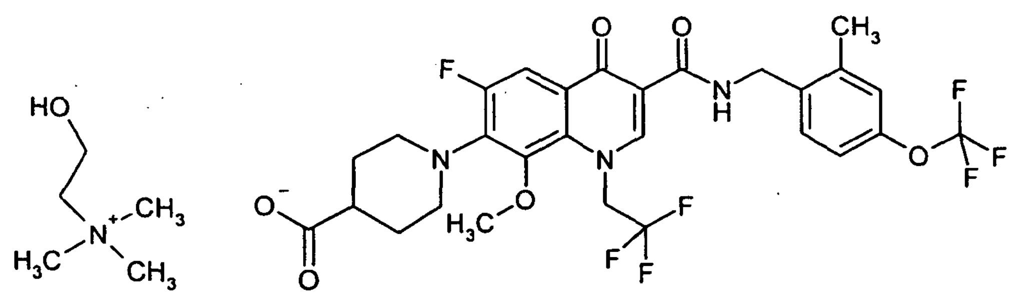

400 mg (0.63 mmol) 1-[6-Fluor-8-methoxy-3-(([2-methyl-4-(trifluormethoxy)benzyl]amino)carbonyl)-4-oxo-1-(2,2,2-trifluorethyl)-1,4-dihydrochinolin-7-yl]piperidin-4-carbonsäure (Beispiel 47) werden in 20 ml entionisiertem Wasser und 20 ml Acetonitril bei RT suspendiert. Dazu werden 140 μl (153 mg, 0.63 mmol) β-Hydroxyethyltrimethylammonium Hydroxid ("Cholinhydroxid") zugegeben und die Mischung über Nacht bei RT verrührt. Die entstandene Lösung wird am Rotationsverdampfer vom Acetonitril befreit. Die verbleibende wässrige Lösung wurde eingefroren und lyophilisiert. Man erhält 494 mg (100% d. Th.) Rückstand, der analytisch der Titelverbindung entspricht.

$^1$H-NMR (400 MHz, $CDCl_3$): δ = 1.88 (dq, J = 3.8, ~12 Hz, 2H), 2.01 (br d, J - 12 Hz, 2H), 2.33 (tt, J = 3.6, 11.6 Hz, 1H), 2.40 (s, 3H), 3.18 (br. t, J = 12 Hz, 2H), 3.49 (br.d, J - 12 Hz, 2H), 3.83 (br.s, 2H), 3.835 (s, 3H), 4.22 (br.s,

2H), 4.62 (d, J = 5.7 Hz, 2H), 5.27 (q, J = 8.0 Hz, 2H), 7.00-7.05 (m, 2H), 7.35 (d, J = 8.3 Hz, 1H), 7.845 (d, J = 12.5 Hz, 1H), 8.54 (s, 1H), 10.10 (t, J = 5.7 Hz, 1H).

**Beispiel 77**

1-[6-Fluor-8-hydroxy-3-({[2-methyl-4-(trifluormethoxy)benzyl]amino}carbonyl)-4-oxo-1-(2,2,2-trifluorethyl)-1,4-dihydrochinolin-7-yl]piperidin-4-carbonsäure

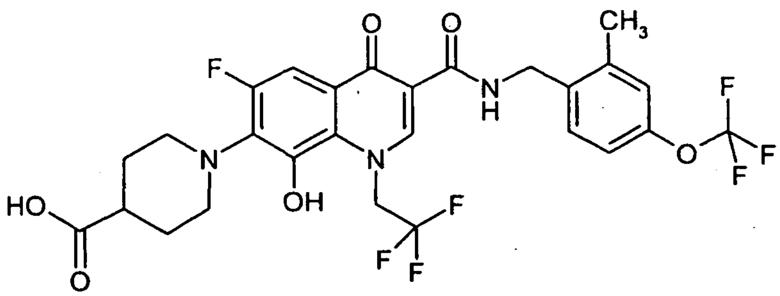

150 mg (0.237 mmol) der Verbindung aus Beispiel 47 werden in 3 ml Dichlormethan vorgelegt, mit 943 μl Trimethylsilyliodid (6.63 mmol) versetzt und die Mischung 4 Tage bei RT verrührt. Um das überschüssige Trimethylsilyliodid abzufangen, wird die Reaktionsmischung auf 0°C gekühlt, mit einer Mischung aus 414 μl Ethanol (7.1 mmol) und 575 μl Pyridin (7.1 mmol) versetzt. Nach 5 min entfernt man die flüchtigen Komponenten am Rotationsverdampfer. Der Rückstand wird in 5 ml Wasser - Acetonitril Mischung (1:1) verrührt und der Feststoff abfiltriert. Dieser wird im HV getrocknet. Man erhält 136 mg der Titelverbindung (91 % d. Th.).

LC-MS (Methode 2): $R_t$ = 2.68 min

MS (ES+): m/z = 620 $(M+H)^+$

$^1$H-NMR (400 MHz, $CDCl_s$): δ = 10.06 (t, J = 5.7 Hz, 1H), 8.69 (br. s, 1H), 8.66 (s, 1H), 7.72 (d, J = 11.5 Hz, 1H), 7.36 (d, J = 8.1 Hz, 1H), 7.04 (s, 1H), 7.03 (d, J - 8Hz, 1H), 5.38 (q, J = 7.8 Hz, 2H), 4.63 (d, J = 5.7 Hz, 2H), 3.32 (br. t, J - 12 Hz, 2H), 3.02 (br.d, J - 12 Hz, 2H), 2.59 (m, 1H), 2.41 (s, 3H), 2.21 (br.d, J ~ 13 Hz, 2H), 1.97-1.83 (m, 2H).

**Beispiel 78**

1-[8-Ethoxy-6-fluor-3-({[2-methyl-4-(trifluormethoxy)benzyl]amino}carbonyl)-4-oxo-1-(2,2,2-trifluorethyl)-1,4-dihydrochinolin-7-yl]piperidin-4-carbonsäureethylester

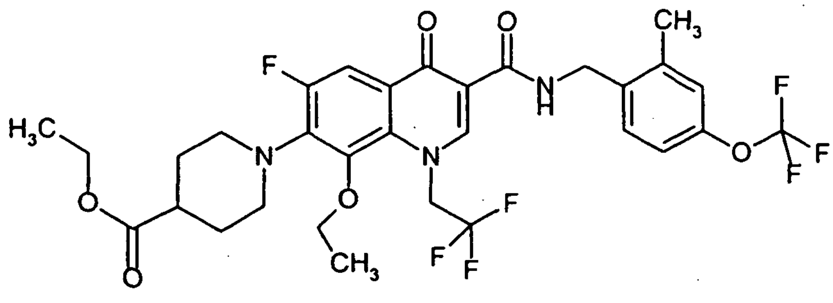

110 mg (0.178 mmol) der Verbindung aus Beispiel 77, 135 mg Kaliumcarbonat (0.98 mmol) und 142 μl Ethyliodid (1.78 mmol) werden mit 2.0 ml DMF in einem verschlossenem Gefäß bei 80°C während 4 h verrührt. Nach Abkühlung auf RT wird die Mischung auf 30 ml Wasser gegossen. Nach kurzem Rühren der Suspension wird der Feststoff abgesaugt,

mit Wasser gewaschen und im HV getrocknet. Man erhält 111 mg der Titelverbindung (93 % d. Th.).

LC-MS (Methode 1): $R_t$ = 3.37 min

MS (ES+): m/z = 676 $(M+H)^+$

$^1$H-NMR (400 MHz, DMSO-$d_6$): δ = 10.02 (t, J = 5.8 Hz, 1H), 8.85 (s, 1H), 7.76 (d, J = 12.5 Hz, 1H), 7.36 (d, J = 8.3 Hz, 1H), 7.22 (s, 1H), 7.17 (d, J - 8.7, 1H), 5.75 (q, J = 8.6 Hz, 2H), 4.55 (d, J = 5.9 Hz, 2H), 4.10 (q, J = 7.1 Hz, 2H), 3.99 (q, J = 7.1 Hz, 2H), 3.43-3.28 (m, 2H), 3.17 (br.t, J ~ 12 Hz, 2H), ca. 2.55 (m, 1H), 2.37 (s, 3H), 1.93 (br.d, J ~ 12 Hz, 2H), 1.77-1.64 (m, 2H), 1.33 (t, J = 7.1 Hz, 3H), 1.20 (t,J= 7.1 Hz, 3H).

## Beispiel 79

1-[8-Ethoxy-6-fluor-3-({[2-methyl-4-(trifluormethoxy)benzyl]amino}carbonyl)-4-oxo-1-(2,2,2-trifluorethyl)-1,4-dihydrochinolin-7-yl]piperidin-4-carbonsäure

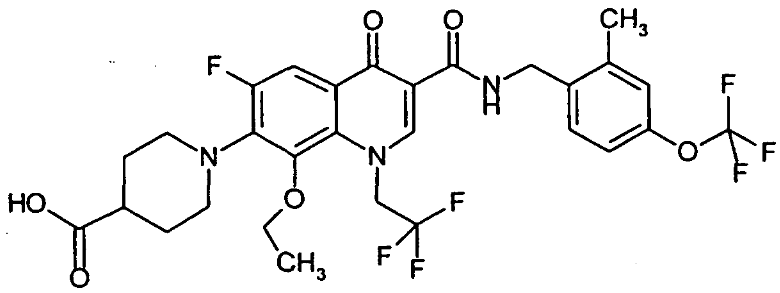

85 mg (0.126 mmol) der Verbindung aus Beispiel 78 werden mit 2 ml Methanol, 2.5 ml DMF und 2 ml Natronlauge (2N) versetzt. Die Mischung wird 1 h bei RT verrührt, dann mit 1N-HCl auf pH 1 angesäuert, mit Wasser verdünnt und drei mal mit Essigsäureethylester extrahiert. Die vereinigten organischen Phasen werden mit ges. Natriumchlorid-Lösung gewaschen, über Natriumsulfat getrocknet und am Rotationsverdampfer vom Lösungsmittel befreit. Der Rückstand wird im HV getrocknet. Man erhält 81 mg der Titelverbindung (96 % d. Th.).

LC-MS (Methode 2): $R_t$ = 2.78 min

MS (ES+): m/z = 648 $(M+H)^+$

$^1$H-NMR (500 MHz, $CDCl_3$): δ = 10.11 (t, J = 5.4 Hz, 1H), 8.62 (s, 1H), 7.88 (d, J = 12.4 Hz, 1H), 7.36 (d, J = 8.0 Hz, 1H), 7.05-7.00 (m, 2H), 5.34 (q, J = 7.9 Hz, 2H), 4.63 (d, J = 5.6 Hz, 2H), 4.02 (q, J = 7.0 Hz, 2H), 3.54-3.48 (m, 2H), 3.21 (br.t, J ~ 12 Hz, 2H), 2.58 (m, 1H), 2.41 (s, 3H), 2.11-2.05 (m, 2H), 1.95-1.85 (m, 2H), 1.41 (t, J = 7.0 Hz, 3H).

## B. Bewertung der physiologischen Wirksamkeit

Die *in vitro*-Wirkung der erfindungsgemäßen Verbindungen kann in folgenden Assays gezeigt werden:

### Anti-HCMV- (Anti-Humanes Cytomegalo-Virus) Zytopathogenitätstests

Die Testverbindungen werden als 50 millimolare (mM) Lösungen in Dimethysulfoxid (DMSO) eingesetzt. Ganciclovir®, Foscarnet® und Cidofovir® dienen als Referenzverbindungen. Nach der Zugabe von jeweils 2 μl der 50, 5, 0.5 und 0.05 mM DMSO-Stammlösungen zu je 98 μl Zellkulturmedium in der Reihe 2 A-H in Doppelbestimmung werden 1:2-Verdünnungen mit je 50 μl Medium bis zur Reihe 11 der 96-WellPlatte durchgeführt. Die Wells in den Reihen 1 und 12 enthalten je 50 μl Medium. In die Wells werden dann je 150 μl einer Suspension von 1 x $10^4$ Zellen (humane Vorhautfibroblasten [NHDF]) pipettiert (Reihe 1 = Zellkontrolle) bzw. in die Reihen 2-12 ein Gemisch von HCMV-infizierten und nichtinfizierten NHDF-Zellen (M.O.I. = 0.001 - 0.002), d.h. 1-2 infizierte Zellen auf 1000 nicht-infizierte Zellen. Die Reihe 12 (ohne Substanz) dient als Viruskontrolle. Die End-Testkonzentrationen liegen bei 250 - 0.0005 μM. Die Platten werden 6 Tage bei 37˚C / 5% $CO_2$ inkubiert, d.h. bis in den Viruskontrollen alle Zellen infiziert sind (100% cytopathogener Effekt [CPE]). Die Wells werden dann durch Zugabe eines Gemisches von Formalin und Giemsa's Farbstoff fixiert und gefärbt (30 Minuten), mit aqua bidest, gewaschen und im Trockenschrank bei 50˚C getrocknet. Danach werden die Platten mit einem Overhead-Mikroskop (Plaque Multiplier der Firma Technomara) visuell ausgewertet.

Die folgenden Daten können von den Testplatten ermittelt werden:

$CC_{50}$ (NHDF) = Substanzkonzentration in μM, bei der im Vergleich zur unbehandelten Zellkontrolle keine

sichtbaren cytostatischen Effekte auf die Zellen erkennbar sind;
$EC_{50}$ (HCMV) = Substanzkonzentration in µM, die den CPE (cytopathischen Effekt) um 50% im Vergleich zur unbehandelten Viruskontrolle hemmt;

$$SI\ (Selektivitätsindex) = CC_{50}\ (NHDF)\ /\ EC_{50}\ (HCMV).$$

Repräsentative in-vitro-Wirkdaten für die erfindungsgemäßen Verbindungen sind in Tabelle A wiedergegeben:

Tabelle A

| Beispiel- Nr. | HCMV $EC_{50}$ [µM] | NHDF $CC_{50}$ [µM] |
|---|---|---|
| 1 | 0.009 | 94 |
| 2 | 0.005 | 47 |
| 3 | 0.008 | 24 |
| 7 | 0.018 | 94 |
| 13 | 0.019 | 23 |
| 27 | 0.013 | 21 |
| 28 | 0.014 | 21 |
| 30 | 0.034 | 11 |
| 31 | 0.008 | 16 |
| 32 | 0.017 | 11 |
| 33 | 0.017 | 19 |
| 34 | 0.040 | 11 |
| 36 | 0.014 | 21 |
| 47 | 0.004 | 86 |
| 48 | 0.003 | 20 |
| 49 | 0.006 | 94 |
| 50 | 0.012 | 47 |
| 51 | 0.026 | 47 |
| 53 | 0.005 | 94 |
| 54 | 0.022 | 21 |
| 55 | 0.031 | 16 |
| 57 | 0.026 | 23 |
| 58 | 0.006 | 47 |
| 61 | 0.007 | 21 |
| 62 | 0.001 | 148 |
| 63 | 0.008 | 47 |
| 65 | 0.079 | 106 |
| 67 | 0.061 | 21 |
| 73 | 0.003 | 47 |
| 75 | 0.002 | 47 |
| 76 | 0.002 | 47 |

(fortgesetzt)

| Beispiel- Nr. | HCMV $EC_{50}$ [μM] | NHDF $CC_{50}$ [μM] |
|---|---|---|
| 77 | 0.006 | 250 |
| 79 | 0.002 | 125 |

Die Eignung der erfindungsgemäßen Verbindungen zur Behandlung von HCMV-Infektionen kann im folgenden Tiermodell gezeigt werden:

**HCMV Xenograft-Gelfoam®-Modell**

Tiere:

5-6 Wochen alte immundefiziente Mäuse (16 - 20 g), Fox Chase SCID.NOD oder NOD.CB17-Prkdc/J werden von kommerziellen Züchtern (Taconic M&B, Dänemark; Jackson, USA) bezogen. Die Tiere werden unter sterilen Bedingungen (einschließlich Streu und Futter) in Isolatoren gehalten.

Virusanzucht:

Humanes Cytomegalovirus (HCMV), Stamm Davis oder AD169, wird *in vitro* auf humanen embryonalen Vorhautfibroblasten (NHDF-Zellen) angezüchtet. Nach Infektion der NHDF-Zellen mit einer Multiplizität der Infektion (M.O.I) von 0.01-0.03 werden die virusinfizierten Zellen 5-10 Tage später geerntet und in Gegenwart von Minimal Essential Medium (MEM), 20% foetalem Kälberserum (FKS) (v/v), 1% Glutamin (v/v), 1% Pen/Strep (v/v) mit 10% DMSO bei -80°C aufbewahrt. Nach serieller Verdünnung der virusinfizierten Zellen in Zehnerschritten erfolgt die Titerbestimmung auf 24-Well-Platten konfluenter NHDF-Zellen nach Fixierung und Färbung mit einer Giemsa-Formaldehyd-Lösung.

Vorbereitung der Schwämme. Transplantation. Behandlung und Auswertung:

1x1x1 cm große Kollagenschwämme (Gelfoam®; Fa. Peasel & Lorey, Best.-Nr. 407534; K.T. Chong et al., Abstracts of 39th Interscience Conference on Antimicrobial Agents and Chemotherapy, 1999, S. 439) werden zunächst mit Phosphat-gepufferter Saline (PBS) benetzt, die eingeschlossenen Luftblasen durch Entgasen entfernt und dann in MEM, 10% FKS (v/v), 1% Glutamin (v/v), 1% Pen/Strep (v/v) aufbewahrt. 1 x $10^6$ virusinfizierte NHDF-Zellen (Infektion mit HCMV-Davis oder HCMV AD169 M.O.I = 0.03) werden 3 Stunden nach Infektion abgelöst und in 20 μl MEM, 10% FKS (v/v), 1% Glutamin (v/v), 1% Pen/Strep (v/v) auf einen feuchten Schwamm getropft. Die Schwämme werden 3-4 Stunden inkubiert, um das Adhärieren der Zellen zu ermöglichen. Anschließend werden die Schwämme nach Zugabe von Medium (MEM, 10% FKS) (v/v), 1% Glutamin (v/v), 1% Pen/Strep (v/v)) über Nacht inkubiert. Zur Transplantation werden die immundefizienten Mäuse mit Avertin oder mit einer Ketamin/Xylazin/Azepromazin Mischung narkotisiert, das Rückenfell mit Hilfe eines Rasierers entfernt, die Oberhaut 1-2 cm geöffnet, entlastet und die feuchten Schwämme unter die Rückenhaut transplantiert. Die Operationswunde wird mit Gewebekleber oder Klammern verschlossen. 4 - 6 Stunden nach der Transplantation können die Mäuse zum ersten Mal behandelt werden (am Tag der Operation wird einmal behandelt). An den folgenden Tagen wird über einen Zeitraum von 8 Tagen dreimal täglich (7.00 Uhr und 14.00 Uhr und 19.00 Uhr), zweimal täglich (8 Uhr und 18 Uhr) oder einmal täglich (9 Uhr) peroral mit Substanz behandelt. Die Tagesdosis beträgt beispielsweise 1 oder 3 oder 10 oder 30 oder 100 mg/kg Körpergewicht, das Applikationsvolumen ist 10 ml/kg Körpergewicht. Die Formulierung der Substanzen erfolgt in Form einer 0.5%-igen Tylosesuspension / PBS mit 2% DMSO oder einer anderen geeigneten Mischung, die die Löslichkeit der Substanzen unterstützt, z. B. 2 % Ethanol, 2.5% Solutol, 95.5% PBS. 10 Tage nach Transplantation und ca. 16 Stunden nach der letzten Substanzapplikation werden die Tiere schmerzlos getötet und der Schwamm, entnommen. Die virusinfizierten Zellen werden durch Kollagenaseverdau (330 U/ 1.5 ml) aus dem Schwamm freigesetzt und in Gegenwart von MEM, 10% FKS (v/v), 1% Glutamin (v/v), 1% Pen/Strep (v/v), 10% DMSO bei -140°C aufbewahrt. Die Auswertung erfolgt nach serieller Verdünnung der virusinfizierten Zellen in Zehnerschritten durch Titerbestimmung auf 24-Well-Platten konfluenter NHDF-Zellen nach Fixierung und Färbung mit einer Giemsa-Formaldehyd-Lösung. Ermittelt wird die Anzahl infizierter Zellen bzw. infektiöser Viruspartikel (infectious center assay) nach Substanzbehandlung im Vergleich zur placebobehandelten Kontrollgruppe. Die statistische Auswertung erfolgt mittels geeigneter Computerprogramme, z. B. GraphPad Prism.

**hERG-Bindungs-Test:**

Die hERG-Bindung der Verbindungen kann in einem [$^3$H]-Astemizole Bindungs-Test in HEK293-Zellen gemessen werden, wie in der folgenden Veröffentlichung beschrieben: Peter J.S.Chiu et al., J. Pharmacol. Sci 95, 311-319 (2004).

### C. Ausführungsbeispiele für pharmazeutische Zusammensetzungen

Die erfindungsgemäßen Verbindungen können folgendermaßen in pharmazeutische Zubereitungen überführt werden:

#### Tablette:

Zusammensetzung:

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

Herstellung:

Die Mischung aus Wirkstoff, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Presskraft von 15 kN verwendet.

#### Oral applizierbare Suspension:

Zusammensetzung:

1000 mg der Verbindung von Beispiel 1, 1000 mg Ethanol (96%), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 ml orale Suspension.

Herstellung:

Das Rhodigel wird in Ethanol suspendiert, der Wirkstoff wird der Suspension zugefügt. Unter Rühren erfolgt die Zugabe des Wassers. Bis zum Abschluss der Quellung des Rhodigels wird ca. 6h gerührt.

#### Intravenös applizierbare Lösung:

Zusammensetzung:

10-500 mg der Verbindung von Beispiel 1, 15 g Polyethylenglykol 400 und 250 g Wasser für Injektionszwecke.

Herstellung:

Die Verbindung von Beispiel 1 wird zusammen mit Polyethylenglykol 400 in dem Wasser unter Rühren gelöst. Die Lösung wird sterilfiltriert (Porendurchmesser 0.22 µm) und unter aseptischen Bedingungen in hitzesterilisierte Infusionsflaschen abgefüllt. Diese werden mit Infusionsstopfen und Bördelkappen verschlossen.

## Patentansprüche

**1.** Verbindung der Formel

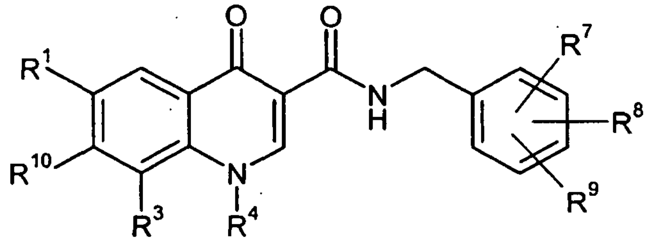

(I),

in welcher

$R^1$ für Wasserstoff, Fluor, Chlor oder Trifluormethyl steht,
$R^3$ für Halogen, Hydroxy, $C_1$-$C_4$-Alkoxy, Cyano, Trifluormethyl, Mono- fluormethoxy, Difluormethoxy, Trifluormethoxy oder Ethinyl steht,
$R^4$ für $C_1$-$C_6$-Alkyl oder $C_3$-$C_8$-Cycloalkyl steht, wobei Alkyl substituiert sein kann mit 1 bis 3 Substituenten, wobei die Substituenten unabhängig voneinander ausgewählt werden aus der Gruppe bestehend aus Halogen, Hydroxy, Amino, Cyano, Trifluor- methyl, Hydroxycarbonyl, Aminocarbonyl, $C_1$-$C_6$-Alkoxy, $C_1$-$C_6$-Alkyl- amino, $C_1$-$C_6$-Alkylcarbonyl und $C_1$-$C_6$-Alkoxycarbonyl,
und
wobei Cycloalkyl substituiert sein kann mit 1 bis 3 Substituenten, wo- bei die Substituenten unabhängig voneinander ausgewählt werden aus der Gruppe bestehend aus Halogen, Hydroxy, Amino, Cyano, Trifluor- methyl, Hydroxycarbonyl, Aminocarbonyl, $C_1$-$C_6$-Alkyl, $C_1$-$C_6$-Alkoxy, $C_1$-$C_6$-Alkylamino, $C_1$-$C_6$-Alkylcarbonyl und $C_1$-$C_6$-Alkoxycarbonyl,

oder

$R^3$ und $R^4$ bilden gemeinsam mit den Atomen an die sie gebunden sind einen Ring durch eine Gruppe der Formel

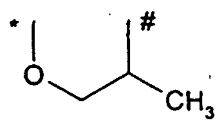

wobei

* die Anknüpfstelle an das Kohlenstoffatom ist,
und
\# die Anknüpfstelle an das Stickstoffatom ist,

$R^7$ und $R^8$ unabhängig voneinander für Halogen, Hydroxy, Cyano, Tri- fluormethyl, Monofluormethoxy, Difluormethoxy, Trifluormethoxy, $C_1$-$C_3$-Alkyl oder $C_1$-$C_3$-Alkoxy stehen,
und
$R^9$ für Wasserstoff, Halogen, Hydroxy, Cyano, Trifluormethyl, Mono- fluormethoxy, Difluormethoxy, Trifluormethoxy, $C_1$-$C_3$-Alkyl oder $C_1$-$C_3$-Alkoxy steht,
oder
$R^8$ für Trifluormethoxy steht,
und
$R^7$ und $R^9$ für Wasserstoff stehen,
$R^{10}$ für eine Gruppe der Formel

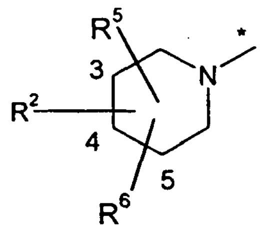, 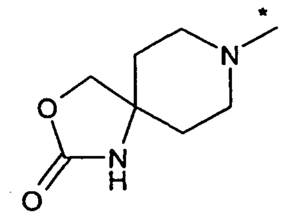 oder 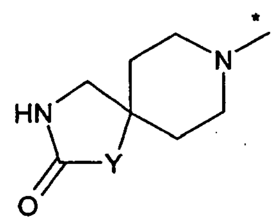

steht,
wobei

* die Anknüpfstelle an das Kohlenstoffatom ist,
$R^2$ an die Position 3 oder 4 gebunden ist und für Hydroxy, Hydro- xycarbonyl, Aminocarbonyl, $C_1$-$C_4$-Alkyl, $C_1$-$C_4$-Alkoxycarbonyl, $C_3$-$C_6$-Cycloalkylaminocarbonyl oder gegebenenfalls Hydroxy substituiertes $C_1$-$C_6$-Alkylaminocarbonyl steht, wobei Alkyl substituiert ist mit einem Substituenten, wobei der Substituent ausgewählt wird aus der Gruppe bestehend aus Hydroxy, Hydroxycarbonyl, Aminocarbonyl, $C_1$-$C_4$-Alkoxy-carb- onyl und 2-Oxopyrrolidin-1-yl,
$R^5$ und $R^6$ unabhängig voneinander an die Position 3, 4 oder 5 ge- bunden sind und unabhängig voneinander für Wasserstoff, Hydroxy, Methyl oder Ethyl stehen,
und
Y für eine Methylengruppe oder ein Sauerstoffatom steht,

oder eines ihrer Salze, ihrer Solvate oder der Solvate ihrer Salze.

**2.** Verbindung nach Anspruch 1, **dadurch gekennzeichnet, dass** sie der Formel

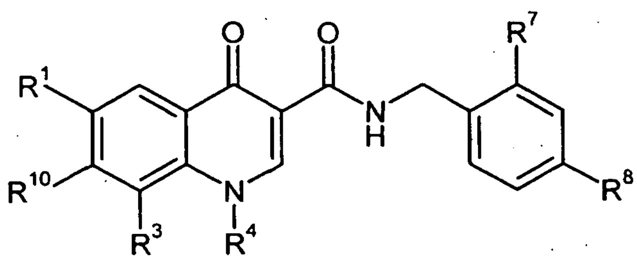
(Ia),

entspricht,
in welcher

$R^1$ für Wasserstoff, Fluor, Chlor oder Trifluormethyl steht,
$R^3$ für Halogen, Hydroxy, $C_1$-$C_4$-Alkoxy, Cyano, Trifluormethyl, Mono- fluormethoxy, Difluormethoxy, Trifluormethoxy oder Ethinyl steht,
$R^4$ für $C_1$-$C_6$-Alkyl oder $C_3$-$C_8$-Cycloalkyl steht, oder wobei Alkyl substituiert sein kann mit 1 bis 3 Substituenten, wobei die Substituenten unabhängig voneinander ausgewählt werden aus der Gruppe bestehend aus Halogen, Hydroxy, Amino, Cyano, Trifluor- methyl, Hydroxycarbonyl, Aminocarbonyl, $C_1$-$C_6$-Alkoxy, $C_1$-$C_6$-Alkyl- amino, $C_1$-$C_6$-Alkylcarbonyl und $C_1$-$C_6$-Alkoxycarbonyl, und wobei Cycloalkyl substituiert sein kann mit 1 bis 3 Substituenten, wo- bei die Substituenten unabhängig voneinander ausgewählt werden aus der Gruppe bestehend aus Halogen, Hydroxy, Amino, Cyano, Trifluor- methyl, Hydroxycarbonyl, Aminocarbonyl, $C_1$-$C_6$-Alkyl, $C_1$-$C_6$-Alkoxy, $C_1$-$C_6$-Alkylamino, $C_1$-$C_6$-Alkylcarbonyl und $C_1$-$C_6$-Alkoxycarbonyl,
$R^3$ und $R^4$ bilden gemeinsam mit den Atomen an die sie gebunden sind einen Ring durch eine Gruppe der Formel

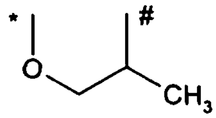

wobei

* die Anknüpfstelle an das Kohlenstoffatom ist,
und
# die Anknüpfstelle an das Stickstoffatom ist,

$R^7$ und $R^8$ unabhängig voneinander für Halogen, Hydroxy, Cyano, Tri- fluormethyl, Monofluormethoxy, Difluormethoxy, Trifluormethoxy, $C_1$-$C_3$-Alkyl oder $C_1$-$C_3$-Alkoxy stehen,
$R^{10}$ für eine Gruppe der Formel

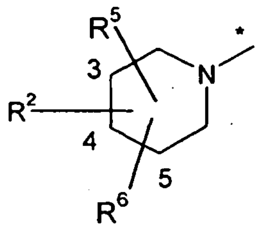
,
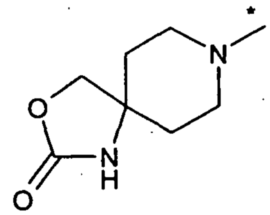
oder
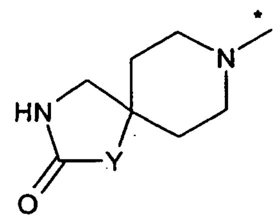

steht,
wobei

* die Anknüpfstelle an das Kohlenstoffatom ist,
$R^2$ an die Position 3 oder 4 gebunden ist und für Hydroxy, Hydro- xycarbonyl, Aminocarbonyl, $C_1$-$C_4$-Alkyl oder $C_1$-$C_4$-Alkoxycarb- onyl steht, wobei Alkyl substituiert ist mit einem Substituenten, wobei der Substituent ausgewählt wird aus der Gruppe bestehend aus Hydroxy, Hydroxycarbonyl, Aminocarbonyl und $C_1$-$C_4$-Alkoxy- carbonyl,
$R^5$ und $R^6$ unabhängig voneinander an die Position 3, 4 oder 5 ge- bunden sind und unabhängig voneinander für Wasserstoff, Hydroxy, Methyl oder Ethyl stehen,
und
Y für eine Methylengruppe oder ein Sauerstoffatom steht,

oder eines ihrer Salze, ihrer Solvate oder der Solvate ihrer Salze.

**3.** Verbindung nach Anspruch 2, **dadurch gekennzeichnet, dass**

$R^1$ für Wasserstoff, Fluor oder Chlor steht,
$R^3$ für Halogen, Hydroxy, $C_1$-$C_3$-Alkoxy, Cyano, Trifluormethyl, Monofluormethoxy, Difluormethoxy oder Trifluormethoxy steht,
$R^4$ für $C_1$-$C_6$-Alkyl oder $C_3$-$C_6$-Cycloalkyl steht,
wobei Alkyl substituiert sein kann mit 1 bis 3 Substituenten, wobei die Substituenten unabhängig voneinander ausgewählt werden aus der Gruppe bestehend aus Halogen, Hydroxy, Amino, Cyano, Trifluor- methyl und $C_1$-$C_4$-Alkoxy,
und
wobei Cycloalkyl substituiert sein kann mit 1 bis 3 Substituenten, wo- bei die Substituenten unabhängig voneinander ausgewählt werden aus der Gruppe bestehend aus Halogen, Hydroxy, Amino, Cyano, Trifluor- methyl, $C_1$-$C_4$-Alkyl und $C_1$-$C_4$-Alkoxy,

oder

$R^3$ und $R^4$ bilden gemeinsam mit den Atomen an die sie gebunden sind einen Ring durch eine Gruppe der Formel

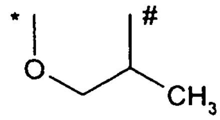

wobei

* die Anknüpfstelle an das Kohlenstoffatom ist,
und
# die Anknüpfstelle an das Stickstoffatom ist,

$R^7$ und $R^8$ unabhängig voneinander für Halogen, Cyano, Trifluormethyl, Monofluormethoxy, Difluormethoxy, Trifluormethoxy, $C_1$-$C_3$-Alkyl oder$C_1$-$C_3$-Alkoxy stehen,
$R^{10}$ für eine Gruppe der Formel

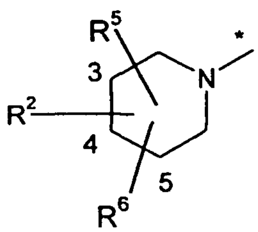 , 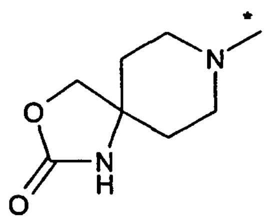 oder 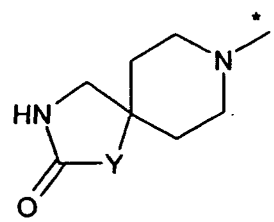

steht,
wobei

* die Anknüpfstelle an das Kohlenstoffatom ist,
$R^2$ an die Position 3 oder 4 gebunden ist und für Hydroxy, Hydro- xycarbonyl, Aminocarbonyl, $C_1$-$C_4$-Alkyl oder $C_1$-$C_4$-Alkoxy- carbonyl steht, wobei Alkyl substituiert ist mit einem Substituenten, wobei der Substituent ausgewählt wird aus der Gruppe bestehend aus Hydroxycarbonyl und $C_1$-$C_4$-Alkoxycarbonyl,
$R^5$ und $R^6$ unabhängig voneinander an die Position 3, 4 oder 5 ge- bunden sind und unabhängig voneinander für Wasserstoff, Hydroxy, Methyl oder Ethyl stehen,
und
Y für eine Methylengruppe oder ein Sauerstoffatom steht, oder eines ihrer Salze, ihrer Solvate oder der Solvate ihrer Salze.

4. Verfahren zur Herstellung einer Verbindung der Formel (I) nach Anspruch 1, **dadurch gekennzeichnet, dass**

[A] eine Verbindung der Formel

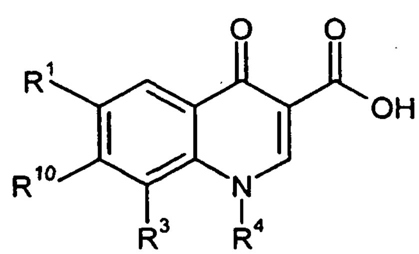 (II),

in welcher $R^1$, $R^3$, $R^4$ und $R^{10}$ die in Anspruch 1 angegebene Bedeutung aufweisen,

mit einer Verbindung der Formel

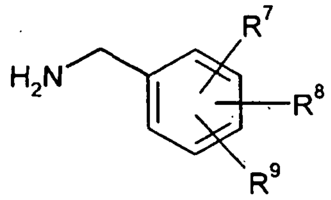

(III),

in welcher
$R^7$, $R^8$ und $R^9$ die in Anspruch 1 angegebene Bedeutung aufweisen,
umgesetzt wird,
oder
[B] eine Verbindung der Formel

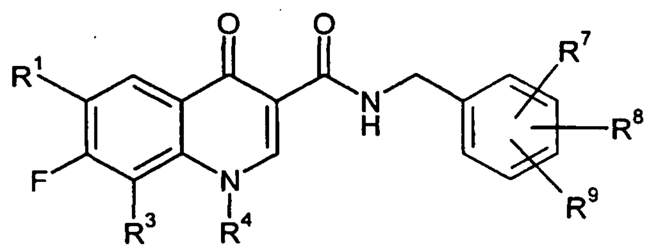

(IV),

in welcher
$R^1$, $R^3$, $R^4$, $R^7$, $R^8$ und $R^9$ die in Anspruch 1 angegebene Bedeutung aufweisen,
mit einer Verbindung der Formel

$R^{10}$-H (V),

in welcher
$R^{10}$ die in Anspruch 1 angegebene Bedeutung aufweist,
umgesetzt wird,
oder
[C] eine Verbindung, die nach Verfahren [A] oder [B] entsteht und eine Ester-Gruppe im Rest $R^{10}$ trägt, mit einer Base zu der entsprechenden Säure verseift wird.

**5.** Verbindung nach einem der Ansprüche 1 bis 3 zur Behandlung und/oder Prophylaxe von Krankheiten.

**6.** Arzneimittel enthaltend eine Verbindung nach einem der Ansprüche 1 bis 3 in Kombination mit einem inerten, nichttoxischen, pharmazeutisch geeigneten Hilfsstoff

**7.** Verwendung einer Verbindung nach einem der Ansprüche 1 bis 3 zur Herstellung eines Arzneimittels zur Behandlung und/oder Prophylaxe von Virusinfektionen.

**8.** Verwendung nach Anspruch 7, **dadurch gekennzeichnet, dass** die Virusinfektion eine Infektion mit dem humanen Cytomegalovirus (HCMV) oder einem anderen Vertreter der Gruppe der Herpes viridae ist.

**9.** Arzneimittel nach Anspruch 6 zur Behandlung und/oder Prophylaxe von Virusinfektionen.

**10.** Verbindung nach einem der Ansprüche 1 bis 3, Arzneimittel nach Anspruch 6 oder nach Anspruch 7 oder 8 erhältliches Arzneimittel zur Verwendung in einem Verfahren zur Bekämpfung von Virusinfektionen in Menschen und

Tieren.

## Claims

**1.** Compound of formula

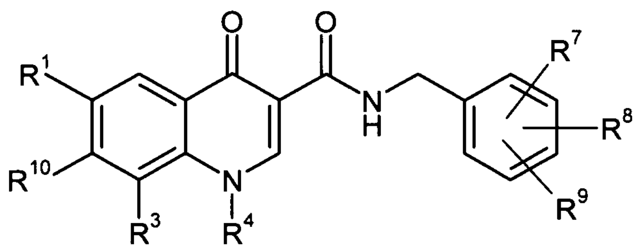

(I),

in which

$R^1$ represents hydrogen, fluorine, chlorine or trifluoromethyl,
$R^3$ represents halogen, hydroxy, $C_1$-$C_4$-alkoxy, cyano, trifluoromethyl, monofluoromethoxy, difluoromethoxy, trifluoromethoxy or ethynyl,
$R^4$ represents $C_1$-$C_6$-alkyl or $C_3$-$C_8$-cycloalkyl,
whereby alkyl can be substituted with 1 to 3 substituents, whereby the substituents are selected independently of one another from the group consisting of halogen, hydroxy, amino, cyano, trifluoromethyl, hydroxycarbonyl, aminocarbonyl, $C_1$-$C_6$-alkoxy, $C_{1-}C_6$-alkylamino, $C_1$- $C_6$-alkylcarbonyl and $C_1$-$C_6$-alkoxycarbonyl,
and
whereby cycloalkyl can be substituted with 1 to 3 substituents, whereby the substituents are selected independently of one another from the group consisting of halogen, hydroxy, amino, cyano, trifluoromethyl, hydroxycarbonyl, aminocarbonyl, $C_1$-$C_6$-alkyl, $C_1$-$C_6$-alkoxy, $C_1$-$C_6$-alk- ylamino, $C_1$-$C_6$-alkylcarbonyl and $C_1$-$C_6$-alkoxycarbonyl,

or

$R^3$ and $R^4$, together with the atoms to which they are attached, form a ring through a group of formula

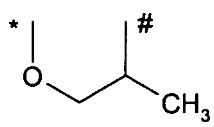

whereby

* is the linkage site to the carbon atom,
and
\# is the linkage site to the nitrogen atom,

$R^7$ and $R^8$ independently of one another represent halogen, hydroxy, cyano, trifluoromethyl, monofluoromethoxy, difluoromethoxy, tri- fluoromethoxy, $C_1$-$C_3$-alkyl or $C_1$-$C_3$-alkoxy,
and
$R^9$ represents hydrogen, halogen, hydroxy, cyano, trifluoromethyl, mono- fluoromethoxy, difluoromethoxy, trifluoromethoxy, $C_1$-$C_3$-alkyl or $C_1$- $C_3$-alkoxy,
or
$R^8$ represents trifluoromethoxy,
and

$R^7$ and $R^9$ represent hydrogen,
$R^{10}$ represents a group of formula

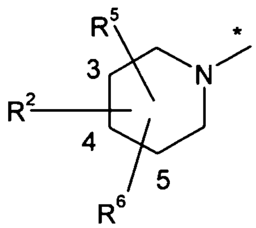 , 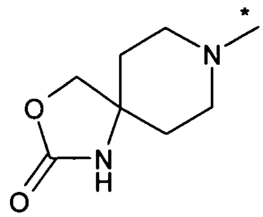 or 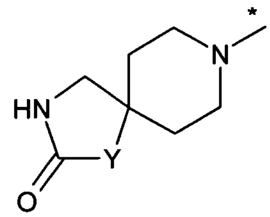

whereby

* is the linkage site to the carbon atom,
$R^2$ is attached at position 3 or 4 and represents hydroxy, hydroxy- carbonyl, aminocarbonyl, $C_1$-$C_4$-alkyl, $C_1$-$C_4$-alkoxycarbonyl, $C_3$- $C_6$-cycloalkylaminocarbonyl or optionally hydroxy-substituted $C_1$-$C_6$-alkylaminocarbonyl, whereby alkyl is substituted with a substituent, whereby the substituent is selected from the group consisting of hydroxy, hydroxycarbonyl, aminocarbonyl, $C_1$-$C_4$-alkoxycarbonyl and 2- oxopyrrolidin-1-yl,
$R^4$ and $R^6$ independently of one another are attached at position 3, 4 or 5 and independently of one another represent hydrogen, hydroxy, methyl, or ethyl,
and
Y represents a methylene group or an oxygen atom,

or one of its salts, its solvates or the solvates of its salts.

2. Compound according to Claim 1, **characterized in that** it conforms to formula

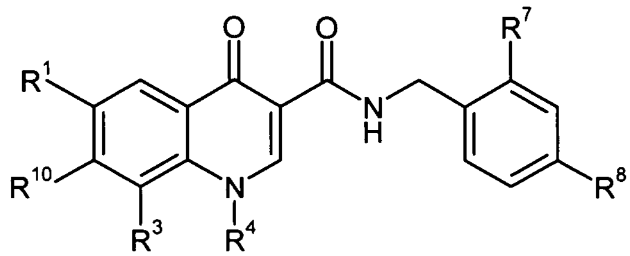 (Ia),

in which

$R^1$ represents hydrogen, fluorine, chlorine or trifluoromethyl,
$R^3$ represents halogen, hydroxy, $C_1$-$C_4$-alkoxy, cyano, trifluoromethyl, mo- nofluoromethoxy, difluoromethoxy, trifluoromethoxy or ethynyl,
$R^4$ represents $C_1$-$C_6$-alkyl or $C_3$-$C_8$-cycloalkyl, whereby alkyl can be substituted with 1 to 3 substituents, whereby the substituents are selected independently of one another from the group consisting of halogen, hydroxy, amino, cyano, trifluoromethyl, hy- droxycarbonyl, aminocarbonyl, $C_1$-$C_6$-alkoxy, $C_1$-$C_6$-alkylamino, $C_1$-$C_6$- alkylcarbonyl and $C_1$-$C_6$-alkoxycarbonyl,
and
whereby cycloalkyl can be substituted with 1 to 3 substituents, whereby the substituents are selected independently of one another from the group consisting of halogen, hydroxy, amino, cyano, trifluoromethyl, hydroxycarbonyl, aminocarbonyl, $C_1$-$C_6$-alkyl, $C_1$-$C_6$-alkoxy, $C_1$-$C_6$- alkylamino, $C_1$-$C_6$-alkylcarbonyl and $C_1$-$C_6$-alkoxycarbonyl,

or

$R^3$ and $R^4$, together with the atoms to which they are attached, form a ring through a group of formula

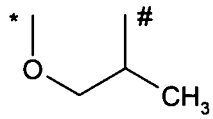

where

* is the linkage site to the carbon atom,
and
# is the linkage site to the nitrogen atom,

$R^7$ and $R^8$ independently of one another represent halogen, hydroxy, cyano, trifluoromethyl, monofluoromethoxy, difluoromethoxy, trifluo- romethoxy, $C_1$-$C_3$-alkyl or $C_1$-$C_3$-alkoxy,
$R^{10}$ represents a group of formula

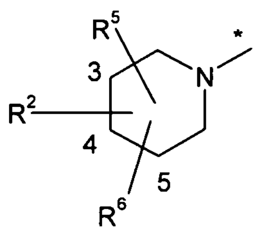

,

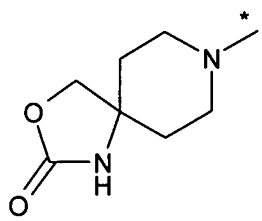

or

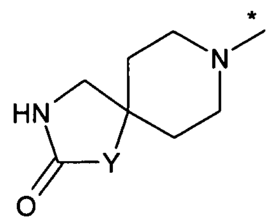

whereby

* is the linkage site to the carbon atom,
$R^2$ is attached at position 3 or 4 and represents hydroxy, hydroxycarbonyl, aminocarbonyl, $C_1$-$C_4$-alkyl or $C_1$-$C_4$-alk- oxycarbonyl, whereby alkyl is substituted with a substituent, whereby the substituent is selected from the group consisting of hydroxy, hydroxycarbonyl, aminocarbonyl and $C_1$-$C_4$-alkoxycarbonyl,
$R^5$ and $R^6$ independently of one another are attached at position 3, 4 or 5 and independently of one another represent hydrogen, hydroxy, methyl, or ethyl,
and
Y represents a methylene group or an oxygen atom,

or one of its salts, its solvates or the solvates of its salts.

**3.** Compound according to Claim 2, **characterized in that**

$R^1$ represents hydrogen, fluorine or chlorine,
$R^3$ represents halogen, hydroxy, $C_1$-$C_3$-alkoxy, cyano, trifluoromethyl, monofluoromethoxy, difluoromethoxy or trifluoromethoxy,
$R^4$ represents $C_1$-$C_6$-alkyl or $C_3$-$C_6$-cycloalkyl, whereby alkyl can be substituted with 1 to 3 substituents, whereby the substituents are selected independently of one another from the group consisting of halogen, hydroxy, amino, cyano, trifluoromethyl and $C_1$- $C_4$-alkoxy, and whereby cycloalkyl can be substituted with 1 to 3 substituents, whereby the substituents are selected independently of one another from the group consisting of halogen, hydroxy, amino, cyano, trifluoromethyl, $C_1$-$C_4$-alkyl and$C_1$-$C_4$-alkoxy,
or
$R^3$ and $R^4$, together with the atoms to which they are attached, form a ring through a group of formula

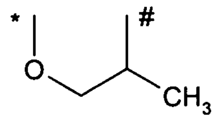

whereby

* is the linkage site to the carbon atom,
and
# is the linkage site to the nitrogen atom,

$R^7$ and $R^8$ independently of one another represent halogen, cyano, trifluoromethyl, monofluoromethoxy, difluoromethoxy, trifluorometh- oxy, $C_1$-$C_3$-alkyl or$C_1$-$C_3$-alkoxy,
$R^{10}$ represents a group of formula

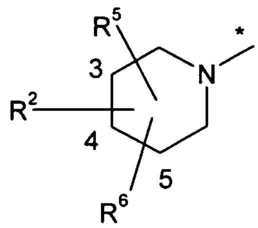 , 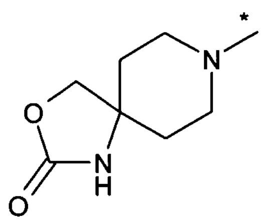 or 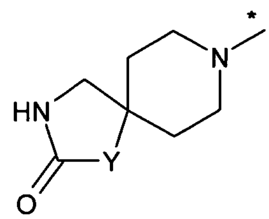

whereby

* is the linkage site to the carbon atom,
$R^2$ is attached at position 3 or 4 and represents hydroxy, hydroxy- carbonyl, aminocarbonyl, $C_1$-$C_4$-alkyl or $C_1$-$C_4$-alkoxycarbonyl, whereby alkyl is substituted with a substituent, whereby the substituent is selected from the group consisting of hydroxycarb- onyl and $C_1$-$C_4$-alkoxycarbonyl,
$R^5$ and $R^6$ independently of one another are attached at position 3, 4 or 5 and independently of one another represent hydrogen, hydroxy, methyl or ethyl,
and
Y represents a methylene group or an oxygen atom,

or one of its salts, its solvates or the solvates of its salts.

**4.** Method for preparing a compound of formula (I) according to Claim 1, **characterized in that**

[A] a compound of formula

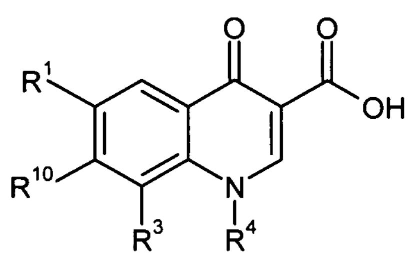 (II),

in which
$R^1$, $R^3$, $R^4$ and $R^{10}$ have the meaning indicated in Claim 1,

is reacted with a compound of formula

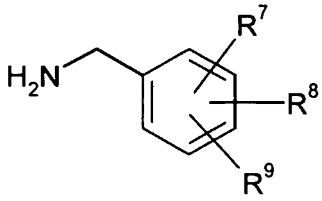
(III),

in which $R^7$, $R^8$ and $R^9$ have the meaning indicated in Claim 1,
or
[B] a compound of formula

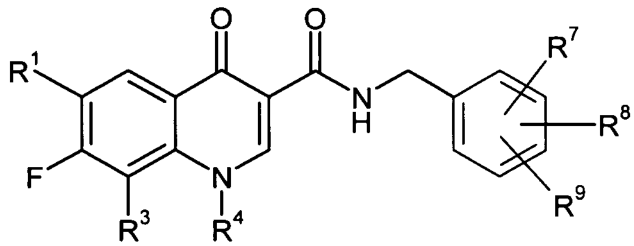
(IV),

in which
$R^1$, $R^3$, $R^4$, $R^7$, $R^8$ and $R^9$ have the meaning indicated in Claim 1,
is reacted with a compound of formula $R^{10}$-H (V),
in which
$R^{10}$ has the meaning indicated in Claim 1,
or
[C] a compound formed by method [A] or [B] and carrying an ester group in the radical $R^{10}$ is hydrolysed with a base to form the corresponding acid.

**5.** Compound according to one of Claims 1 to 3 for the treatment and/or prophylaxis of diseases.

**6.** Medicament comprising a compound according to any one of Claims 1 to 3 in combination with an inert, non-toxic, pharmaceutically acceptable excipient.

**7.** Use of a compound according to any one of Claims 1 to 3 for the production of a medicament for the treatment and/or prophylaxis of viral infections.

**8.** Use according to Claim 7, **characterized in that** the viral infection is an infection with the human cytomegalovirus (HCMV) or another representative of the group of herpes viridae.

**9.** Medicament according to Claim 6 for the treatment and/or prophylaxis of viral infections.

**10.** Compound according to any one of Claims 1 to 3, medicament according to Claim 6 or medicament obtained according to Claim 7 or 8 for use in a method of controlling viral infections in humans and animals.

**Revendications**

**1.** Composé de formule

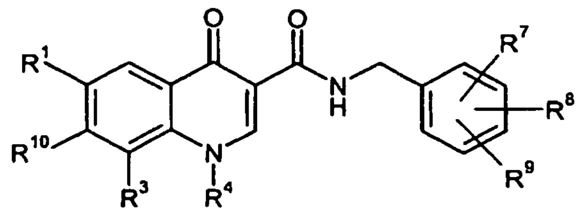

(I),

dans laquelle
$R^1$ est hydrogène, fluor, chlore ou trifluorométhyle,
$R^3$ est halogène, hydroxy, alcoxy en $C_1$ à $C_4$, cyano, trifluorométhyle, monofluorométhoxy, difluorométhoxy, trifluorométhoxy ou éthynyle,
$R^4$ est alkyle en $C_1$ à $C_6$ ou cycloalkyle en $C_3$ à $C_8$,
l'alkyle pouvant être substitué par 1 à 3 substituants, lesquels substituants sont choisis indépendamment les uns des autres dans le groupe constitué par halogène, hydroxy, amino, cyano, trifluorométhyle, hydroxycarbonyle, aminocarbonyle, alcoxy en $C_1$ à $C_6$, alkylamino en $C_1$ à $C_6$, alkylcarbonyle en $C_1$ à $C_6$ et alcoxycarbonyle en $C_1$ à $C_6$,
et
le cycloalkyle pouvant être substitué par 1 à 3 substituants, lesquels substituants sont choisis indépendamment les uns des autres dans le groupe constitué par halogène, hydroxy, amino, cyano, trifluorométhyle, hydroxycarbonyle, aminocarbonyle, alkyle en $C_1$ à $C_6$, alcoxy en $C_1$ à $C_6$, alkylamino en $C_1$ à $C_6$, alkylcarbonyle en $C_1$ à $C_6$ et alcoxycarbonyle en $C_1$ à $C_6$,
ou
$R^3$ et $R^4$ forment ensemble avec les atomes auxquels ils sont rattachés un noyau cyclique par le biais d'un groupe de formule

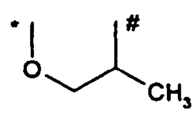

où
* désigne le point de rattachement à l'atome de carbone, et
# désigne le point de rattachement à l'atome d'azote,
$R^7$ et $R^8$ sont indépendamment l'un de l'autre halogène, hydroxy, cyano, trifluorométhyle, monofluorométhoxy, difluorométhoxy, trifluorométhoxy, alkyle en $C_1$ à $C_3$ ou alcoxy en $C_1$ à $C_3$,
et
$R^9$ est hydrogène, halogène, hydroxy, cyano, trifluorométhyle, monofluorométhoxy, difluorométhoxy, trifluorométhoxy, alkyle en $C_1$ à $C_3$ ou alcoxy en $C_1$ à $C_3$,
ou
$R^8$ est trifluorométhoxy,
et
$R^7$ et $R^9$ sont chacun hydrogène
$R^{10}$ est un groupe de formule

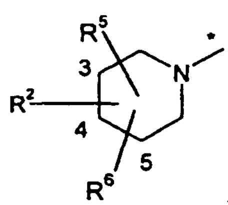

,

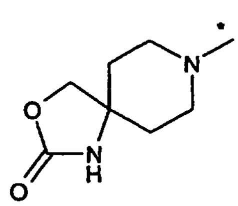

ou

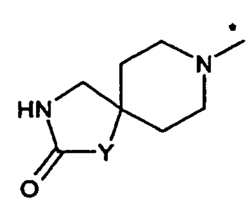

où * désigne le point de rattachement à l'atome de carbone,
$R^2$ est rattaché en position 3 ou 4 et est hydroxy, hydroxycarbonyle, aminocarbonyle, alkyle en $C_1$ à $C_4$, alcoxycarbonyle en $C_1$ à $C_4$, cycloalkylaminocarbonyle en $C_3$ à $C_6$, ou alkylaminocarbonyle en $C_1$ à $C_6$ le cas échéant hydroxysubstitué,
l'alkyle étant substitué par un substituant, lequel substituant est choisi dans le groupe constitué par hydroxy, hydroxycarbonyle, aminocarbonyle, alcoxycarbonyle en $C_1$ à $C_4$ et 2-oxopyrrolidin-1-yle,
$R^5$ et $R^6$ sont rattachés indépendamment l'un de l'autre en position 3, 4 ou 5 et sont indépendamment l'un de l'autre hydrogène, hydroxy, méthyle ou éthyle,
et
Y est un groupe méthylène ou un atome d'oxygène,
ou un de ses sels, de ses solvates ou des solvates de ses sels.

**2.** Composé selon la revendication 1, **caractérisé en ce qu'**il correspond à la formule

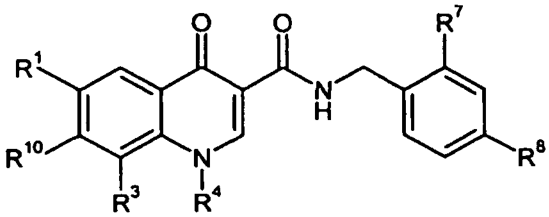

(Ia),

dans laquelle
$R^1$ est hydrogène, fluor, chlore ou trifluorométhyle,
$R^3$ est halogène, hydroxy, alcoxy en $C_1$ à $C_4$, cyano, trifluorométhyle, monofluorométhoxy, difluorométhoxy, trifluorométhoxy ou éthynyle,
$R^4$ est alkyle en $C_1$ à $C_6$ ou cycloalkyle en $C_3$ à $C_8$,
l'alkyle pouvant être substitué par 1 à 3 substituants, lesquels substituants sont choisis indépendamment les uns des autres dans le groupe constitué par halogène, hydroxy, amino, cyano, trifluorométhyle, hydroxycarbonyle, aminocarbonyle, alcoxy en $C_1$ à $C_6$, alkylamino en $C_1$ à $C_6$, alkylcarbonyle en $C_1$ à $C_6$ et alcoxycarbonyle en $C_1$ à $C_6$,
et
le cycloalkyle pouvant être substitué par 1 à 3 substituants, lesquels substituants sont choisis indépendamment les uns des autres dans le groupe constitué par halogène, hydroxy, amino, cyano, trifluorométhyle, hydroxycarbonyle, aminocarbonyle, alkyle en $C_1$ à $C_6$, alcoxy en $C_1$ à $C_6$, alkylamino en $C_1$ à $C_6$, alkylcarbonyle en $C_1$ à $C_6$ et alcoxycarbonyle en $C_1$ à $C_6$,
ou
$R^3$ et $R^4$ forment ensemble avec les atomes auxquels ils sont rattachés un noyau cyclique par le biais d'un groupe de formule

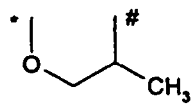

où
* désigne le point de rattachement à l'atome de carbone,
et
# désigne le point de rattachement à l'atome d'azote,
$R^7$ et $R^8$ sont indépendamment l'un de l'autre halogène, hydroxy, cyano, trifluorométhyle, monofluorométhoxy, difluorométhoxy, trifluorométhoxy, alkyle en $C_1$ à $C_3$ ou alcoxy en $C_1$ à $C_3$,
$R^{10}$ est un groupe de formule

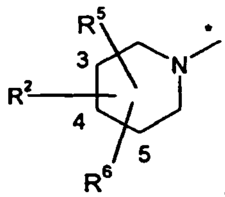
,
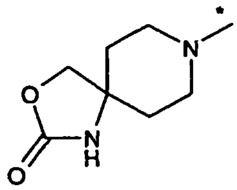
ou
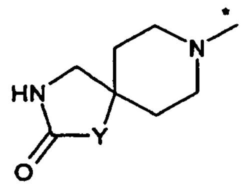

où
* désigne le point de rattachement à l'atome de carbone,
$R^2$ est rattaché en position 3 ou 4 et est hydroxy, hydroxycarbonyle, aminocarbonyle, alkyle en $C_1$ à $C_4$ ou alcoxycarbonyle en $C_1$ à $C_4$,
l'alkyle étant substitué par un substituant, lequel substituant est choisi dans le groupe constitué par hydroxy, hydroxycarbonyle, aminocarbonyle et alcoxycarbonyle en $C_1$ à $C_4$,
$R^5$ et $R^6$ sont rattachés indépendamment l'un de l'autre en position 3, 4 ou 5 et sont indépendamment l'un de l'autre hydrogène, hydroxy, méthyle ou éthyle,
et
Y est un groupe méthylène ou un atome d'oxygène,
ou un de ses sels, de ses solvates ou des solvates de ses sels.

**3.** Composé selon la revendication 2, **caractérisé en ce que**
$R^1$ est hydrogène, fluor ou chlore,
$R^3$ est halogène, hydroxy, alcoxy en $C_1$ à $C_3$, cyano, trifluorométhyle, monofluorométhoxy, difluorométhoxy ou trifluorométhoxy,
$R^4$ est alkyle en $C_1$ à $C_6$ ou cycloalkyle en $C_3$ à $C_6$,
l'alkyle pouvant être substitué par 1 à 3 substituants, lesquels substituants sont choisis indépendamment les uns des autres dans le groupe constitué par halogène, hydroxy, amino, cyano, trifluorométhyle et alcoxy en $C_1$ à $C_4$,
et
le cycloalkyle pouvant être substitué par 1 à 3 substituants, lesquels substituants sont choisis indépendamment les uns des autres dans le groupe constitué par halogène, hydroxy, amino, cyano, trifluorométhyle, alkyle en $C_1$ à $C_4$ et alcoxy en $C_1$ à $C_4$,
ou
$R^3$ et $R^4$ forment ensemble avec les atomes auxquels ils sont rattachés
un noyau cyclique par le biais d'un groupe de formule

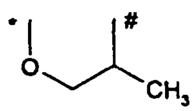

où
* désigne le point de rattachement à l'atome de carbone,
et
# désigne le point de rattachement à l'atome d'azote,
$R^7$ et $R^8$ sont indépendamment l'un de l'autre halogène, cyano, trifluorométhyle, monofluorométhoxy, difluorométhoxy, trifluorométhoxy, alkyle en $C_1$ à $C_3$ ou alcoxy en $C_1$ à $C_3$,
$R^{10}$ est un groupe de formule

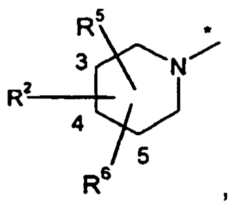
,
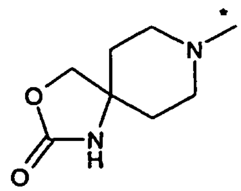
ou
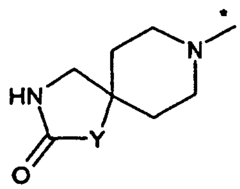

où
* désigne le point de rattachement à l'atome de carbone,
$R^2$ est rattaché en position 3 ou 4 et est hydroxy, hydroxycarbonyle, aminocarbonyle, alkyle en $C_1$ à $C_4$ ou alcoxycarbonyle en $C_1$ à $C_4$,
l'alkyle étant substitué par un substituant, lequel substituant est choisi dans le groupe constitué par hydroxycarbonyle et alcoxycarbonyle en $C_1$ à $C_4$,
$R^5$ et $R^6$ sont rattachés indépendamment l'un de l'autre en position 3, 4 ou 5 et sont indépendamment l'un de l'autre hydrogène, hydroxy, méthyle ou éthyle,
et
Y est un groupe méthylène ou un atome d'oxygène,
ou un de ses sels, de ses solvates ou des solvates de ses sels.

**4.** Procédé de préparation d'un composé de formule (I) selon la revendication 1, **caractérisé en ce que**

[A] on fait réagir un composé de formule

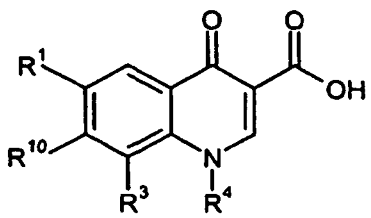
(II),

dans laquelle
$R^1$, $R^3$, $R^4$ et $R^{10}$ ont la signification indiquée dans la revendication 1, avec un composé de formule *

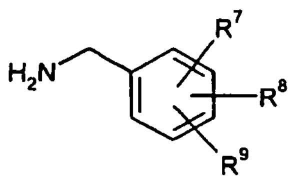
(III),

dans laquelle $R^7$, $R^8$ et $R^9$ ont la signification indiquée dans la revendication 1,
ou
[B] on fait réagir un composé de formule

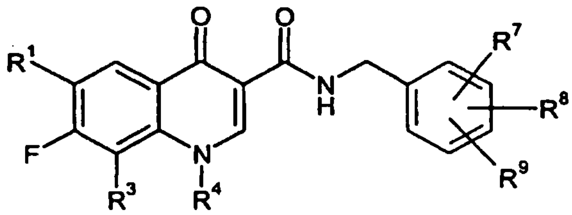

(IV),

dans laquelle $R^1$, $R^3$, $R^4$, $R^7$, $R^8$ et $R^9$ ont la signification indiquée dans la revendication 1, avec un composé de formule

$R^{10}$-H (V),

dans laquelle
$R^{10}$ a la signification indiquée dans la revendication 1,
ou
[C] on saponifie un composé obtenu par le procédé [A] ou [B] et portant un groupe ester dans le reste $R^{10}$ avec une base pour donner l'acide correspondant.

**5.** Composé selon l'une des revendications 1 à 3, destiné au traitement et/ou à la prévention de maladies.

**6.** Médicament contenant un composé selon l'une des revendications 1 à 3 en combinaison avec un adjuvant inerte, non toxique, pharmaceutiquement adéquat.

**7.** Utilisation d'un composé selon l'une des revendications 1 à 3 pour fabriquer un médicament destiné au traitement et/ou à la prévention d'infections virales.

**8.** Utilisation selon la revendication 7, **caractérisé en ce que** l'infection virale est une infection par le cytomégalovirus humain (CMVH) ou un autre représentant du groupe des herpès viridés.

**9.** Médicament selon la revendication 6, destiné au traitement et/ou à la prévention d'infections virales

**10.** Composé selon l'une des revendications 1 à 3, médicament selon la revendication 6, ou médicament pouvant être obtenu selon la revendication 7 ou la revendication 8, destiné à une utilisation dans un procédé de lutte contre des infections virales chez l'homme et les animaux.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 00040561 A
- US 4959363 A
- EP 612731 A
- WO 02009758 A
- WO 02085886 A
- WO 03050107 A
- WO 97004775 A
- WO 97004779 A
- EP 276700 A
- WO 02026713 A
- WO 9601262 A
- EP 0241206 A
- DE 3420743
- WO 2003031397 A
- GB 932487 A


### In der Beschreibung aufgeführte Nicht-Patentliteratur


- **A. Da Silva ; M. De Almeida ; V. De Souza ; M. Couri.** *Current Medicinal Chemistry,* 2003, vol. 10, 21-39
- **J. Cinatl et al.** *FEMS Microbiology Reviews,* 2004, vol. 28, 59-77
- *Journal of Medicinal Chemistry,* 1964, vol. 7 (6), 726-728
- *Journal of Medicinal Chemistry,* 1995, vol. 38 (19), 3772-3780
- *Eur. J. Med. Chim. Ther.,* 1974, vol. 9, 424-433
- *Journal of Medicinal Chemistry,* 1995, vol. 38 (22), 4478-87
- *Journal of Medicinal Chemistry,* 1992, vol. 35 (4), 611
- *Journal of Medicinal Chemistry,* 1981, vol. 24, 1320-28
- *Journal of Medicinal Chemistry,* 1995, vol. 38 (22), 4478-4487
- **Y. Kimura et al.** *J. Med. Chem.,* 1994, vol. 37 (20), 3344
- **K.T. Chong et al.** *Abstracts of 39th Interscience Conference on Antimicrobial Agents and Chemotherapy,* 1999, 439
- **Peter J.S.Chiu et al.** *J. Pharmacol. Sci,* 2004, vol. 95, 311-319